# EUROPEAN PATENT APPLICATION

(11) **EP 4 253 958 A2**
(43) Date of publication of application: **04.10.2023**
(21) Application number: 23172000.4
(22) Date of filing: 25.03.2019
(51) Int. Cl.: G01N 33/574

(54) **MEANS AND METHODS FOR GLYCOPROFILING OF A PROTEIN**

(30) Priority: 26.03.2018 EP 18163899
(62) Divisional of application: 19718572.1
(71) Applicant: Glycanostics s.r.o., 841 01 Bratislava (SK)
(72) Inventor: BERTOK, Tomas, 841 01 Bratislava (SK); TKAC, Jan, 841 01 Bratislava (SK)
(74) Representative: Schiweck Weinzierl Koch Patentanwälte Partnerschaft mbB

(57) **Abstract**

The present invention provides magnetic carriers, anti-glycoprotein antibodies, the antigen binding portions thereof, one or more lectins, compositions, kits, methods and uses based thereon including uses in methods for glycoprofiling of glycoproteins using lectins, e.g., in diagnostics of cancer. The magnetic carriers, anti-glycoprotein antibodies, the antigen binding portions thereof, one or more lectins, compositions, kits, methods and uses based thereon are applicable to any glycoprotein.

## Description

This application contains a Sequence Listing in computer readable form, which is incorporated herein by reference.

### Technical field

The present invention relates to magnetic carriers, anti-glycoprotein antibodies, the antigen binding portions thereof, one or more lectins, compositions, kits, methods and uses thereof including uses in methods for glycoprofiling of glycoproteins using lectins, e.g., in diagnostics of cancer (e.g., Table 1). The magnetic carriers, anti-glycoprotein antibodies, the antigen binding portions thereof, one or more lectins, compositions, kits, methods and uses based thereon are applicable to any glycoprotein (e.g., Table 1). However, due to their superior sensitivity and/or specificity magnetic carriers, anti-glycoprotein antibodies, the antigen binding portions thereof, one or more lectins, compositions, kits and methods and uses based thereon of the present invention are particularly suitable for isoform-specific detection and analysis of glycoproteins (e.g., in diagnostics of cancer).

### Background of the invention

The Prostate Specific Antigen (PSA) analysis revolutionised prostate cancer (PCa) screening since elevated PSA level in serum can precede clinical diagnosis of the PCa disease by 5 to 10 years or even longer. However, PSA is elevated in many benign conditions as well, resulting in a large number of false positive PSA tests. Furthermore, PSA does not discriminate significant and insignificant tumours. Therefore, in a significant subset of patients diagnosed with PCa, the disease will be slow growing and clinically harmless. These patients are exposed to risk of side effects of unnecessary treatment. Thus, the major problem of PSA-based PCa screening is unnecessary further expensive and for the patient cumbersome examinations (e.g., imaging, prostate biopsies etc.). Due to these reasons the United States Preventive Services Task Force (USPSTF) suggested not using PSA for PCa screening in 2012. The same agency issued in 2017 a guide to use PSA for PCa screening on an individual basis for men aged from 55 to 69.

Among PCa patients diagnosed after a moderately positive (4-10 ng/ml) PSA screening tests almost 75% of tumours are organ confined and potentially curable, while for patients with PSA level above 10 ng/mL, the proportion of organ-confined and curable PCa drops below 50%. Thus, detecting curable PCa with high specificity (avoiding unnecessary follow-up examination procedures) is a diagnostic challenge. Furthermore, overdiagnosis/overtreatment can lead to complication including urinary incontinence, sexual dysfunction and bowel problems in patients with harmless PCa. Therefore, an improved screening test should also allow to classify tumours according to their clinical significance.

PCa screening is usually recommended for 55+ years old men by PSA measurement in the serum. Existing methods used by clinical laboratories worldwide is not sensitive and specific enough and so in many cases it is very difficult to correctly identify healthy people from PCa patients at an initial and potentially curable stage. Thus, the clinicians/urologists need novel diagnostic PCa biomarkers.

### Summary of the invention

The present invention relates to a method of determining the glycoprofile of a protein, comprising: (a) contacting a sample comprising said protein with an antibody directed against said protein to form an antibody-protein complex; (b) isolating the antibody-protein complex obtained in step (a); and (c) contacting the antibody-protein complex with one or more lectins to determine the glycoprofile of said protein.

In the method of determining the glycoprofile of a protein, it is preferred that said antibody of step (a) is not immobilized, preferably not immobilized on a solid surface, and/or it is preferred that said protein is not released from said antibody while performing the method.

The present invention furthermore relates to a method for diagnosing whether a subject may be at a risk or may suffer from cancer, comprising
(a) contacting a sample obtained from said subject, said sample comprising a cancer biomarker protein, with an antibody directed against said cancer biomarker protein to form an antibody-cancer biomarker protein complex; and
(b) isolating the antibody-protein complex obtained in step (a); and
(c) contacting the antibody-cancer biomarker protein complex with one or more lectins to determine the glycoprofile of said cancer biomarker protein,
wherein a deviation of said glycoprofile from the healthy glycoprofile of said cancer biomarker protein is indicative that said subject may be at a risk or may suffer from cancer.

In the method for diagnosing whether a subject may be at a risk or may suffer from cancer, it is preferred that said antibody of step (a) is not immobilized, preferably not immobilized on a solid surface, and/or it is preferred that said protein is not released from said antibody while performing the method.

The present invention also relates to a method for diagnosing whether a subject may be at a risk or may suffer from an autoimmune disease, comprising
(a) contacting a sample obtained from said subject, said sample comprising an autoimmune disease biomarker protein, with an antibody directed against said autoimmune disease biomarker protein to form an antibody-autoimmune disease biomarker protein complex; and
(b) isolating the antibody-protein complex obtained in step (a); and
(c) contacting the antibody-autoimmune disease biomarker protein complex with one or more lectins to determine the glycoprofile of said autoimmune disease biomarker protein,
wherein a deviation of said glycoprofile from the healthy glycoprofile of said autoimmune disease biomarker protein is indicative that said subject may be at a risk or may suffer from an autoimmune disease.

In the method for diagnosing whether a subject may be at a risk or may suffer from an autoimmune disease, it is preferred that said antibody of step (a) is not immobilized, preferably not immobilized on a solid surface, and/or it is preferred that said protein is not released from said antibody while performing the method.

The present invention moreover relates to a method for diagnosing whether a subject may be at a risk or may suffer from an inflammatory disease, comprising
(a) contacting a sample obtained from said subject, said sample comprising an inflammatory disease biomarker protein, with an antibody directed against said inflammatory disease biomarker protein to form an antibody-inflammatory biomarker protein complex; and
(b) isolating the antibody-protein complex obtained in step (a); and
(c) contacting the antibody-inflammatory biomarker protein complex with one or more lectins to determine the glycoprofile of said inflammatory disease biomarker protein,
wherein a deviation of said glycoprofile from the healthy glycoprofile of said inflammatory disease biomarker protein is indicative that said subject may be at a risk or may suffer from an inflammatory disease.

In the method for diagnosing whether a subject may be at a risk or may suffer from an inflammatory disease, it is preferred that said antibody of step (a) is not immobilized, preferably not immobilized on a solid surface, and/or it is preferred that said protein is not released from said antibody while performing the method.

In addition, the present invention provides for a kit for perfoming the method of determining the glycoprofile of a protein as described herein, comprising an antibody specific for said protein as described herein and a lectin as described herein.

Also, the present invention provides for a kit for perfoming the method for diagnosing whether a subject may be at a risk or may suffer from cancer, comprising an antibody specific for a cancer biomarker protein as described herein and one or more lectins as described herein.

Furthermore, the present invention provides for a kit for perfoming the method for diagnosing whether a subject may be at a risk or may suffer from an autoimmune disease, comprising an antibody specific for an autoimmune disease biomarker protein which is IgG and one or more lectins as described herein.

Moreover, the present invention provides for a kit for perfoming the method for diagnosing whether a subject may be at a risk or may suffer from an inflammatory disease, comprising an antibody specific for an inflammatory biomarker protein which is IgG, IgA or CRP and one or more lectins as described herein.

The present invention further relates to a magnetic carrier comprising: i) an immobilized anti-glycoprotein antibody or the antigen binding portion thereof and ii) an immobilized polypeptide having peroxidase activity, wherein said immobilized polypeptide having peroxidase activity has a molecular weight of less than 2 kDa. The present invention further relates to an anti-glycoprotein antibody or the antigen binding portion thereof immobilized on a magnetic carrier, wherein said magnetic carrier further comprises a polypeptide having peroxidase activity immobilized on said magnetic carrier, wherein said polypeptide has a molecular weight of less than 2 kDa.

The present application satisfies this demand by the provision of magnetic carriers, anti-glycoprotein antibodies, the antigen binding portions thereof, one or more lectins, compositions and kits described herein below, characterized in the claims and illustrated by the appended Examples and Figures.

**Other suitable lectins (including post-translationally processed- and mature forms thereof) within the meaning of the present invention further include:**
Neu5Ac(α2-6)Gal/GalNAc (α2-6Neu5Ac) specific agglutinin (SNA-I) from *Sambucus nigra*, UniProtKB Accession Number: Q945S3.
Galβ1-3GalNAc binding *Agaricus bisporus* agglutinin (ABA), UniProtKB Accession Number: Q00022.
Neu5Acα2-3Galβ1-4GlcNAc binding *Allomyrina dichotoma* agglutinin (AlloA), No UniProtKB Accession Number currently available, but which, for example, can be purified as described by Umetsu et al., 1984, i.e., by a purification method comprising affinity chromatography on acid-treated crosslined, beaded-form of agarose (Sepharose) and diethylaminoethanol-spherical cellulose beads (DEAE-Cellulofine).
Galβ1-3GalNAc binding *Amaranthus caudatus* agglutinin (ACA), UniProtKB Accession Number: Q6YNX3 or Q71QF2.
Galβ1-3GalNAc binding *Arachis hypogaea agglutinin (AHA)* = *Peanut agglutinin (PNA),* UniProtKB Accession Number: P02872.
Galβ1-3GalNAc binding *Artocarpus integrifolia* agglutinin (AIA) = Jacalin, UniProtKB Accession Number: P18670.
Fucose binding *Aspergillus oryzae* lectin (AOL), UniProtKB Accession Number: Q2UNX8.
Mannose/glucose binding *Musa paradisiaca* lectin (BanLec), UniProtKB Accession Number: Q8L5H4 (e.g., also available in RCSB Protein data bank (https://www.rcsb.org) under PDB accession code 1X1V; Released Date: 2005-11-08; Version 1.2: 2011-07-13, also referenced by Singh et al., 2005).
(GlcNAcβ1-4)₂₋₄, Galβ1-4GlcNAc binding *Datura stramonium* agglutinin (Jacalin) (DSA), UniProtKB Accession Number: A0A089ZWN7.
GalNAcα1-3GalNAc binding *Dolichos biflorus* agglutinin (DBA), UniProtKB Accession Number: P05045 or P19588.
Galβ4GlcNAc binding *Erythrina cristagalli* lectin (ECL), UniProtKB Accession Number: P83410.
Galactose binding galectin 3, UniProtKB Accession Number: P17931.
Galactose and lactose binding galectin 4, UniProtKB Accession Number: P56470.
α-GalNAc, α-Gal binding *Griffonia (Bandeiraea) simplicifolia* lectin I (GSL I), UniProtKB Accession Number: P24146.
α-GlcNAc, β-GlcNAc and GlcNAcα1-4Galβ1-4GlcNAc binding *Griffonia (Bandeiraea) simplicifolia* lectin II (GSL II), UniProtKB Accession Number: Q41263.
α-mannose binding *Hippeastrum* hybrid (*Amaryllis*) lectin (HHL), UniProtKB Accession Number: Q39990.
α-GalNAc and GalNAcβ1-4Gal binding *Helix pomatia* agglutinin (HPA), UniProtKB Accession Number: Q2F1K8.
(GlcNAcβ1-4)₁₋₄ binding *Lycopersicon esculentum* (tomato) lectin (LEL), UniProtKB Accession Number: G9M5T0 or B3XYC5.
D-mannose or Fucα1-6GlcNAc-N-Asn containing *N*-linked oligosaccharides *specific Lens culinaris* agglutinin (LCA), UniProtKB Accession Number: P02870.
Fucα1-2Galβ1-4(Fucα1-3)GlcNAc specific *Lotus tetragonolobus* lectin (LTA), UniProtKB Accession Number: P19664.
Galβ1-4GlcNAc specific *Maackia amurensis* agglutinin I (MAA I), UniProtKB Accession Number: P0DKL3.
Galβ1-3(Fucα1-4)GlcNAc and Galβ1-4(Fucα1-3)GlcNAc binding macrophage galactose binding lectin 1 (MGBL 1), UniProtKB Accession Number: P49300.
GalNAc and galactose binding macrophage galactose binding lectin 2 (MGBL 2), UniProtKB Accession Number: A9XX86.
α-mannose binding *Narcissus pseudonarcissus* (Daffodil) lectin (NPA), UniProtKB Accession Number: Q40423.
GalNAcα1-3(Fucα1-2)Gal binding *Phaseolus lunatus* agglutinin (lima bean, LBA), UniProtKB Accession Number: P16300.
*N*-linked bi-antennary binding *Phaseolus vulgaris* agglutinin E (PHA E), UniProtKB Accession Number: P05088.
*N*-linked tri/tetra-antennary binding *Phaseolus vulgaris* agglutinin L (PHA L), UniProtKB Accession Number: P05087.
Fucα1-6-specific *Pholiota squarrosa* lectin (PhoSL) purified as described in Kobayashi et al., 2012 (e.g., also SEQ ID NO: 58 herein), No UniProtKB Accession Number currently available.
GIcNAc binding *Phytolacca Americana* agglutinin (PWM), UniProtKB Accession Number: Q9AVB0.
α-mannose, α-glucose or Fucα1-6GlcNAc binding *Pisum sativum* lectin (PSL), UniProtKB Accession Number: P02867.
GalNAc or galactose binding *Psophocarpus tetragonolobus* lectin I (PTA I), UniProtKB Accession Number: O24313.
GalNAc or galactose binding *Psophocarpus tetragonolobus* lectin II (PTA II), UniProtKB Accession Number: Q9SM56.
Galactose binding *Ricinus communis* agglutinin I (RCA I), UniProtKB Accession Number: P06750.
Galactose and GalNAc binding *Ricinus communis* agglutinin II (RCA II), UniProtKB Accession Number: B9SPG3.
Sialic acid-binding immunoglobulin-like lectin 1 (Siglec 1), preferential binding of Neu5Acα2-3Galβ1-4Glc/GlcNAc over Neu5Acα2-6Galβ1-4Glc/GlcNAc by Siglec 1, UniProtKB Accession Number: Q9BZZ2.
Sialic acid-binding immunoglobulin-like lectin 4 (Siglec 4), preferential binding of Neu5Acα2-3Galβ1-4Glc/GlcNAc over Neu5Acα2-6Galβ1-4Glc/GlcNAc by Siglec 4, UniProtKB Accession Number: P20916.
Sialic acid-binding immunoglobulin-like lectin 8 (Siglec 8), preferential binding of Neu5Acα2-3Galβ1-4Glc/GlcNAc over Neu5Acα2-6Galβ1-4Glc/GlcNAc by Siglec 8, UniProtKB Accession Number: Q9NYZ4.
Sialic acid-binding immunoglobulin-like lectin 11 (Siglec 11), preferential binding of Neu5Acα2-8Neu5Ac (polysialic acid) by Siglec 11, UniProtKB Accession Number: Q96RL6.
GalNAcα1-3GalNAc binding Soybean agglutinin (SBA), UniProtKB Accession Number: P05046.
βGalNAc binding *Sophora japonica* agglutinin (SJA), UniProtKB Accession Number: P93535.
Neu5Ac(α2-6)Gal/GalNAc binding *Sambucus sieboldiana* agglutinin (SSA), purified as described in Kaku et al., 1996 (e.g., also SEQ ID NO: 59 herein), No UniProtKB Accession Number currently available.
GalNAc binding *Salvia sclarea* lectin, purified as described in Wu AM., 2005; No UniProtKB Accession Number currently available.
(GlcNAcβ1-4)₂₋₅ and Neu5Ac binding *Triticum vulgaris* agglutinin (TVA) = WGA - wheat germ agglutinin, UniProtKB Accession Number: P02876, P10968, or P10969.
Fucα1-2Gal_binding *Ulex europaeus* agglutinin (UEA), UniProtKB Accession Number: P22972.
GalNAc-serine binding *Vicia villosa* lectin (VVL), UniProtKB Accession Number: P56625.

The lectins of the present invention can be isolated and optionally purified using conventional methods known in the art. For example, when isolated from its natural source, the lectin can be purified to homogeneity on appropriate immobilized carbohydrate matrices and eluted by proper haptens. See, Goldstein & Poretz (1986) In The lectins. Properties, functions and applications in biology and medicine (ed. Liener et al.), pp. 33-247. Academic Press, Orlando, Fla.; Rudiger (1993) In Glycosciences: Status and perspectives (ed. Gabius & Gabius), pp. 415-438. Chapman and Hall, Weinheim, Germany. Alternatively, the lectin can be produced by recombinant methods according to established methods. See Streicher & Sharon (2003) Methods Enzymol. 363:47-77. As yet another alternative, lectins can be generated using standard peptide synthesis technology or using chemical cleavage methods well-known in the art based on the amino acid sequences of known lectins or the lectin disclosed herein (e.g., US 9169327 B2). Another alternative can be artificial lectins prepared by chemical modification of any above specified lectins (see YW. Lu, C.W. Chien, P.C. Lin, L.D. Huang, C.Y Chen, S.W. Wu, C.L. Han, K.H. Khoo, C.C. Lin, YJ. Chen, BAD-Lectins: Boronic Acid-Decorated Lectins with Enhanced Binding Affinity for the Selective Enrichment of Glycoproteins, Analytical Chemistry, 85 (2013) 8268-8276.).

### Brief description of the drawings

**Figure 1****:** PSA with the glycan composition (A) from healthy men and from (B) PCa patients (one from quite many different possible glycan structures is shown). Assay protocol for detection of (C) PSA level, (D) PSA glycoprofiling in an ELLA format, (E) PSA glycoprofiling in a MELLA (i.e., magnetic ELLA) format. Abbreviations: PSA - prostate specific antigen; Ab1 - anti-PSA antibody 1 is labelled with horseradish peroxidase (HRP); Ab2 - anti- PSA antibody 2, L - lectin, HRP - horseradish peroxidase.
**Figure 2****:** ROC for PSA glycoprofiling in a magnetic ELLA format vs. PSA in ELISA format with two different lectins - AAL (left), Con A (right); BPH vs. PCa samples.
**Figure 3****:** ROC for PSA glycoprofiling in a magnetic ELLA format vs. PSA in ELISA format with two different lectins - MAA-II (left) and SNA-I (right); BPH vs. PCa samples.
**Figure 4****:** ROC for PSA glycoprofiling in a magnetic ELLA format vs. PSA in ELISA format with WFA lectin (left) and four different lectins - AAL (negative predictor), Con A (negative predictor), MAA-II (positive predictor) and SNA-I (negative predictor); BPH vs. PCa samples.
**Figure 5****:** ROC for PSA glycoprofiling in a magnetic ELLA format vs. PSA using double biomarkers. BPH vs. PCa samples.
**Figure 6****:** ROC for PSA glycoprofiling in a magnetic ELLA format vs. PSA using triple biomarkers. BPH vs. PCa samples.
**Figure 7****:** ROC for PSA glycoprofiling in a magnetic ELLA format vs. PSA using single biomarkers (AAL and Con A). PCa- vs. PCa+ samples.
**Figure 8****:** ROC for PSA glycoprofiling in a magnetic ELLA format vs. PSA using single biomarkers (MAA II and SNA-I). PCa- vs. PCa+ samples. PCa- stands for prostate cancer without metastasis and PCa+ means prostate cancer with metastasis.
**Figure 9****:** Box plots showing ability to differentiate two different biomarkers (PSA and MAA) on various types of samples including BPH, PCa+, PCa- and PCa (combined PCa+ and PCa-). PCa- stands for prostate cancer without metastasis and PCa+ means prostate cancer with metastasis.
**Figure 10****:** Box plots showing ability to differentiate samples with PSA biomarker on various types of samples including BPH, PCa+, PCa- and PCa (combined PCa+ and PCa-). PCa- stands for prostate cancer without metastasis and PCa+ means prostate cancer with metastasis.
**Figure 11****:** ROC curve for analysis of human serum samples to distinguish BPH (benign prostatic hyperplasia) from PCa (prostate cancer) patients using magnetic particles (MPs) without immobilised antibody.
**Figure 12****:** ROC curve for analysis of human serum samples to distinguish BPH from PCa patients using magnetic particles with immobilised antibody and with PSA released from MPs for subsequent lectin-based glycoprofiling.
**Figure 13****:** ROC curve for analysis of human serum samples to distinguish BPH from PCa patients using antibodies immobilised on ELISA plate with subsequent lectin-based glycoprofiling.

### Detailed description of the invention

### Definitions

Carbohydrate abbreviations as used herein include: "Neu5Ac" for *N*-acetylneuraminic acid; "Fuc" for fucose, "GalNAc" for *N*-acetylgalactosamine; "GIcNAc" for *N*-acetylglucosamine; "Gal" for galactose (e.g., Varki A, Cummings RD, Esko JD, Freeze HH, Stanley P, Bertozzi CR, Hart GW, E. ME., Essentials of Glycobiology, 2nd edition, Cold Spring Harbor Laboratory Press, Cold Spring Harbor (NY), 2009).

Furthermore, as used herein the following terms are defined below:
"core fucose" means fucose is linked via an α-glycosidic bond of its C1 atom to the C6 atom of *N*-acetylglucosamine,
"antennary fucose" means fucose is linked via an α-glycosidic bond of its C1 atom to the C3 atom of *N*-acetylglucosamine or fucose is linked via an α-glycosidic bond of its C1 atom to the C2 atom of neighbouring fucose,
"Fucα1-6GlcNAc-*N*-Asn containing *N*-linked oligosaccharides" means oligosaccharides which have fucose linked via a α-glycosidic bond of its C1 atom to the C6 atom of *N-*acetylglucosamine, which is linked to asparagine via *N*-glycosidic bond,
"Fucα1-6/3GlcNAc" means fucose is linked via a α-glycosidic bond of its C1 atom to the C6 (C3) atom of *N*-acetylglucosamine,
"α-L-Fuc" means α-L-fucose,
"Fucα1-2Galβ1-4(Fucα1-3)GlcNAc" means fucose is linked via an α-glycosidic bond of its C1 atom to the C2 atom of galactose, which is linked via an β glycosidic bond of its C1 atom to the C4 atom of *N*-acetylglucosamine; at the same time second fucose is linked via an α-glycosidic bond of its C1 atom to the C3 atom of *N*-acetylglucosamine,
"Fucα1-2Gal" means fucose is linked via a α-glycosidic bond of its C1 atom to the C2 atom of galactose,
"Fucα1-6GlcNAc" means fucose is linked via a α-glycosidic bond of its C1 atom to the C6 atom of *N*-acetylglucosamine,
"Manβ1-4GlcNAcβ1-4GlcNAc" means mannose is linked via a β-glycosidic bond of its C1 atom to the C4 atom of N-acetylglucosamine, which is linked via a β-glycosidic bond of its C1 atom to the C4 atom of *N*-acetylglucosamine,
"branched *N*-linked hexa-saccharide" means non-linear glycan composed of six carbohydrates linked to asparagine by *N*-glycosidic bond
"Manα1-3Man" means mannose is linked via a α-glycosidic bond of its C1 atom to the C3 atom of mannose,
"α-D-Man" means α-D-mannose,
"(GlcNAcβ1-4)₂₋₄" means *N*-acetylglucosamine is linked via a β-glycosidic bond of its C1 atom to the C4 atom of *N*-acetylglucosamine repeatedly,
"Galβ1-4GlcNAc" means galactose is linked via a β-glycosidic bond of its C1 atom to the C4 atom of N-acetylglucosamine,
"GlcNAcα1-4Galβ1-4GlcNAc" means *N*-acetylglucosamine is linked via a α-glycosidic bond of its C1 atom to the C4 atom of galactose, which is linked via a β-glycosidic bond of its C1 atom to the C4 atom of N-acetylglucosamine,
"*N*-acetylglucosamine" means amide between glucosamine and acetic acid ,
"(GlcNAcβ1-4)₂₋₅" means *N*-acetylglucosamine is linked via a β-glycosidic bond of its C1 atom to the C4 atom of *N*-acetylglucosamine repeatedly,
"Neu5Ac" (or sialic acid) means *N*-acetylneuraminic acid,
"Galβ1-3GalNAc-serine/threonine" means galactose is linked via a β-glycosidic bond of its C1 atom to the C3 atom of *N*-acetylglucosamine, which is linked to serine/threonine,
"Galα1-3GalNAc" means galactose is linked via a α-glycosidic bond of its C1 atom to the C3 atom of *N*-acetylgalactosamine,
"Galβ1-6Gal" means galactose is linked via a β-glycosidic bond of its C1 atom to the C6 atom of galactose,
"Galβ1-4GlcNAc" means galactose is linked via a β-glycosidic bond of its C1 atom to the C3 atom of *N*-acetylglucosamine,
"Galβ1-3GalNAc" means galactose is linked via a β-glycosidic bond of its C1 atom to the C3 atom of *N*-acetylgalactosamine,
"GalNAcα1-3GalNAc" means *N*-acetylgalactosamine is linked via a α-glycosidic bond of its C1 atom to the C3 atom of *N*-acetylgalactosamine,
"GalNAcα1-3Gal" means *N*-acetylgalactosamine is linked via a α-glycosidic bond of its C1 atom to the C3 atom of galactose,
"GalNAcα/β1-3/4Gal" means *N*-acetylgalactosamine is linked via a α- or β-glycosidic bond of its C1 atom to the C3 or C4 atom of galactose,
"α-GalNAc" means amide between α-galactosamine and acetic acid,
"GalNAcβ1-4Gal" means *N*-acetylgalactosamine is linked via a β-glycosidic bond of its C1 atom to the C4 atom of galactose,
"GalNAcα1-3(Fucα1-2)Gal" means *N*-acetylgalactosamine is linked via a α-glycosidic bond of its C1 atom to the C3 atom of galactose, at the same time fucose is linked via a α-glycosidic bond of its C1 atom to the C2 atom of galactose,
"GalNAcα1-2Gal" means *N*-acetylgalactosamine is linked via a α-glycosidic bond of its C1 atom to the C3 atom of galactose,
"GalNAcα1-3GalNAc" means *N*-acetylgalactosamine is linked via a α-glycosidic bond of its C1 atom to the C3 atom of *N*-acetylgalactosamine,
"GalNAcβ1-3/4Gal" means *N*-acetylgalactosamine is linked via a β-glycosidic bond of its C1 atom to the C3 or C4 atom of galactose,
"GalNAc-Ser/Thr" (or Tn antigen,) means *N*-acetylgalactosamine is linked to serine/threonine via *O*-glycosidic bond,
"Galβ1-3GalNAc-Ser/Thr" (T antigen or Thomsen-Friedenreich antigen) means galactose is linked via a β-glycosidic bond of its C1 atom to the C3 atom of *N*-acetylgalactosamine, which is linked to serine/threonine via *O*-glycosidic bond,
"GalNAcβ1-4GlcNAc" (or LacdiNAc) means *N*-acetylgalactosamine is linked via a β-glycosidic bond of its C1 atom to the C4 atom of *N*-acetylglucosamine,
"α2-3Neu5Ac" (or α2-3-linked sialic acid) means *N*-acetylneuraminic acid is linked via a α-glycosidic bond of its C2 atom to the C3 atom of a neighbouring saccharide,
"α2-6Neu5Ac" (or α2-6-linked sialic acid) means *N*-acetylneuraminic acid is linked via a α-glycosidic bond of its C2 atom to the C6 atom of a neighbouring saccharide,
"α2-8Neu5Ac" (or α2-8-linked sialic acid) means *N*-acetylneuraminic acid is linked via a α-glycosidic bond of its C2 atom to the C8 atom of a neighbouring *N*-acetylneuraminic acid,
"Neu5Acα4/9-O-Ac-Neu5Ac" means *N*-acetylneuraminic acid is linked via a α-glycosidic bond of its C4 atom to the C9 atom of a neighbouring *O*-acetyl *N*-acetylneuraminic acid,
"Neu5Acα2-3Galβ1-4Glc/GlcNAc" means *N*-acetylneuraminic acid is linked via a α-glycosidic bond of its C2 atom to the C3 atom of galactose, which is linked via β-glycosidic bond of its C1 atom to the C4 atom of glucose or *N*-acetylglusosamine,
"Neu5Acα2-6Gal/GalNAc" means *N*-acetylneuraminic acid is linked via a α-glycosidic bond of its C2 atom to the C6 atom of galactose or *N*-acetylgalactosamine,
"*N*-linked bi-antennary" means non-linear glycan with two antennas (carbohydrate chains) linked to asparagine by *N*-glycosidic bond,
"*N*-linked tri/tetra-antennary" means non-linear glycan with three/tetra antennas (carbohydrate chains) linked to asparagine by *N*-glycosidic bond,
"branched β1-6GlcNAc" means *N*-acetylglusosamine is linked via a β-glycosidic bond of its C1 atom to the C6 atom of neighbouring saccharide,
"Galα1-3(Fucα1-2)Galβ1-3/4GlcNAc" means galactose is linked via a α-glycosidic bond of its C1 atom to the C3 atom of galactose, which is linked via a β-glycosidic bond of its C1 atom to the C3 or C4 atom of *N*-acetylglusosamine; at the same time fucose is linked via a α-glycosidic bond of its C1 atom to the C2 atom of *N*-acetylglusosamine,
"Galβ1-3(Fucα1-4)GlcNAc" means galactose is linked via a β-glycosidic bond of its C1 atom to the C3 atom of *N*-acetylglusosamine; at the same time fucose is linked via α-glycosidic bond of its C1 atom to the C4 atom of *N*-acetylglusosamine,
"NeuAcα2-3Galβ1-3(Fucα1-4)GlcNAc" means *N*-acetylneuraminic acid is linked via a α-glycosidic bond of its C2 atom to the C3 atom of galactose, which is linked via β-glycosidic bond of its C1 atom to the C3 atom *N*-acetylglusosamine; at the same time fucose is linked via a α-glycosidic bond of its C1 atom to the C4 atom of *N-*acetylglusosamine,
"Fucα1-2Galβ1-3(Fucα1-4)GlcNAc" means fucose is linked via a α-glycosidic bond of its C1 atom to the C2 atom of galactose, which is linked via β-glycosidic bond of its C1 atom to the C3 atom *N*-acetylglusosamine; at the same time second fucose is linked via αglycosidic bond of its C1 atom to the C4 atom of *N*-acetylglusosamine,
"Galβ1-4(Fucα1-3)GlcNAc" means galactose is linked via β-glycosidic bond of its C1 atom to the C4 atom of *N*-acetylglusosamine; at the same time fucose is linked via α-glycosidic bond of its C1 atom to the C3 atom of *N*-acetylglusosamine,
"NeuAcα2-3Galβ1-4(Fucα1-3)GlcNAc" means *N*-acetylneuraminic acid is linked via α-glycosidic bond of its C2 atom to the C3 atom of galactose, which is linked via β-glycosidic bond of its C1 atom to the C4 atom *N*-acetylglusosamine; at the same time fucose is linked via α-glycosidic bond of its C1 atom to the C3 atom of *N*-acetylglusosamine,
"Fucα1-2Galβ1-4(Fucα1-3)GlcNAc" means fucose is linked via α-glycosidic bond of its C1 atom to the C2 atom of galactose, which is linked via β-glycosidic bond of its C1 atom to the C4 atom *N*-acetylglusosamine; at the same time second fucose is linked via α-glycosidic bond of its C1 atom to the C3 atom of *N*-acetylglusosamine,
"high mannose" means glycan containing more than three mannose units,
"sialyl Lewis^{a}" (sialyl Le^{a}) antigen is Neu5Acα,2-3/6Galβ1-3(Fucα1-4)GlcNAc meaning *N-*acetylneuraminic acid is linked via α-glycosidic bond of its C2 atom to the C3 or C6 atom of galactose, which is linked via β-glycosidic bond of its C1 atom to the C3 atom *N-*acetylglusosamine; at the same time fucose is linked via α-glycosidic bond of its C1 atom to the C4 atom of *N*-acetylglusosamine,
"sialyl Lewis^{x} " (sialyl Le^{x}) antigen is Neu5Acα2-3/6Galβ1-4(Fucα1-3)GlcNAc meaning *N-*acetylneuraminic acid is is linked via α-glycosidic bond of its C2 atom to the C3 or C6 atom of galactose, which is linked via β-glycosidic bond of its C1 atom to the C4 atom *N-*acetylglusosamine; at the same time fucose is linked via α-glycosidic bond of its C1 atom to the C3 atom of *N*-acetylglusosamine,
"Lewis^{x} " (Le^{x}) antigen is "Galβ1-4(Fucα1-3)GlcNAc" meaning galactose is linked via β-glycosidic bond of its C1 atom to the C4 atom of *N*-acetylglusosamine; at the same time fucose is linked via α-glycosidic bond of its C1 atom to the C3 atom of *N-*acetylglusosamine,
"sialyl Tn antigen" is "Neu5Acα2-3/6GalNAc-Ser/Thr" meaning *N*-acetylneuraminic acid is is linked via α-glycosidic bond of its C2 atom to the C3 or C6 atom of *N-*acetylgalactosamine, which is linked to serine/threonine via *O*-glycosidic bond,
"sialyl T antigen" is "Neu5Acα2-3/6Galβ1-3GalNAc-Ser/Thr" meaning *N*-acetylneuraminic acid is linked via α-glycosidic bond of its C2 atom to the C3 or C6 atom of galactose, which is linked via β-glycosidic bond of its C1 atom to the C3 atom of *N*-acetylgalactosamine, which is linked to serine/threonine via *O*-glycosidic bond,
"Lewis^{y} " (Le^{y}) antigen is "Fucα1-2Galβ1-4(Fucα1-3)GlcNAc" meaning fucose is linked via α-glycosidic bond of its C1 atom to the C2 atom of galactose, which is linked via β-glycosidic bond of its C1 atom to the C4 atom of *N*-acetylglusosamine; at the same time second fucose is linked via α-glycosidic bond of its C1 atom to the C3 atom of *N-*acetylglusosamine,
"sulfated core1 glycan" is a glycan based on suflated extended form of T antigen,
"core 2 glycan" is a glycan based on an extended form of Galβ1-3(GlcNAcβ1-6)GalNAc-Ser/Thr meaning an extended form of glycan having galactose linked via β-glycosidic bond of its C1 atom to the C3 atom of *N*-acetylgalactosamine, at the same time *N-*acetylglusosamine is linked via β-glycosidic bond of its C1 atom to the C6 atom of *N-*acetylgalactosamine, which is linked to serine/threonine
"Lewis^{a} " (Le^{a}) antigen is Galβ1-3(Fucα1-4)GlcNAc meaning galactose is linked via β-glycosidic bond of its C1 atom to the C3 atom *N*-acetylglusosamine; at the same time fucose is linked via α-glycosidic bond of its C1 atom to the C4 atom of *N*-acetylglusosamine,
"(GlcNAcβ1-4)ₙ" means *N*-acetylglusosamine is linked via β-glycosidic bond of its C1 atom to the C4 atom of *N*-acetylglusosamine repeatedly,
"β-D-GIcNAc" means amide between β-D-glucosamine and acetic acid,
"GalNAc" means amide between galactosamine and acetic acid,
"Gal-GlcNAc" means galactose is linked to *N*-acetylglusosamine via non-specified linkage,
"GIcNAc" means amide between glucosamine and acetic acid.
"Galα1-3Gal" means galactose is linked via α-glycosidic bond of its C1 atom to the C3 atom of galactose,
"Galβ1-3GalNAc" means galactose is linked via β-glycosidic bond of its C1 atom to the C3 atom of *N*-acetylgalactosamine,
"α-Gal" means α-galactose,
"α-GalNAc" means amide between α -D-galactosamine and acetic acid,
"(GlcNAc)ₙ" means *N*-acetylglusosamine is linked to *N*-acetylglusosamine via non-specified linkage,
"branched (LacNAc)ₙ" is branched and repeated form of Galβ1,4-GlcNAc meaning a branched and repeated form of galactose linked via β-glycosidic bond of its C1 atom to the C4 atom of N-acetylglucosamine.

The term "glycoprotein" (or "glycosylated protein") as used herein means a protein containing one or more *N*-, *O*-, *S*- or *C*- covalently linked carbohydrates of various types, e.g., ranging from monosaccharides to branched polysaccharides (including their modifications such as sulfo- or phospho- group attachment). N-linked glycans are carbohydrates bound to -NH₂ group of asparagine. *O*-linked glycans are carbohydrates bound to -OH group of serine, threonine, or hydroxylated amino acids. S-linked glycans are carbohydrates bound to -SH group of cysteine. *C*-linked glycans are carbohydrates bound to tryptophan *via* C-C bond.

The term "carbohydrates" means compounds (e.g., such as aldoses and ketoses) having the stoichiometric formula Cₙ(H₂O)ₙ. The generic term "carbohydrate" includes monosaccharides, oligosaccharides and polysaccharides as well as substances derived from monosaccharides by reduction of the carbonyl group (alditols), by oxidation of one or more terminal groups to carboxylic acids, or by replacement of one or more hydroxy group(s) by a hydrogen atom, an amino group, thiol group or similar groups. It also includes derivatives of these compounds.

The term "glycoprofile of a protein" means a carbohydrate structure of a protein, e.g., composition and/or structure of covalently linked carbohydrates, e.g., quantity, presence, or absence of covalently linked carbohydrates.

The term "glycoprofiling" means determining a carbohydrate structure (e.g., composition and/or structure of covalently linked carbohydrates, e.g., quantity, presence, or absence of covalently linked carbohydrates) of a glycoconjugate, such as a glycoprotein, glycolipid, or a proteoglycan.

The term "DART" means dual-affinity re-targeting antibodies, which are bispecific, antibody-based molecules that can bind 2 distinct cell-surface molecules simultaneously (e.g., Sung JAM et al., 2015).

The term "adnectine" (or "monobody") means synthetic binding protein capable of binding antigens e.g., they can be constructed using a fibronectin type III domain (FN3) as a molecular scaffold. Monobodies are a simple and robust alternative to antibodies for creating target-binding proteins.

The term "single-domain antibody" (or "nanobody") means an antibody fragment consisting of a single monomeric variable antibody domain.

The term "FN3 scaffold" means fibronectin type III domain (FN3) scaffold which can be used as non-antibody scaffold for generating binding proteins (e.g., antigen binding proteins) (e.g., Koide A. et al., 2012).

The term "affibody" refers to a class of engineered affinity proteins that can bind to target proteins or peptides with high affinity imitating monoclonal antibodies and are therefore a member of the family of antibody mimetics (e.g., Löfblom J et al., 2010).

The term "anticalin" refers to an artificial protein able to bind to antigens, either to proteins or to small molecules. Anticalins are not structurally related to antibodies, which makes them a type of antibody mimetic. Preferably, "anticalin" is a protein derived from a lipocalin (also known as cytosolic fatty acid binding protein), which is genetically engineered to modify its binding properties. Anticalins have the advantage of a monoclonal antibody's specificity for small lipid molecules (e.g., steroids, bilins, retinoids and lipids), better tissue penetration and thermostability, but without the large size (e.g., they are 8-fold smaller) and can also be batched in *E coli*, eliminating the need for animal extraction.

The term "avimer" (e.g., short for avidity multimer) refers to an artificial protein that is able to specifically bind to antigens via multiple binding sites. Amimer is not structurally related to antibody and is classified as a type of antibody mimetic.

The term "cyclic peptide" refers to polypeptide chains which contain a circular sequence of bonds. The term "bicyclic peptide" (e.g., such as the amatoxin amanitin and the phallotoxin phalloidin) refers to a cyclic peptide containing a bridging group (e.g., thioether or disulfide bond), generally between two of the polypeptide side chains. In the amatoxins, this bridge is formed as a thioether between the Trp and Cys residues. Other bicyclic peptides include echinomycin, triostin A, and Celogentin C. There are also cyclic peptide hormones, which are cyclized through a disulfide bond between two cysteines, for example somatostatin and oxytocin. For example, a "bicyclic peptide" screening and production method can make use of a phage library displaying peptides containing three Cys residues. The phages are treated under mild conditions with Tris-(bromomethyl)benzene, which reacts with all three cysteines, forming two peptide loops of six amino acids linked to the benzene ring (e.g., Mund T et al., 2014).

The term "DARPins" (an acronym for "designed ankyrin repeat proteins") refers to engineered antibody mimetic proteins exhibiting highly specific and high-affinity target protein binding. They are derived from natural ankyrin proteins, which are responsible for diverse functions such as cell signaling, regulation and structural integrity of the cell. DARPins consist of at least three, repeat motifs proteins, and usually consist of four or five. Their molecular mass is about 14 or 18 kDa (kilodaltons) for four- or five-repeat DARPins, respectively (e.g., Plückthun A, 2015; Rasool M et al., 2017).

The term "Kunitz domain" refers to an active domain of a protein capable of inhibiting the function of protein degrading enzyme (e.g., a protein domain characteristic of inhibitors of the S1 serine peptidase family. Preferred examples include aprotinin, trypstatin, a rat mast cell inhibitor of trypsin, and tissue factor pathway inhibitor (TFPI).

The term "Obody" refers to a single-domain protein module (e.g., single-domain scaffold) that can bind to a specific target molecule (e.g., protein, carbohydrate, nucleic acid and small-molecule ligands) (e.g., Steemson JD et al., 2014).

The term "aptamer" refers to oligonucleotide or peptide molecules that bind to a specific target molecule.

The term "agglutinin" means any substance causing agglutination (i.e., clumping together) of cells, particularly a specific antibody formed in the blood in response to the presence of an invading agent, or lectins having such effect. More specifically the term "agglutinin" refers to glycan binding protein (lectin, see 153), when used herein.

The term "glycan" refers to compounds consisting of monosaccharides linked glycosidically and may also refer to carbohydrate portion of a glycoconjugate, such as a glycoprotein, glycolipid, or a proteoglycan, even if the carbohydrate is only a monosaccharide or an oligosaccharide.

The term "lectin" when used herein refers to a carbohydrate-binding protein. A lectin can be highly specific for a carbohydrate moiety or carbohydrate moieties (e.g., it reacts specifically with terminal glycosidic residues of other molecules such as a glycan/s of a glycoprotein (e.g., branching sugar molecules of glycoproteins, e.g., such as target polypeptides within the meaning of the present invention and biomarkers as described in Table 1 herein). Lectins are commonly known in the art. A skilled person is readily available to determine which lectin may be used for binding a carbohydrate moiety or carbohydrate moieties of interest, e.g. a carbohydrate moiety or carbohydrate moieties of a glycan attached to a protein. Preferred lectins applied in the context of the present invention are described herein. Also included by the term "lectin" are Siglecs (sialic acid-binding immunoglobulin-like lectins). Notably, the term "lectin" when used herein also refers to glycan-binding antibodies. Accordingly, the term "lectin" when used herein encompasses lectins, Siglecs as well as glycan-binding antibodies.

An "antibody" when used herein is a protein comprising one or more polypeptides (comprising one or more binding domains, preferably antigen binding domains) substantially or partially encoded by immunoglobulin genes or fragments of immunoglobulin genes. Preferably, an antibody which is directed against a protein whose glycoprofile is determined as described herein, is not directed against a glycan attached to said protein. Put differently, an antibody which is directed against a protein whose glycoprofile is determined as described herein is preferably directed against the protein as such, i.e., is directed against an epitope within the amino acid sequence of said protein. The epitope may be a linear or conformational epitope. It may be a continuous or discontinuous epitope. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, lgA1, and IgA2, with IgG being preferred in the context of the present invention. An antibody of the present invention is also envisaged which has an IgE constant domain or portion thereof that is bound by the Fc epsilon receptor I. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen, but can exhibit various effector functions, such as participation of the antibody dependent cellular cytotoxicity (ADCC). If an antibody should exert ADCC, it is preferably of the IgG1 subtype, while the IgG4 subtype would not have the capability to exert ADCC.

The term "antibody" also includes, but is not limited to, but encompasses monoclonal, monospecific, poly- or multi-specific antibodies such as bispecific antibodies, humanized, camelized, human, single-chain, chimeric, synthetic, recombinant, hybrid, mutated, grafted, and in vitro generated antibodies, with chimeric or humanized antibodies being preferred. The term "humanized antibody" is commonly defined for an antibody in which the specificity encoding CDRs of HC and LC have been transferred to an appropriate human variable frameworks ("CDR grafting"). The term "antibody" also includes scFvs, single chain antibodies, diabodies or tetrabodies, domain antibodies (dAbs) and nanobodies. In terms of the present invention, the term "antibody" shall also comprise bi-, tri- or multimeric or bi-, tri- or multifunctional antibodies having several antigen binding sites. Said term also includes antigen binding portion(s). Also included by the term "antibody" is FN3 scaffold, adnectin, affibody, anticalin, avimer, a bicyclic peptide, DARPin, a Kunitz domain, an Obody or an aptamer, such as a DNA, RNA or peptide aptamer.

Preferred antibodies of the present invention include, but are not limited to, an anti-PSA, anti-AFP, anti-MUC16, anti-WFDC2, anti-MUC1, anti-ERBB2, anti-CEACAM5, anti-FUT3 or anti-TG antibodies etc. Further preferred antibodies relating to the present invention are shown in Table 1 below.

Furthermore, the term "antibody" as employed in the invention also relates to derivatives of the antibodies (including fragments) described herein. A "derivative" of an antibody comprises an amino acid sequence which has been altered by the introduction of amino acid residue substitutions, deletions or additions. Additionally, a derivative encompasses antibodies which have been modified by a covalent attachment of a molecule of any type to the antibody or protein. Examples of such molecules include sugars, PEG, hydroxyl-, ethoxy-, carboxy- or amine-groups but are not limited to these. In effect the covalent modifications of the antibodies lead to the glycosylation, pegylation, acetylation, phosphorylation, amidation, without being limited to these.

The antibody of the present invention is preferably an "isolated" antibody. "Isolated" when used to describe antibodies disclosed herein, means an antibody that has been identified, separated and/or recovered from a component of its production environment. Preferably, the isolated antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

As used herein the term "antigen binding portion" refers to a fragment of immunoglobulin (or intact antibody), and encompasses any polypeptide comprising an antigen-binding fragment or an antigen-binding domain. Preferably, the fragment such as Fab, F(ab'), F(ab')₂, Fv, scFv, Fd, disulfide-linked Fvs (sdFv), and other antibody fragments that retain antigen-binding function as described herein (e.g., a single chain antibody fragment (scAb)). Typically, such fragments would comprise an antigen-binding domain and have the same properties as the antibodies described herein.

Preferred antigen binding portions of antibodies of the present invention include, but are not limited to, antigen binding portions of an anti-PSA, anti-AFP, anti-MUC16, anti-WFDC2, anti-MUC1, anti-ERBB2, anti-CEACAM5, anti-FUT3 or anti-TG antibodies. Further preferred antibodies relating to the present invention are shown in Table 1 below.

As used herein, the term "specifically binds" refers to antibodies or fragments or derivatives thereof that specifically bind to a target glycoprotein or target polypeptide and do not specifically bind to another protein or polypeptide. The antibodies or fragments or derivatives thereof according to the invention bind to their respective targets through the variable domain of the antibody.

Preferred anti-glycoprotein antibody or the antigen binding portions thereof specifically bind to a target polypeptide or target glycoprotein comprising a polypeptide selected from the group consisting of (e.g., target glycoprotein comprises said polypeptide): i) Prostate-specific antigen (PSA), preferably SEQ ID NOs: 1, 2, 3, 4, 5 or 6; further preferably SEQ ID NO: 6; ii) Alpha-fetoprotein (AFP), preferably SEQ ID NOs: 11 or 12; further preferably SEQ ID NO: 12; iii) Mucin-16 (MUC16), preferably SEQ ID NO: 13; vi) WAP four-disulfide core domain protein 2 (WFDC2), preferably SEQ ID NOs: 14, 15, 16, 17, 18 or 19; further preferably SEQ ID NO: 19; v) Mucin-1 (MUC1), preferably SEQ ID NOs: 20, 21, 22, 23, 24, 25, 26, 27, 28, 29, 30, 31, 32, 33, 34, 35, 36 or 37; further preferably SEQ ID NO: 37; vii) Receptor tyrosine-protein kinase erbB-2 (ERBB2), preferably SEQ ID NOs: 38, 39, 40, 41, 42, 43, or 44; further preferably SEQ ID NO: 44; viii) Carcinoembryonic antigen-related cell adhesion molecule 5 (CEACAM5), preferably SEQ ID NOs: 45, 46, or 47; further preferably SEQ ID NO: 47; ix) Galactoside 3(4)-L-fucosyltransferase (FUT3), preferably SEQ ID NO: 48; x) Thyroglobulin (TG), preferably SEQ ID NOs: 49, 50, or 51; further preferably SEQ ID NO: 51. Further preferred antibodies relating to the present invention are shown in Table 1 below.

The term "epitope" also refers to a site on an antigen (in the context of the present invention, the antigen is a glycoprotein) to which the antibody molecule binds. Preferably, an epitope is a site on a molecule (in the context of the present invention, the antigen is a glycoprotein) against which an antibody or antigen binding portion thereof, preferably an antibody will be produced and/or to which an antibody will bind. For example, an epitope can be recognized by an antibody or antigen binding portion thereof. The epitope may be a linear or conformational epitope. It may be a continuous or discontinuous epitope. A "linear epitope" is an epitope where an amino acid primary sequence comprises the epitope recognized. A linear epitope typically includes at least 3, and more usually, at least 5, for example, about 8 to about 10 amino acids in a unique sequence.

Specific binding is believed to be affected by specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the antigen-interaction-site with its specific antigen may alternatively result in the initiation of a signal, e.g. due to the induction of a change of the conformation of the antigen, an oligomerization of the antigen, etc. A preferred example of a binding domain in line with the present invention is an antibody.

Typically, binding is considered specific when the binding affinity is higher than 10⁻⁶M. Preferably, binding is considered specific when binding affinity is about 10⁻¹¹ to 10⁻⁸ M (K_{D}), preferably of about 10⁻¹¹ to 10⁻⁹ M. If necessary, nonspecific binding can be reduced without substantially affecting specific binding by varying the binding conditions.

In case of binding of glycans to lectins the binding affinity is preferably in the range 10⁻³ to 10⁻⁶ (K_{D}). The methods of measuring corresponding K_{D}s for binding of glycans to lectins are known in the art and are readily available to a person skilled in the art.

Whether the antibody or antigen binding portion thereof specifically reacts as defined herein above can easily be tested, inter alia, by comparing the reaction of said antibody or antigen binding portion thereof respective target glycoprotein with the reaction of said antibody or antigen binding portion thereof with (an) another non-target protein(s).

The term polypeptide" is equally used herein with the term "protein". Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is affected e.g. by post-translational modifications like glycosylation, acetylation, phosphorylation and the like. Such modifications are well known in the art.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

"Polyclonal antibodies" or "polyclonal antisera" refer to immune serum containing a mixture of antibodies specific for one (monovalent or specific antisera) or more (polyvalent antisera) antigens which may be prepared from the blood of animals immunized with the antigen or antigens.

Furthermore, the term "antibody" as employed in the invention also relates to derivatives or variants of the antibodies described herein which display the same specificity as the described antibodies. Examples of "antibody variants" include humanized variants of non-human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260).

The terms "antigen-binding domain", "antigen binding portion", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH> are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies and is not to be construed as requiring production of the antibody by any method. For example, the monoclonal antibodies to be used in accordance with the present invention may be made by the hybridoma method first described by Kohler et al., Nature, 256: 495 (1975), or may be made by recombinant DNA methods (see, e.g., U. S. Patent No. 4,816, 567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

Furthermore, the term "monoclonal antibody" as employed in the invention also relates to specific monoclonal antibodies as produced by the company DB Biotech (http://www.dbbiotech.com/about-us.html), said method including *in vitro* cloning technology enabling production of a pure immunoglobulin fraction corresponding to a single clone of B lymphocytes, wherein the obtained immunoglobulin recognizes only one single linear epitope on the antigen molecule.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain (s) is (are) identical with or homologous to corresponding sequences in antibodies derived from another species or belonging to another antibody class or subclass, as well as fragments of such antibodies, so long as they exhibit the desired biological activity (U. S. Patent No. 4,816, 567; Morrison et al., Proc. Natl. Acad. Sci. USA, 81: 6851-6855 (1984)). Chimeric antibodies of interest herein include "primitized" antibodies comprising variable domain antigen-binding sequences derived from a non-human primate (e.g., Old World Monkey, Ape etc.) and human constant region sequences.

"Humanized" forms of non-human (e.g., murine) antibodies are chimeric immunoglobulins, immunoglobulin chains or fragments thereof (such as Fv, Fab, Fab', F (ab') 2 or other antigen-binding subsequences of antibodies) of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, "humanized antibodies" as used herein may also comprise residues which are found neither in the recipient antibody nor the donor antibody. These modifications are made to further refine and optimize antibody performance. The humanized antibody optimally also will comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

The term "human antibody" includes antibodies having variable and constant regions corresponding substantially to human germline immunoglobulin sequences known in the art, including, for example, those described by Kabat et al. (See Kabat, et al. (1991) loc. cit.). The human antibodies of the invention may include amino acid residues not encoded by human germline immunoglobulin sequences (e.g., mutations introduced by random or site-specific mutagenesis in vitro or by somatic mutation in vivo), for example in the CDRs, and in particular, CDR3. The human antibody can have at least one, two, three, four, five, or more positions replaced with an amino acid residue that is not encoded by the human germline immunoglobulin sequence.

As used herein, "in vitro generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, e.g., Songsivilai & Lachmann, Clin. Exp. Immunol. 79:315-321 (1990); Kostelny et al., J. Immunol. 148, 1547-1553 (1992). In one embodiment, the bispecific antibody comprises a first binding domain polypeptide, such as a Fab' fragment, linked via an immunoglobulin constant region to a second binding domain polypeptide.

Antibodies described herein may be used for forming bispecific molecules. An anti-PSA antibody, or antigen-binding portions thereof, can be derivatized or linked to another functional molecule, e.g., another peptide or protein (e.g., another antibody or ligand for a receptor) to generate a bispecific molecule that binds to at least two different binding sites or target molecules. The antibody described herein may in fact be derivatized or linked to more than one other functional molecule to generate multispecific molecules that bind to more than two different binding sites and/or target molecules; such multispecific molecules are also intended to be encompassed by the term "bispecific molecule" as used herein. To create a bispecific molecule described herein, an antibody described herein can be functionally linked (e.g., by chemical coupling, genetic fusion, noncovalent association or otherwise) to one or more other binding molecules, such as another antibody, antibody fragment, peptide or binding mimetic, such that a bispecific molecule results.

Immunoconjugates and antibody derivatives. Antibodies described herein can be used for diagnostic purposes, including sample testing and in vivo imaging, and for this purpose the antibody (or binding fragment thereof) can be conjugated to an appropriate detectable agent, to form an immunoconjugate. For diagnostic purposes, appropriate agents are detectable labels that include radioisotopes, for whole body imaging, and radioisotopes, enzymes, fluorescent labels and other suitable antibody tags for sample testing. The detectable labels can be any of the various types used currently in the field of in vitro diagnostics, including particulate labels including metal sols such as colloidal gold, isotopes, chromophores including fluorescent markers, biotin, luminescent markers, phosphorescent markers and the like, as well as enzyme labels that convert a given substrate to a detectable marker, and polynucleotide tags that are revealed following amplification such as by polymerase chain reaction. A biotinylated antibody would then be detectable by avidin or streptavidin binding. Suitable enzyme labels include horseradish peroxidase, alkaline phosphatase and the like. For instance, the label can be the enzyme alkaline phosphatase, detected by measuring the presence or formation of chemiluminescence following conversion of 1,2 dioxetane substrates such as adamantyl methoxy phosphoryloxy phenyl dioxetane (AMPPD), disodium 3-(4-(methoxyspiro{ 1,2-dioxetane-3,2'-(5'-chloro)tricyclo{3.3.1.1 3,7}decan}-4-yl) phenyl phosphate (CSPD), as well as CDP and CDP-star^{®} or other luminescent substrates well-known to those in the art, for example the chelates of suitable lanthanides such as Terbium(III) and Europium(III). The detection means is determined by the chosen label. Appearance of the label or its reaction products can be achieved using the naked eye, in the case where the label is particulate and accumulates at appropriate levels, or using instruments such as a spectrophotometer, a luminometer, a fluorimeter, and the like, all in accordance with standard practice.

Numerous methods known to those skilled in the art are available for obtaining antibodies or antigen-binding fragments thereof. For example, antibodies can be produced using recombinant DNA methods (U.S. Patent 4,816,567). Monoclonal antibodies may also be produced by generation of hybridomas (see e.g., Kohler and Milstein (1975) Nature, 256: 495-499) in accordance with known methods. Hybridomas formed in this manner are then screened using standard methods, such as enzyme-linked immunosorbent assay (ELISA) and surface plasmon resonance (BIACORE^{™}) analysis, to identify one or more hybridomas that produce an antibody that specifically binds with a specified antigen. Any form of the specified antigen may be used as the immunogen, e.g., recombinant antigen, naturally occurring forms, any variants or fragments thereof, as well as antigenic peptide thereof.

One exemplary method of making antibodies includes screening protein expression libraries, e.g., phage or ribosome display libraries. Phage display is described, for example, in Ladner etal., U.S. Patent No. 5,223,409; Smith (1985) Science 228:1315-1317; Clackson et al. (1991) Nature, 352: 624-628; Marks et al. (1991) J. Mol. Biol., 222: 581-597WO 92/18619; WO 91/17271; WO 92/20791; WO 92/15679; WO 93/01288; WO 92/01047; WO 92/09690; and WO 90/02809.

In addition to the use of display libraries, the specified antigen can be used to immunize a non-human animal, e.g., a rodent, e.g., a mouse, hamster, or rat. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XENOMOUSE^{™}, Green et al. (1994) Nature Genetics 7:13-21, US 2003-0070185, WO 96/34096, and WO96/33735.

In another embodiment, a monoclonal antibody is obtained from the non-human animal, and then modified, e.g., humanized, deimmunized, chimeric, may be produced using recombinant DNA techniques known in the art. A variety of approaches for making chimeric antibodies have been described. See e.g., Morrison et al., Proc. Natl. Acad. Sci. U.S.A. 81:6851, 1985; Takeda et al., Nature 314:452, 1985, Cabilly et al., U.S. Patent No. 4,816,567; Boss et al., U.S. Patent No. 4,816,397; Tanaguchi et al., EP 171496; EP 173494, GB 2177096. Humanized antibodies may also be produced, for example, using transgenic mice that express human heavy and light chain genes, but are incapable of expressing the endogenous mouse immunoglobulin heavy and light chain genes. Winter describes an exemplary CDR-grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Those methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

In certain embodiments, a humanized antibody is optimized by the introduction of conservative substitutions, consensus sequence substitutions, germline substitutions and/or backmutations. Such altered immunoglobulin molecules can be made by any of several techniques known in the art, (e.g., Teng et al., Proc. Natl. Acad. Sci. U.S.A., 80: 7308-7312, 1983; Kozbor et al, Immunology Today, 4: 7279, 1983; Olsson et al., Meth. Enzymol., 92: 3-16, 1982), and may be made according to the teachings of WO 92/06193 or EP 239400).

An antibody or fragment thereof may also be modified by specific deletion of human T cell epitopes or "deimmunization" by the methods disclosed in WO 98/52976 and WO 00/34317. Briefly, the heavy and light chain variable domains of an antibody can be analysed for peptides that bind to MHC Class II; these peptides represent potential T-cell epitopes (as defined in WO 98/52976 and WO 00/34317). For detection of potential T-cell epitopes, a computer modelling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T-cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences, e.g., are disclosed in Tomlinson, et at. (1992) J. Mol. Biol. 227:776-798; Cook, G. P. et al. (1995) Immunol. Today Vol. 16 (5): 237-242; Chothia, et al. (1992) J. Mol. Biol. 227:799-817; and Tomlinson et al. (1995) EMBO J. 14:4628-4638. The V BASE directory provides a comprehensive directory of human immunoglobulin variable region sequences (compiled by Tomlinson, LA. et al. MRC Centre for Protein Engineering, Cambridge, UK). These sequences can be used as a source of human sequence, e.g., for framework regions and CDRs. Consensus human framework regions can also be used, e.g., as described in U.S. Patent No. 6,300,064.

Techniques for production of antibodies, including polyclonal, monoclonal, humanized, bispecific and heteroconjugate antibodies are known in the art, some of which are exemplified below.

### Heteroconjugate Antibodies

Heteroconjugate antibodies are also within the scope of the present invention.

Heteroconjugate antibodies are composed of two covalently joined (e.g., linked) antibodies. For example, one of the antibodies in the heteroconjugate can be coupled to avidin, the other to biotin. It is contemplated that the antibodies may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U. S. Patent No. 4,676,980. Heteroconjugate antibodies may be made using any convenient cross-linking methods. Suitable cross-linking agents are well known in the art, and are disclosed in US Patent No. 4,676, 980, along with a number of cross-linking techniques.

For additional antibody production techniques, see Antibodies: A Laboratory Manual, eds. Harlow et al., Cold Spring Harbor Laboratory, 1988. The present invention is not necessarily limited to any particular source, method of production, or other special characteristics of an antibody.

The antibody of the present invention is preferably an "isolated" antibody. "Isolated" when used to describe antibodies disclosed herein, means an antibody that has been identified, separated and/or recovered from a component of its production environment. Preferably, the isolated antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

The term "position" when used in accordance with the present invention means the position of an amino acid within an amino acid sequence depicted herein. The term "corresponding" as used herein also includes that a position is not only determined by the number of the preceding nucleotides/amino acids.

The position of a given amino acid in accordance with the present invention which may be substituted due to deletion or addition of amino acids elsewhere in a polypeptide.

Thus, under a "corresponding position" in accordance with the present invention it is to be understood that amino acids may differ in the indicated number but may still have similar neighbouring amino acids. Said amino acids which may be exchanged, deleted or added are also comprised by the term "corresponding position".

In order to determine whether an amino acid residue in a given amino acid sequence corresponds to a certain position in the amino acid sequence, the skilled person can use means and methods well-known in the art, e.g., alignments, either manually or by using computer programs such as BLAST2.0, which stands for Basic Local Alignment Search Tool or ClustalW or any other suitable program which is suitable to generate sequence alignments.

As used herein, the term "% identity" refers to the percentage of identical amino acid residues at the corresponding position within the sequence when comparing two amino acid sequences with an optimal sequence alignment as exemplified by the ClustalW or X techniques as available from www.clustal.org, or equivalent techniques. Accordingly, both sequences (reference sequence and sequence of interest) are aligned, identical amino acid residues between both sequences are identified and the total number of identical amino acids is divided by the total number of amino acids (amino acid length). The result of this division is a percent value, i.e. percent identity value/degree.

In another aspect, the present invention provides an antibody or antigen binding portion thereof of the present invention for use as diagnostic composition. Accordingly, the antibody or antigen binding portion thereof can be used in diagnostic assays for their antigen, e.g., detecting its expression in specific cells, tissues, or serum.

Various diagnostic assay techniques known in the art may be used, such as competitive binding assays, direct or indirect sandwich assays and immunoprecipitation assays conducted in either heterogeneous or homogeneous phases (Zola, Monoclonal Antibodies: A Manual of Techniques, CRC Press, Inc. (1987) pp. 147-158). The antibody or antigen binding portion thereof used in the diagnostic assays can be labelled with a detectable moiety. For example, antibody or antigen binding portion thereof may be modified with detectable markers, including ligand groups (e.g., biotin), fluorophores and chromophores, radioisotopes, electron-dense reagents, or enzymes. Enzymes are detected by their activity. For example, horseradish peroxidase is detected by its ability to convert tetramethylbenzidine (TMB) to a blue pigment, quantifiable with a spectrophotometer. Other suitable binding partners include biotin and avidin, IgG and protein A, and other receptor-ligand pairs known in the art.

The detectable moiety should be capable of producing, either directly or indirectly, a detectable signal. For example, the detectable moiety may be a radioisotope, such as ³H, ¹⁴C, ³²P ³⁵S or ¹²⁵I a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed.

The antibody or antigen binding portion thereof of the present invention when administered to a subject is preferably in the form of a composition. The composition is preferably suitable for pharmaceutical use and administration to subjects.

Accordingly, the antibody or antigen binding portion thereof of the present invention is envisaged for use in therapy. Accordingly, the present invention envisages a pharmaceutical composition (or medicament) comprising the antibody or antigen binding portion thereof described herein.

In yet another embodiment, the invention provides a method of treating a subject comprising administering a therapeutically effective amount of the antibody or antigen binding portion thereof of the present invention, wherein the subject has cancer.

As used herein, "cancer" refers a broad group of diseases characterized by the uncontrolled growth of abnormal cells in the body. Unregulated cell division may result in the formation of malignant tumours or cells that invade neighbouring tissues and may metastasize to distant parts of the body through the lymphatic system or bloodstream.

Cancers whose growth may be inhibited using the antibodies of the invention include cancers typically responsive to immunotherapy. Non-limiting examples of cancers for treatment include squamous cell carcinoma, small-cell lung cancer, non- small cell lung cancer, squamous non-small cell lung cancer (NSCLC), non NSCLC, glioma, gastrointestinal cancer, renal cancer (e.g. clear cell carcinoma), ovarian cancer, liver cancer, colorectal cancer, endometrial cancer, kidney cancer (e.g., renal cell carcinoma (RCC)), prostate cancer (e.g. hormone refractory prostate adenocarcinoma), thyroid cancer, neuroblastoma, pancreatic cancer, glioblastoma (glioblastoma multiforme), cervical cancer, stomach cancer, bladder cancer, hepatoma, breast cancer, colon carcinoma, and head and neck cancer (or carcinoma), gastric cancer, germ cell tumour, pediatric sarcoma, sinonasal natural killer, melanoma (e.g., metastatic malignant melanoma, such as cutaneous or intraocular malignant melanoma), bone cancer, skin cancer, uterine cancer, cancer of the anal region, testicular cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the parathyroid gland, cancer of the adrenal gland, sarcoma of soft tissue, cancer of the urethra, cancer of the penis, solid tumours of childhood, cancer of the ureter, carcinoma of the renal pelvis, neoplasm of the central nervous system (CNS), primary CNS lymphoma, tumour angiogenesis, spinal axis tumour, brain stem glioma, pituitary adenoma, Kaposi's sarcoma, epidermoid cancer, squamous cell cancer, T-cell lymphoma, environmentally-induced cancers including those induced by asbestos, virus-related cancers (e.g., human papilloma virus (HPV)- related tumour), and hematologic malignancies derived from either of the two major blood cell lineages, i.e., the myeloid cell line (which produces granulocytes, erythrocytes, thrombocytes, macrophages and mast cells) or lymphoid cell line (which produces B, T, NK and plasma cells), such as all types of luekemias, lymphomas, and myelomas, e.g., acute, chronic, lymphocytic and/or myelogenous leukemias, such as acute leukemia (ALL), acute myelogenous leukemia (AML), chronic lymphocytic leukemia (CLL), and chronic myelogenous leukemia (CML), undifferentiated AML (MO), myeloblastic leukemia (MI), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphomas, such as Hodgkin' s lymphoma (HL), non-Hodgkin' s lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic (e.g., Ki 1+) large-cell lymphoma, adult T-cell lymphoma/leukemia, mantle cell lymphoma, angio immunoblastic T-cell lymphoma, angiocentric lymphoma, intestinal T-cell lymphoma, primary mediastinal B-cell lymphoma, precursor T-lymphoblastic lymphoma, T-lymphoblastic; and lymphoma/leukemia (T-Lbly/T-ALL), peripheral T- cell lymphoma, lymphoblastic lymphoma, post-transplantation, lymphoproliferative disorder, true histiocytic lymphoma, primary central nervous system lymphoma, primary effusion lymphoma, lymphoblastic lymphoma (LBL), hematopoietic tumours of lymphoid lineage, acute lymphoblastic leukemia, diffuse large B-cell lymphoma, Burkitt's lymphoma, follicular lymphoma, diffuse histiocytic lymphoma (DHL), immunoblastic large cell lymphoma, precursor B-lymphoblastic lymphoma, cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, nonsecretory myeloma, smoldering myeloma (also called indolent myeloma), solitary, plasmocytoma, and multiple myelomas, chronic lymphocytic leukemia (CLL), hairy cell lymphoma; hematopoietic tumours of myeloid lineage, tumours of mesenchymal origin, including fibrosarcoma and rhabdomyoscarcoma; seminoma, teratocarcinoma, tumours of the central and peripheral nervous, including astrocytoma, schwannomas; tumours of mesenchymal origin, including fibrosarcoma, rhabdomyoscaroma, and osteosarcoma; and other tumours, including melanoma, xeroderma pigmentosum, keratoacanthoma, seminoma, thyroid follicular cancer and teratocarcinoma, hematopoietic tumours of lymphoid lineage, for example T-cell and B-cell tumours, including but not limited to T-cell disorders such as T-prolymphocytic leukemia (T-PLL), including of the small cell and cerebriform cell type; large granular lymphocyte leukemia (LGL) preferably of the T-cell type; aid T-NHL hepatosplenic lymphoma; peripheral/post-thymic T cell lymphoma (pleomorphic and immunoblastic subtypes); angiocentric (nasal) T-cell lymphoma; cancer of the head or neck, renal cancer, rectal cancer, cancer of the thyroid gland; acute myeloid lymphoma, as well as any combinations of said cancers. The methods described herein may also be used for treatment of metastatic cancers, refractory cancers (e.g., cancers refractory to previous immunotherapy, e.g., with a blocking CTLA-4 or PD-1 or PD-L1 antibody), and recurrent cancers.

Preferred cancers of the present invention are also shown in Table 1 below.

In preferred embodiments a cancer is selected from a group consisting of: leukemia, lymphoma, myeloma, breast cancer, colorectal cancer, glioblastoma, ovarian cancer, hematological cancer, epithelial cancer, pancreatic cancer, bladder cancer, uterine/cervical cancer, ovarian cancer, prostate cancer, testicular cancer, esophageal cancer, gastrointestinal cancer, colon cancer, kidney cancer, head and neck cancer, lung cancer, stomach cancer, germ cell cancer, bone cancer, liver cancer, thyroid cancer, skin cancer, neoplasm of the central nervous system, sarcoma, and virus-related cancer.

As used herein, an "autoimmune disease" refers a broad group of diseases characterized by disease associated with the production of antibodies directed against one's own tissues. Non-limiting examples of an autoimmune disease include, but are not limited to, Hashimoto's disease, primary biliary cirrhosis, systemic lupus erythematosus, rheumatic fever, rheumatoid arthritis, autoimmune hemolytic anemia, idiopathic thrombocytopenic purpura, and postviral encephalomyelitis, Addison's disease, autoimmune enteropathy, primary biliary cirrhosis, Goodpasture's syndrome, Hashimoto's thyroiditis, myasthenia gravis, myxoedema, pemphigoid, rheumatoid arthritis, Sjogren's syndrome, symphathetic ophthalmitis, both forms of lupus erythematosus, thyrotoxicosis, ulcerative colitis, multiple sclerosis, celiac disease, diabetes mellitus type 1, Graves' disease, inflammatory bowel disease and psoriasis.

As used herein, an "inflammatory disease" refers a broad group of diseases characterized by impairment and/or abnormal functioning of inflammatory mechanisms of the body. Non-limiting examples of an inflammatory disease include, but are not limited to, necrotizing enterocolitis, gastroenteritis, pelvic inflammatory disease (PID), empyema, pleurisy, pyelitis, pharyngitis, angina, arthritis, acne, urinary tract infections, Acne vulgaris, Asthma, Celiac disease, Chronic prostatitis, Colitis, Diverticulitis, Glomerulonephritis, Hidradenitis suppurativa, Hypersensitivities, Inflammatory bowel diseases, Interstitial cystitis, Mast Cell Activation Syndrome, Mastocytosis, Otitis, Pelvic inflammatory disease, Reperfusion injury, Rheumatic fever, Rheumatoid arthritis, Rhinitis, Sarcoidosis, Transplant rejection, Vasculitis.

The term "subject" is intended to include living organisms. Examples of subjects include mammals, e.g., humans, dogs, cows, horses, pigs, sheep, goats, cats, mice, rabbits, rats, and transgenic non-human animals. In preferred embodiments of the invention, the subject is a human.

As used herein, the terms "disorder" and "disease" are used interchangeably to refer to a condition in a subject. In particular, the term "cancer" is used interchangeably with the term "tumour".

Moreover, antibodies of the invention can be used for diagnostic purposes to detect, diagnose, or monitor diseases, or disorders, in particular cancer and cancer-related diseases. Antibodies or fragments or derivatives thereof according to the invention can be used to assay glycoprotein levels in a biological sample using classical immunohistological methods as described herein or as known to those of skill in the art (e.g., see Jalkanen et al., 1985, J. Cell. Biol. 101: 976-985; Jalkanen et al., 1987, J. Cell. Biol. 105: 3087-3096). Other antibody-based methods useful for detecting protein gene expression include immunoassays, such as the enzyme linked immunosorbent assay (ELISA) and the radioimmunoassay (RIA).

Therefore, the present invention further relates to a diagnostic composition comprising an antibody of the invention.

As used herein, the term "diagnostic" refers to any use of the inventive antibody for diagnosing the presence of a target polypeptide or glycoprotein in a cancer or related disease.

In a further embodiment of the invention, there are provided articles of manufacture and kits containing antibody or antigen binding portion thereof which can be used, for instance, for the therapeutic or non-therapeutic applications described above. The article of manufacture comprises a container with a label. Suitable containers include, for example, bottles, vials, and test tubes. The containers may be formed from a variety of materials such as glass or plastic. The container holds a composition which includes an active agent that is effective for therapeutic or non-therapeutic applications, such as described above. The active agent in the composition is the antibody or antigen binding portion thereof. The label on the container indicates that the composition is used for a specific therapy or non-therapeutic application and may also indicate directions for either in vivo or in vitro use, such as those described above.

The kit of the invention will typically comprise the container described above and one or more other containers comprising materials desirable from a commercial and user standpoint, including buffers, diluents, filters, needles, syringes, and package inserts with instructions for use.

As used herein, the term "immobilized" refers to an antibody or the antigen binding portion thereof or lectin that has been bound, usually covalently, to an insoluble organic or inorganic matrix (e.g., magnetic carrier). Yet, it is preferred that an antibody when applied in a method, use or kit of the present invention is not immobilized, preferably not immobilized on a solid surface.

As used herein, the term "magnetic carrier" refers to particles or beads comprising magnetic material or substance (e.g., iron or ferritin). Preferably, the magnetic carrier is a magnetic particle or magnetic bead (e.g., a ferritin conjugate). However, for avoidance of doubt, the magnetic carrier when referred herein is not a solid surface, such as a plate, e.g. a ELISA plate, as used herein.

As used herein, the term "bead" refers to a small spherical object, e.g., made of glass, plastic, metal, agarose, latex, metallic nano- or microparticle, metal oxide nano- or microparticle or magnetic material.

As used herein, the term "microperoxidase" or "MP" refers to a heme containing peptide portion of cytochrome c (e.g., shown as SEQ ID NO: 10, cytochrome c derived from *Equus caballus,* NCBI Reference Sequence: NP_001157486.1) that retains peroxidase activity (e.g., EC 1.11.1.7 enzymatic activity, e.g., microperoxidase-11). Preferably, the heme containing peptide portion of cytochrome c is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 7 (MP-11 peptide), SEQ ID NO: 8 (MP-9 peptide) and SEQ ID NO: 9 (MP-8 peptide), preferably said microperoxidase (MP) peptide is selected from the group consisting of: SEQ ID NO: 7 (MP-11 peptide), SEQ ID NO: 8 (MP-9 peptide) and SEQ ID NO: 9 (MP-8 peptide).

As used herein, some lectins can cause cells to agglutinate. Lectins can be obtained from seeds of leguminous plants, but also from other plant and animal sources. Lectins can contain binding sites for specific mono- and oligosaccharides (e.g., glycans of glycoproteins). They can agglutinate cells by binding to specific sugar residues in membrane glycoproteins. Preferably, lectins of the present invention are selected from the group consisting of: *Maackia amurensis* lectin II (MAA II); Concanavalin A (Con A); *Aleuria aurantia* lectin (AAL); *Sambucus nigra* (SNA-I) lectin; *Wisteria floribunda* lectin (WFL) as defined herein.

Further preferred lectins of the present invention are shown in Table 1 below.

Particularly preferred lectins of the present invention are lectins with the following UniProtKB Accession Numbers: P0DKL3, P02866, P18891, O04366, A0A218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, O24313, Q9SM56, P06750, B9SPG3, Q9BZZ2, P20916, Q9NYZ4, Q96RL6, P05046, P93535, P02876, P10968, P10969, P22972 or P56625 as well as corresponding mature forms thereof.

Exemplary lectins of the present invention further include:

*Maackia amurensis* lectin II (MAA II) is the hemagglutinin isolectin from *Maackia* seeds. Sialic acid-binding lectin recognizing oligosaccharides containing terminal sialic acid linked via α2-3 bond to penultimate galactose residues. Binds the trisaccharide sequence Neu5Acα2-3-Gal-β-1-4-GlcNAc. Preferably, MAA II has a SEQ ID NO: 52 (or its mature form).

Concanavalin A (Con A) a D-mannose specific lectin originally extracted from the jack-bean, *Canavalia ensiformis.* Preferably, Con A has a SEQ ID NO: 53 or SEQ ID NO: 54 (Con A, mature form).

*Aleuria aurantia* lectin (AAL) is a fucose-specific lectin extracted from *Aleuria aurantia* (Orange peel mushroom). Preferably, AAL has a SEQ ID NO: 55 (or its mature form). The isolation of AAL is, for example, described in (Debray et al., Kochibe et al.).

*Sambucus nigra* (SNA-I) lectin is a Neu5Acα2-6)Gal/GalNAc specific agglutinin extracted from *Sambucus nigra* (European elder). Preferably, SNA-I has a SEQ ID NO: 56 (or its mature form).

*Wisteria floribunda* lectin (WFL) is an agglutinin extracted from *Wisteria floribunda* (Japanese wisteria). Preferably, WFL has a SEQ ID NO: 57 (or its mature form).

Other preferred lectins of the present invention are also disclosed herein in sections *"Other lectins (e.g., post-translationally processed and mature forms thereof) within the meaning of the present invention"* and in *"Sequence listing".*

Furthermore, suitable lectins within the meaning of the present invention explicitly include post-translationally processed- and mature forms of the lectins as disclosed herein.

The problem to be solved by the present invention can inter alia be seen in one or more of the following: i) Identifying novel means (e.g., biomarkers and methods) for improved cancer diagnostics (e.g. prostate cancer diagnostics etc. Table 1); ii) improving sensitivity of cancer detection (e.g. prostate cancer detection), e.g., allowing for a reduced amount of sample needed (e.g., 0.04 ml or less) for accurate diagnostics of cancer (e.g. prostate cancer diagnostics); iii) shortening the analysis time of cancer diagnostics (e.g. prostate cancer diagnostics), e.g., by means of using magnetic particles for PSA enrichment and/or signal generation; iv) increasing versatility of cancer diagnostics (e.g. prostate cancer diagnostics etc. Table 1), e.g., by means of identifying novel means (e.g., biomarkers and methods) for glycoprofiling of any protein (e.g., cancer biomarker); v) reducing number of false positives (e.g. false positives arising from elevated glycoprotein/s levels in benign conditions) in glycoprotein-based (e.g., PSA-based) diagnostics of cancer (e.g. prostate cancer); vi) discriminating between significant and insignificant tumours, e.g., by means of glycoprotein-based (e.g., PSA-based) diagnostics of cancer (e.g. prostate cancer); vii) discriminating between slow growing (e.g., clinically harmless) and fast growing (e.g., clinically relevant) tumours, e.g., by means of glycoprotein-based (e.g., PSA-based) diagnostics of cancer (e.g. prostate cancer); viii) reducing the risk of side effects and/or unnecessary treatment of cancer therapy (e.g., prostate cancer therapy, e.g., side effects such as urinary incontinence, sexual dysfunction and bowel problems, etc.) in patients with harmless PCa); ix) reducing the cost of cancer diagnostics (e.g., prostate cancer diagnostics, e.g., glycoprotein-based (e.g., PSA-based) prostate cancer diagnostics); x) increasing the convenience of cancer diagnostics (e.g., prostate cancer diagnostics, e.g., glycoprotein-based (e.g., PSA-based) prostate cancer diagnostics) for subjects in need thereof, e.g., by means of reducing the number of cumbersome examinations (e.g., imaging, prostate biopsies, etc.) necessary; xi) detecting curable cancers (e.g., prostate cancer) with high and improved specificity (e.g., avoiding unnecessary follow-up examination procedures); xii) identifying organ confined and/or potentially curable cancers (e.g., prostate cancer), e.g., by means of glycoprotein-based (e.g., PSA-based) diagnostics of cancer. The problem is solved according to the claims of the present invention. The magnetic carriers, anti-glycoprotein antibodies, the antigen binding portions thereof, one or more lectins, compositions, kits and methods and uses based thereon are applicable to any glycoprotein, e.g., to any cancer biomarkers with aberrant glycosylation.

Preferred cancers, cancer biomarkers with aberrant glycosylation, lectins, antibodies and corresponding glycan modifications within the meaning of the present invention are also shown in **Table 1** below. Lectin abbreviations used in Table 1: AAA - Anguilla anguilla agglutinin (UniProtKB Accession Number: Q7SIC1), AAL - *Aleuria aurantia* lectin, ABA - *Agaricus bisporus* agglutinin, ACA - *Amaranthus caudatus* agglutinin, AHA - *Arachis hypogaea* agglutinin = peanut agglutinin (PNA), AIA - *Artocarpus integrifolia* agglutinin = Jacalin, AlloA - *Allomyrina dichotoma* agglutinin, AOL - *Aspergillus oryzae* lectin, BanLec - *Musa paradisiaca* lectin, BS-I - *Bandeiraea simplicifolia* lectin = *Griffonia (Bandeiraea) simplicifolia* lectin I, Con A - Concanavalin A, DBA - *Dolichos biflorus* agglutinin, DSA - *Datura stramonium* agglutinin (Jacalin), ECL - *Erythrina cristagalli* lectin, GNA - *Galanthus nivalis* agglutinin, GSA I (GSL I) - *Griffonia (Bandeiraea) simplicifolia* lectin I, GSL II - *Griffonia (Bandeiraea) simplicifolia* lectin II, HHL - *Hippeastrum* hybrid (*Amaryllis*) lectin, HPA - *Helix pomatia* agglutinin, LBA - *Phaseolus lunatus* (lima bean, LBA), LEL - *Lycopersicon esculentum* (tomato) lectin, LCA - *Lens culinaris* agglutinin, LTA - *Lotus tetragonolobus* lectin, MAA I - *Maackia amurensis* agglutinin I, MAA II - *Maackia amurensis* agglutinin II, MGBL 1 - macrophage galactose binding lectin 1, MGBL 2 (macrophage galactose binding lectin 2, NPA - *Narcissus pseudonarcissus* (Daffodil) lectin, PHA E - *Phaseolus vulgaris* agglutinin E, PHA L - *Phaseolus vulgaris* agglutinin L, PhoSL - *Pholiota squarrosa* lectin, PNA - Peanut agglutinin, PSL - *Pisum sativum* lectin, PTA I - *Psophocarpus tetragonolobus* lectin I, PTA II - *Psophocarpus tetragonolobus* II, PWM - *Phytolacca americana,* RCA I - *Ricinus communis* agglutinin I, RCA II - *Ricinus communis* agglutinin II, SBA - Soybean agglutinin (*Glycine max* agglutinin), SCA - *Sambucus canadensis* agglutinin = *Sambucus nigra* agglutinin (SNA), SJA - *Sophora japonica* agglutinin II, SNA - *Sambucus nigra* agglutinin, SSA - *Sambucus sieboldiana* agglutinin, SSL - *Salvia sclarea* lectin, STL - *Solanum tuberosum* lectin, TJA-I - *Trichosanthes japonica* agglutinin I, TJA-II - *Trichosanthes japonica* agglutinin (Yamashita et al.), TVA - *Triticum vulgaris* agglutinin = WGA - wheat germ agglutinin, UEA - *Ulex europaeus* agglutinin, VVA - *Vicia villosa* lectin, WFA - *Wisteria floribunda* lectin, WGA - wheat germ agglutinin = TVA - *Triticum vulgaris* agglutinin. The symbol "↑", an *upward pointing arrow* means increase in concentration of a corresponding glycan/s or a complex/s (e.g., dimer, trimer etc). The symbol "↓", a *downward pointing arrow* means increase in concentration of a corresponding glycan/s or a complex/s (e.g., dimer, trimer etc).

**Table 1: Cancers, corresponding cancer biomarkers with aberrant glycosylation, lectins and antibodies.**

| **Cancer** | **Biomarker** | **Glycan modification** | **Lectins/antibodies applied** | **Refs.** | **(Other) applicable lectins/Abs** |
|---|---|---|---|---|---|
| ***Prostate*** | Prostate specific antigen (PSA) | ↑ α2-3Neu5Ac | MAA | [1-5] | anti-α2-3-linked sialic acid antibody (i.e. i.e. HYB4), Siglec 1, Siglec 4 or Siglec 8 |
| | fPSA | ↑ α2-3Neu5Ac | SNA* (determination of non-eluted PSA from SNA affinity column) | [6, 7] | anti-α2,3-linked sialic acid antibody (i.e. HYB4i.e. |
| | | | | | i.e. HYB4), MAA, Siglec 1, Siglec 4 or Siglec 8 |
| | fPSA | ↑ α2-3 Neu5Ac | anti-α2-3-linked sialic acid antibody (i.e. HYB4) | [8] | MAA, Siglec 1, Siglec 4 or Siglec 8 |
| | PSA [1], tPSA/fPSA [9] | ↓ bi-antennary glycans | Con A | [1, 9] | |
| | PSA [1], tPSA/fPSA [9] | ↓ high mannose glycans | Con A | [1, 9] | GNA, NPA |
| | PSA | ↓ α2-6Neu5Ac | SNA | [1] | TJA-I, SCA |
| | PSA | ↓ α2-6Neu5Ac | TJA-I | [2] | SNA, SCA |
| | PSA | ↑ tri-, tetra-antennary glycans | DSA (Jacalin) | [2] | PHA-L, PHA-E |
| | PSA | ↑ α1-2fucose, GalNAc | TJA-II | [2] | AAL, UEA-I, LCA, PSL, AAA, LTA, HPA, LBA, WFA, VVA |
| | PSA [2], fPSA/tPSA [10] | ↑ α1-2fucose | UEA-I | [2] [10] | TJA II, AAL, LCA, PSL, AAA, LTA |
| | PSA [2], tPSA [11, 12] | ↑ LacdiNAc, GalNAc | WFA | [2, 11, 12] | DBA, SBA, HPA, LBA, VVA |
| | tPSA | ↑ α1-3/6fucose | AAL | [13] | TJA II, UEA-I, LCA, PSL, AAA, LTA, AOL, PhoSL |
| | PSA in urine | ↓ α1-3/6 fucose | AAL | [14] | TJA II, UEA-I, LCA, PSL, AAA, LTA. AOL, PhoSL |
| | PSA in urine | ↓ core fucose (α1-6fucose) | PhoSL | [14] | AOL |
| | fPSA | ↓ core fucose (α1-6fucose) | PhoSL | [6] | AOL |
| | Tissue inhibitor of metallopeptidase 1 | ↑ α1-3/6fucose | AAL | [13] | AOL, PhoSL, TJA |
| | (TIMP1) | | | | II, UEA-I, LCA, PSL, AAA, LTA |
| | β-haptoglobin | ↑ core fucose (α1-6fucose) | No lectin used, but MS | [15] | PhoSL, AOL |
| | β-haptoglobin | ↑ core/antennary fucose | AAL | [16, 17] | AOL, PhoSL, TJA II, UEA-I, LCA, PSL, AAA, LTA |
| | β-haptoglobin | ↑ α2-6Neu5Ac | SNA | [16, 17] | TJA-I, SCA |
| | β-haptoglobin | ↑ tri-,tetra-antennary glycans | PHA-L | [16, 17] | PHA-E, DSA (Jacalin) |
| | β-haptoglobin | ↑ sialyl Lewis^{a} glycan | Antibody against sialyl Lewis^{a} glycan | [16] | SNA, TJA-I, MAA, anti-α2-3-linked sialic acid I antibody (i.e. HYB4i.e. i.e. HYB4), Siglec 1, Siglec 4 or Siglec 8 |
| | β-haptoglobin | ↑ sialyl Lewis^{x} glycan | Antibody against sialyl Lewis^{x} glycan | [17] | SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ antennary fucose | No lectin used, but MS | [18] | TJA II, AAL, UEA-I, LCA, PSL, AAA, LTA |
| | β-haptoglobin | ↑ tri-,tetra-antennary glycans | No lectin used, but MS | [18] | PHA-L, PHA-E, DSA |
| | β-haptoglobin | ↑ sialyl Lewis^{a} and sialyl Lewis^{x} glycans | No lectin used, but MS | [18] | Antibodies against sialyl Lewis^{a} and Lewis^{x} glycans, SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ antennary fucose | AAL | [19] | TJA II, UEA-I, LCA, PSL, AAA, LTA |
| | | | | | |
| **Ovarian** | α₁-acid glycoprotein | ↑ tri-,tetra-antennary glycans | Capillary electrophoresis (CE) | [20] | PHA-L, PHA-E, DSA |
| | α₁-acid glycoprotein | ↑ core fucose | CE | [20] | PhoSL, AOL |
| | α₁-acid glycoprotein | ↑ α2-6Neu5Ac | 2D PAGE and LC | [21] | TJA-I, SNA |
| | α₁-acid glycoprotein | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), Siglec 1, Siglec 4 or Siglec 8 |
| | α₁-acid glycoprotein | ↓ α2-3Neu5Ac | 2D PAGE and LC | [21] | MAA, anti-α2-3-linked sialic acid antibody (i.e. HYB4i.e. i.e. HYB4), Siglec 1, Siglec 4 or Siglec 8 |
| | C1 esterase inhibitor | ↑ Le^{x} | CE | [20] | Antibody against Le^{x}, LTA |
| | C1 esterase inhibitor | ↑ tri-antennary glycans | CE | [20] | DBA, PHA-E, PHA-L |
| | 2-HS glycoprotein | ↑ tri-,tetra-antennary glycans | CE | [20] | DBA, PHA-E, PHA-L |
| | β-haptoglobin | ↑ tri-,tetra-antennary glycans | CE | [20] | DBA, PHA-E, PHA-L |
| | β-haptoglobin | ↑ Le^{x} | CE | [20] | Antibody against Le^{x}, LTA |
| | β-haptoglobin | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), Siglec 1, Siglec 4 or Siglec 8 |
| | β-haptoglobin | ↑ α2-6Neu5Ac | 2D PAGE and LC | [21] | SNA, TJA-I |
| | β-haptoglobin | ↓ α2-3Neu5Ac | 2D PAGE and LC | [21] | MAA, anti-α2-3-linked sialic acid antibody (i.e. HYB4i.e. i.e. HYB4), , Siglec 1, Siglec 4 or Siglec 8 |
| | β-haptoglobin | ↑ tri-, tetra-antennary glycans | LTA affinity separation AND PAGE | [22] | DBA, PHA-E, PHA-L |
| | β-haptoglobin | ↑ α2-3Neu5Ac | LTA affinity separation AND PAGE | [22] | MAA, anti-α2-3-linked sialic acid antibody (i.e. HYB4i.e. i.e. HYB4), , Siglec 1, Siglec 4 or Siglec 8 |
| | β-haptoglobin | ↓ α2-6Neu5Ac | LTA affinity separation AND PAGE | [22] | SNA, TJA-I |
| | β-haptoglobin | ↑ antennary fucose | LTA affinity separation AND PAGE | [22] | TJA II, AAL, UEA-I, LCA, PSL, AAA, AAL |
| | β-haptoglobin | ↓ bi-antennary glycans | Con A | [22] | NPA, GNA |
| | β-haptoglobin | ↑ α2-3Neu5Ac | MAA | [22] | anti-α2-3-linked sialic acid antibody (i.e. HYB4i.e. i.e. HYB4), , Siglec 1, Siglec 4 or Siglec 8 |
| | α-1-antitrypsin | ↑ tetra-antennary glycans | CE | [20] | DBA, PHA-E, PHA-L |
| | α-1-antitrypsin | ↑ Le^{x} | CE | [20] | Antibody against Le^{x}, LTA |
| | α-1-antitrypsin | ↓ tri-, tetra-antennary glycans | LTA affinity separation AND PAGE | [22] | DBA, PHA-E, PHA-L |
| | α-1-antitrypsin | ↓ α2-3Neu5Ac | LTA affinity separation AND PAGE | [22] | MAA, anti-α2-3-linked sialic acid antibody (i.e. HYB4i.e. i.e. HYB4), , Siglec 1, Siglec 4 or Siglec 8 |
| | α-1-antitrypsin | ↑ α2-6Neu5Ac | LTA affinity separation AND PAGE | [22] | SNA, TJA-I |
| | α-1-antitrypsin | ↑ core fucose | LTA affinity separation AND PAGE | [22] | AOL, PhoSL |
| | α-1-antitrypsin | ↑ bi-antennary glycans | Con A | [22] | NPA, GNA |
| | α-1-antitrypsin | ↑ α2-6Neu5Ac | SNA | [22] | TJA-I, SCA |
| | α-1-antitrypsin | ↓ α2-3Neu5Ac | MAA | [22] | anti-α2-3-linked sialic acid antibody (i.e. HYB4i.e. i.e. HYB4), , Siglec 1, Siglec 4 or Siglec 8 |
| | α-1-antichymotrypsin | ↑ tetra-antennary glycans | CE | [20] | DBA, PHA-E, PHA-L |
| | α-1-antichymotrypsin | ↑ Le^{x} | CE | [20] | Antibody against Le^{x}, LTA |
| | α-1-antichymotrypsin | ↑ sialyl Le^{x} | 2D PAGE and LC | [21] | Antibody against sLe^{x}, SNA, TJA-I, MAA, anti-α2-3-linked sialic acid antibody (i.e. HYB4i.e. i.e. HYB4), , Siglec 1, Siglec 4 or Siglec 8 |
| | α-1-antichymotrypsin | ↑ α2-6Neu5Ac | 2D PAGE and LC | [21] | SNA, TJA-I |
| | α-1-antichymotrypsin | ↓ α2-3Neu5Ac | 2D PAGE and LC | [21] | MAA, anti-α2-3-linked sialic acid antibody (i.e. HYB4i.e. i.e. HYB4), , Siglec 1, Siglec 4 or Siglec 8 |
| | transferrin | ↓ tri-antennary glycans | CE | [20] | DBA, PHA-E, PHA-L |
| | hemopexin | ↑ Le^{x} | CE | [20] | Antibody against Le^{x}, LTA |
| | IgG | ↓ galactose | 2D PAGE and LC | [21] | RCA, RCA120, ABA, Jacalin (DSA), AlloA, ECL, PNA |
| | IgG | ↓ sialic acid | 2D PAGE and LC | [21] | SNA, TJA-I, MAA, anti-α2-3-linked sialic acid antibody (i.e. HYB4i.e. i.e. HYB4), , Siglec 1, Siglec 4 or Siglec 8 |
| | CA125 (MUC16) | ↑ sialyl Tn antigen | VVA lectin after sialidase detection by | [23] | SNA, TJA-I, MAA, anti-α2-3-linked sialic acid antibody (i.e. HYB4i.e. i.e. HYB4), , Siglec 1, Siglec 4 or Siglec 8 |
| | CA125 (MUC16) | ↑ sialyl T antigen | anticarbohydrate IgM antibodies 3C9 after sialidase detection | [23] | SNA, TJA-I, MAA, anti-α2-3-linked sialic acid antibody (i.e. HYB4i.e. i.e. HYB4), , Siglec 1, Siglec 4 or Siglec 8 |
| | CA15-3 (MUC1) | ↑ sialyl Tn antigen | VVA lectin after sialidase detection | [23] | SNA, TJA-I, MAA, anti-α2-3-linked sialic acid antibody (i.e. HYB4), Siglec 1, Siglec 4 or Siglec 8 |
| | CA15-3 (MUC1) | ↑ core fucose | PAGE/LC | [24] | PhoSL, AOL |
| | CA15-3 (MUC1) | ↑ bi-antennary glycans | PAGE/LC | [24] | Con A |
| | CA15-3 (MUC1) | ↓ tri-, tetra-antennary glycans | PAGE/LC | [24] | PHA-E, PHA-L, DBA |
| | CA15-3 (MUC1) | ↑ antennary fucose | PAGE/LC | [24] | AAL, TJA II, UEA-I, LCA, PSL, AAA, LTA |
| | human epididymis protein 4 (HE4) | ↑ Le^{y} antigen | Antibody against Lewis^{y} glycan | [25] | UEA-I |
| | Clusterin | ↑ α2-6Neu5Ac | SNA | [26] | TJA-I, SCA |
| | leucine-rich α-2-glycoprotein | ↑ α2-6Neu5Ac | SNA | [26] | TJA-I, SCA |
| | | | | | |
| **Breast** | CA15-3 (MUC1) | ↑ sulfated core1 glycan | Galectin 4 | [27] | SBA, ABA, VVA, Jacalin (DSA), BPL, PNA, GSL1, SJA |
| | CA15-3 (MUC1) | ↑ Tn, sialyl Tn antigens | | [28] | SBA, DBA, VVA, SNA, SNA, TJA-I, MAA, anti-α2-3-linked sialic acid antibody (i.e. HYB4) |
| | CA15-3 (MUC1) | change sialyl T, Tn antigens | LC | [29] | SNA, TJA-I, MAA, anti-α2-3-linked sialic acid antibody (i.e. HYB4), Siglec 1, Siglec 4 or Siglec 8; SBA, ABA, VVA, BPL, Jacalin, PNA |
| | CA15-3 (MUC1) | change α2-8Neu5Ac | LC | [29] | antibody against poly(sialic acid), Siglec 7 or Siglec 11 |
| | CA15-3 (MUC1) | change in sialylation | LC | [29] | SNA, TJA-I, MAA, anti-α2-3-linked sialic acid antibody (i.e. HYB4), Siglec 1, Siglec 4 or Siglec 8 |
| | CA15-3 (MUC1) | change in core 2 glycan | LC | [29] | RCA, RCA120, ABA, Jacalin (DSA), PNA, WGA |
| | CA15-3 | change in sialylation | MAA | [30] | SNA, TJA-I, MAA, anti-α2-3-linked sialic acid antibody (i.e. HYB4), Siglec 1, Siglec 4 or Siglec 8 |
| | CA15-3 (MUC1) | change in sialylation | MAA, SNA, TVA=WGA | [30] | SNA, TJA-I, MAA, anti-α2-3-linked sialic acid antibody (i.e. HYB4), Siglec 1, Siglec 4 or Siglec 8 |
| | CA27.29 | change in sialylation | MAA | [30] | SNA, TJA-I, MAA, anti-α2-3-linked sialic acid antibody (i.e. HYB4), , Siglec 1, Siglec 4 or Siglec 8 |
| | HER2 | change in antennary fucose | UEA | [30] | TJA II, AAL, LCA, PSL, AAA, LTA |
| | HER2 | change in sialylation | MAA, SNA, TVA=WGA | [30] | SNA, TJA-I, MAA, anti-α2-3-linked sialic acid antibody (i.e. HYB4), , Siglec 1, Siglec 4 or Siglec 8 |
| | CEA | change in tri-, tetra-antennary glycans | | [31] | PHA-E, PHA-L, DBA |
| | | | | | |
| **Colorectal** | β-haptoglobin | ↑ antennary fucose | AAL | [32] | TJA II, UEA-I, LCA, PSL, AAA, LTA |
| | β-haptoglobin | ↑ antennary fucose | AAL | [33] | TJA II, UEA-I, LCA, PSL, AAA, LTA |
| | β-haptoglobin | ↑ bi-antennary glycans | PHA-E | [32] | Con A, PHA-L, DBA |
| | β-haptoglobin | ↑ antennary/core fucose | AAL, AOL, LTA | [34] | TJA II, UEA-I, LCA, PSL, AAA, PhoSL |
| | β-haptoglobin | ↑ dimer: Le^{a} on Le^{a} | mouse monoclonal antibody NCC-ST-421, | [35] | |
| | β-haptoglobin | ↑ Galβ1-4GIcNAc | Galectin 3 | [36] | ECA, AlloA |
| | Carcinoembryonic antigen (CEA) | ↑ Le^{x} | LTA, Antibody against sialyl Lewis^{x} glycan | [37] | |
| | CEA | ↑ Le^{y} | UEA-I, Antibody against sialyl Lewis^{x} glycan | [37] | |
| | CEA | ↑ α2-3Neu5Ac | MAA | [37] | anti-α2-3-linked sialic acid antibody (i.e. HYB4), Siglec 1, Siglec 4 or Siglec 8 |
| | CEA | ↑ *α*-D-Man | NPA | [37] | Con A, GNA |
| | CEA | ↑ tri-, tetra-antennary glycans | PHA-L | [37] | PHA-E, DBA |
| | CEA | ↑ mannose, fucose | DC-SIGN | [37] | NPA, Con A, GNA, AAL, TJA II, UEA-I, LCA, PSL, AAA, LTA, AOL, PhoSL |
| | CEA | ↓ terminal GalNAc | MGBL | [37] | DBA, SBA, VVA, HPA, WFA |
| | CEA | ↑ Gal · 1-4GlcNAc | Galectin 3 | | |
| | CA 19-9 (MUC1) | ↑ T antigen | SBA | [37] | ABA |
| | CA 19-9 (MUC1) | ↑ Galβ1-3GalNAc | PNA | [37] | ABA, Jacalin |
| | CA 19-9 (MUC1) | ↑ antennary fucose | UEA | [37] | TJA II, AAL, LCA, PSL, AAA, LTA |
| | CA 19-9 (MUC1) | ↑ α2-3Neu5Ac | MAA | [37] | anti-α2-3-linked sialic acid antibody (i.e. HYB4), Siglec 1, Siglec 4 or Siglec 8 |
| | CA 19-9 (MUC1) | ↑ α2-6Neu5Ac | SNA | [37] | TJA-I |
| | CA 19-9 (MUC1) | ↓ tri-, tetra-antennary glycans | PHA-E, PHA-L | [37] | DBA |
| | CA 19-9 (MUC1) | ↑ terminal GalNAc | MGBL | [37] | DBA, SBA, HPA, WFA |
| | Complement C3 (UniProtKB: P01024) | ↑ antennary fucose | AAL | [38] | TJA II, UEA-I, LCA, PSL, AAA, LTA |
| | Complement C3 (UniProtKB: P01024) | ↑ Gal *β*1-3GalNAc | PNA | [38] | ABA, Jacalin |
| | Complement C3 (UniProtKB: P01024) | ↑ α2-3Neu5Ac | MAA | [38] | anti-α2-3-linked sialic acid antibody (i.e. HYB4), Siglec 1, Siglec 4 or Siglec 8 |
| | Complement C3 (UniProtKB: P01024) | ↑ α2-6Neu5Ac | SNA | [38] | TJA-I |
| | Kininogen-I (UniProtKB: P01042) | ↑ high mannose | Con A | [38] | NPA, GNA |
| | Kininogen-I (UniProtKB: P01042) | ↑ antennary fucose | AAL | [38] | TJA II, UEA-I, LCA, PSL, AAA, LTA |
| | Kininogen-I (UniProtKB: P01042) | ↑ Gal *β*1-3GalNAc | PNA | [38] | ABA, Jacalin |
| | Kininogen-I (UniProtKB: P01042) | ↑ α2-3Neu5Ac | MAA | [38] | anti-α2-3-linked sialic acid antibody (i.e. HYB4), Siglec 1, Siglec 4 or Siglec 8 |
| | Kininogen-I(UniProtKB: P01042) | ↑ α2-6Neu5Ac | SNA | [38] | TJA-I |
| | Histidine-rich glycoprotein (UniProtKB: P04196) | ↑ antennary fucose | AAL | [38] | TJA II, UEA-I, LCA, PSL, AAA, LTA |
| | Histidine-rich glycoprotein (UniProtKB: | ↑ α2-6Neu5Ac | SNA | [38] | TJA-I |
| | P04196) | | | | |
| **Pancreatic** | α₁-β-glycoprotein | ↑ Neu5Ac | SNA | [39] | TJA-I, anti-α2-3-linked sialic acid antibody (i.e. HYB4), Siglec 1, Siglec 4 or Siglec 8 |
| | Amyloid p-component | ↑ Neu5Ac | SNA | [39] | TJA-I, anti-α2-3-linked sialic acid antibody (i.e. HYB4), Siglec 1, Siglec 4 or Siglec 8 |
| | β-2-glycoprotein 1 (P02749) | ↑ antennary fucose | AAL | [40] | TJA II, UEA-I, LCA, PSL, AAA, LTA |
| | β-2-glycoprotein 1 (UniProtKB: P02749) | ↑ α2-3Neu5Ac | MAA | [40] | anti-α2-3-linked sialic acid antibody (i.e. HYB4), Siglec 1, Siglec 4 or Siglec 8 |
| | β-2-glycoprotein 1 (UniProtKB: P02749) | ↑ α2-6Neu5Ac | SNA | [40] | TJA-I |
| | β-2-glycoprotein 1 (UniProtKB: P02749) | ↑ high mannose | Con A | [40] | NPA, GNA |
| | β-2-glycoprotein 1 (UniProtKB: P02749) | ↑ Gal *β*1-3GalNAc | PNA | [40] | ABA, Jacalin |
| | hemopexin (UniProtKB: P02790) | ↑ antennary fucose | AAL | [40] | TJA II, UEA-I, LCA, PSL, AAA, LTA |
| | hemopexin (UniProtKB: P02790) | ↑ α2-3Neu5Ac | MAA | [40] | anti-α2-3-linked sialic acid antibody (i.e. HYB4), Siglec 1, Siglec 4 or Siglec 8 |
| | hemopexin (UniProtKB: P02790) | ↑ α2-6Neu5Ac | SNA | [40] | TJA-I |
| | hemopexin (UniProtKB: P02790) | ↑ high mannose | Con A | [40] | NPA, GNA |
| | haptoglobin-related protein (UniProtKB: P00739) | ↑ antennary fucose | AAL | [40] | TJA II, UEA-I, LCA, PSL, AAA, LTA |
| | haptoglobin-related protein (UniProtKB: P00739) | ↑ α2-3Neu5Ac | MAA | [40] | anti-α2-3-linked sialic acid antibody (i.e. HYB4), Siglec 1, Siglec 4 or Siglec 8 |
| | haptoglobin-related protein (UniProtKB: P00739) | ↑ α2-6Neu5Ac | SNA | [40] | TJA-I |
| | haptoglobin-related protein (UniProtKB: P00739) | ↑ high mannose | Con A | [40] | NPA, GNA |
| | haptoglobin-related protein (UniProtKB: P00739) | ↑ Gal *β* 1-3GalNAc | PNA | [40] | ABA, Jacalin |
| | serum amyloid P-component (UniProtKB: P02743) | ↑ antennary fucose | AAL | [40] | TJA II, UEA-I, LCA, PSL, AAA, LTA |
| | serum amyloid P-component (UniProtKB: P02743) | ↑ α2-3Neu5Ac | MAA | [40] | anti-α2-3-linked sialic acid antibody (i.e. HYB4), Siglec 1, Siglec 4 or Siglec 8 |
| | serum amyloid P-component (UniProtKB: P02743) | ↑ α2-6Neu5Ac | SNA | [40] | TJA-I |
| | serum amyloid P-component (UniProtKB: P02743) | ↑ high mannose | Con A | [40] | NPA, GNA |
| | serum amyloid P-component (UniProtKB: P02743) | ↑ Gal *β* 1-3GalNAc | PNA | [40] | ABA, Jacalin (DSA) |
| | clusterin (UniProtKB: P10909) | ↑ antennary fucose | AAL | [40] | TJA II, UEA-I, LCA, PSL, AAA, LTA |
| | clusterin (UniProtKB: P10909) | ↑ α2-3Neu5Ac | MAA | [40] | anti-α2-3-linked sialic acid antibody (i.e. HYB4), Siglec 1, Siglec 4 or Siglec 8 |
| | clusterin (UniProtKB: P10909) | ↑ α2-6Neu5Ac | SNA | [40] | TJA-I |
| | clusterin (UniProtKB: P10909) | ↑ Gal *β* 1-3GalNAc | PNA | [40] | ABA, Jacalin |
| | antithrombin-III (UniProtKB: P01008) | ↑ antennary fucose | AAL | [40] | TJA II, UEA-I, LCA, PSL, AAA, LTA |
| | antithrombin-III (UniProtKB: P01008) | ↑ α2-3Neu5Ac | MAA | [40] | anti-α2-3-linked sialic acid antibody (i.e. HYB4), Siglec 1, Siglec 4 or Siglec 8 |
| | antithrombin-III (UniProtKB: P01008) | ↑ α2-6Neu5Ac | SNA | [40] | TJA-I |
| | antithrombin-III (UniProtKB: P01008) | ↑ high mannose | Con A | [40] | NPA, GNA |
| | antithrombin-III (UniProtKB: P01008) | ↑ Gal *β* 1-3GalNAc | PNA | [40] | ABA, Jacalin (DSA) |
| | kininogen-1 (UniProtKB: P01042) | ↑ antennary fucose | AAL | [40] | TJA II, UEA-I, LCA, PSL, AAA, LTA |
| | kininogen-1 (UniProtKB: P01042) | ↑ α2-3Neu5Ac | MAA | [40] | anti-α2-3-linked sialic acid antibody (i.e. HYB4), Siglec 1, Siglec 4 or Siglec 8 |
| | kininogen-1 (UniProtKB: P01042) | ↑ α2-6Neu5Ac | SNA | [40] | TJA-I |
| | kininogen-1 (UniProtKB: P01042) | ↑ high mannose | Con A | [40] | NPA, GNA |
| | kininogen-1 (UniProtKB: P01042) | ↑ Gal *β* 1-3GalNAc | PNA | [40] | ABA, Jacalin (DSA) |
| | plasma protease C1 inhibitor (UniProtKB: P05155) | ↑ α2-6Neu5Ac | SNA | [40] | TJA-I |
| | β-haptoglobin | ↑ antennary fucose | AAL | [41] | TJA II, UEA-I, LCA, PSL, AAA, LTA |
| | β-haptoglobin | ↑ antennary fucose | AAL | [42] | TJA II, UEA-I, LCA, PSL, AAA, LTA |
| | β-haptoglobin | ↑ core fucose | AOL | [41] | PhoSL |
| | β-haptoglobin | ↑ core fucose | PhoSL | [43] | AOL |
| | α-1-antichymotrypsin | ↑ antennary fucose | AAL | [42] | TJA II, UEA-I, LCA, PSL, AAA, LTA |
| | thrombospondin-1 | ↑ antennary fucose | AAL | [42] | TJA II, UEA-I, LCA, PSL, AAA, LTA |
| | α-1-antitrypsin | ↑ antennary fucose | AAL | [42] | TJA II, UEA-I, LCA, PSL, AAA, LTA |
| | Mucin (CAM 17.1) | ↑ *β*-D-GlcNAc, Neu5Ac | WGA | [44, 45] | DSA, LEL, SNA, TJA-I |
| | MUC16 | ↑ antennary fucose | AAL | [46, 47] | TJA II, UEA-I, LCA, PSL, AAA, LTA |
| | MUC16 | ↓ T antigen | BPL, Jacalin (DSA), PNA | [46] | SBA, VVA, ABA, GSL1, SJA |
| | MUC16 | ↓ Gal-GlcNAc | ECL, PHA-L | [46] | PHA-E, AlloA, ECA, |
| | MUC16 | ↓ GalNAc | DBA, GSL1, SBA, VVL, SJA | [46] | ABA, BPL, PNA |
| | MUC16 | ↓ GIcNAc | GSL2, STL | [46] | DSA, LEL, WGA |
| | MUC16 | ↓ mannose | Con A | [46] | GNA, NPA |
| | MUC5ac | ↑ T antigen | Jacalin | [46] | SBA, ABA, VVA, BPL, PNA |
| | MUC5ac | ↑ antennary fucose | AAL | [46] | TJA II, EA-I, LCA, PSL, AAA, LTA |
| | MUC5ac | ↑ T antigen | Jacalin (DSA) | [46] | SBA, ABA, VVA, BPL, PNA, GSL1, SJA |
| | MUC5ac | ↓ Gal-GlcNAc | ECA, PHA-L, RCA120 | [46] | PHA-E, RCA |
| | MUC5ac | ↓ GalNAc | DBA, VVA, SJA | [46] | GSL1, SBA, ABA, BPL, PNA |
| | MUC5ac | ↓ GlcNAc | GSL 2, LEL, STL | [46] | DSA, LEL, WGA, GSL2, STL |
| | MUC1 | ↓ Gal-GlcNAc, tetra-antennary glycans | PHA-L | [46] | ECA, PHA-L, RCA120, PHA-E, RCA; DBA |
| | MUC1 | ↓ T antigen | Jacalin (DSA) | [46] | SBA, ABA, VVA, BPL, PNA, GSL1, SJA |
| | MUC1 | ↓ GalNAc | DBA | [46] | VVA, SJA, GSL1, SBA, ABA, BPL, PNA |
| | MUC1 | ↑ Gal *α* 1-3Gal | GSL1 | [46] | |
| | MUC1 | ↓ GlcNAc | GSL 2, LEL, STL | [46] | DSA, LEL, WGA, GSL2, STL |
| | | | | | |
| Thyroid | Thyroglobulin (TG) | ↓ antennary fucose | LCA | [48, 49] | TJA II, AAL, UEA-I, PSL, AAA, LTA |
| | TG | ↑ terminal galactose | RCA | [50] | RCA120, ABA, AlloA, Jacalin (DSA), ECL, PNA |
| | TG | ↑ Gal-GIcNAc | LC assays | [50] | ECA, PHA-L, RCA120, PHA-E, RCA |
| | TG | ↑ tri-antennary glycans | LC assays | [50] | PHA-E, PHA-L, DBA |
| | TG | ↑ antennary fucose | LC assays | [50] | TJA II, AAL, UEA-I, LCA, PSL, AAA, LTA |
| | TG | ↑ mannose | LC assays | [50] | Con A, NPA, GNA |
| | | | | | |
| Liver | α₁,-antitrypsin (AAT) | ↑ antennary fucose | LCA | [51] | TJA II, UEA-I, AAL, PSL, AAA, LTA |
| | α₁-antitrypsin (AAT) | ↑ antennary fucose | AAL | [52, 53] | TJA II, UEA-I, LCA, PSL, AAA, LTA |
| | α-fetoprotein (AFP) | ↑ antennary fucose | LCA | [51, 54] | TJA II, AAL, UEA-I, PSL, AAA, LTA |
| | α-fetoprotein (AFP) | ↑ antennary fucose | AAL | [54] | TJA II, UEA-I, LCA, PSL, AAA, LTA |
| | AFP-L3 | ↑ antennary fucose | LCA | [55, 56] | TJA II, UEA-I, PSL, AAA, LTA |
| | transferrin | ↑ antennary fucose | LCA | [51] | TJA II, UEA-I, PSL, AAA, LTA |
| | α₁-antichymotrypsin (AAT) | ↑ antennary fucose | AAL | [52] | TJA II, UEA-I, LCA, PSL, AAA, LTA |
| | α-1-acid | ↑ antennary | AAL | [52] | TJA II, |
| | glycoprotein 1 | fucose | | | UEA-I, LCA, PSL, AAA, LTA |
| | ceruloplasmin | ↑ antennary fucose | AAL | [52] | TJA II, UEA-I, LCA, PSL, AAA, LTA |
| | α-2-macroglobulin | ↑ antennary fucose | AAL, LCA | [54] | TJA II, UEA-I, PSL, AAA, LTA |
| | α-2-HS-glycoprotein | ↑ antennary fucose | AAL | [53] | TJA II, UEA-I, LCA, PSL, AAA, LTA |
| | Fetuin A | ↑ antennary fucose | AAL | [57] | TJA II, UEA-I, LCA, PSL, AAA, LTA |
| | hemopexin | ↑ antennary fucose | AAL | [54, 57] | TJA II, UEA-I, LCA, PSL, AAA, LTA |
| | hemopexin | ↑ antennary fucose | LCA | [54] | TJA II, AAL, UEA-I, PSL, AAA, LTA |
| | Ceruloplasmin | ↑ antennary fucose | AAL, LCA | [58] | TJA II, UEA-I, PSL, AAA, LTA |
| | C3 complement | ↑ antennary fucose | AAL, LCA | [58] | TJA II, UEA-I, PSL, AAA, LTA |
| | Histidine rich glycoprotein | ↑ antennary fucose | AAL, LCA | [58] | TJA II, UEA-I, PSL, AAA, LTA |
| | Monocyte differentiation antigen CD14 | ↑ antennary fucose | AAL, LCA | [58] | TJA II, UEA-I, PSL, AAA, LTA |
| | Hepatocyte growth factor activator | ↑ antennary fucose | AAL, LCA | [58] | TJA II, UEA-I, PSL, AAA, LTA |
| | | | | | |
| **Lung** | β-haptoglobin | ↑ antennary fucose | AAL | [59] | TJA II, UEA-I, PSL, AAA, LCA, LTA |
| | β-haptoglobin | ↑ antennary fucose | AAL | [59] | TJA II, UEA-I, PSL, AAA, LCA, LTA |
| | β-haptoglobin | ↑ antennary fucose | MS | [60] | AAL, TJA II, UEA-I, LCA, PSL, AAA, LTA |
| | β-haptoglobin | ↑ core fucose | MS | [60] | AOL, PhoSL |
| | β-haptoglobin | ↑ tri-, tetra-antennary glycans | MS | [60] | PHA-E, PHA-L, DBA |
| | β-haptoglobin | ↑ α2-6Neu5Ac | MS | [61] | SNA, TJA-I |
| | β-haptoglobin | ↑ antennary fucose | MS | [61] | AAL, TJA II, UEA-I, LCA, PSL, AAA, LTA |
| | β-haptoglobin | ↑ sialyl Le^{x} | LC | [62] | SNA, TJA-I, MAA, anti-α2-3-linked sialic acid antibody (i.e. HYB4), Siglec 1, Siglec 4 or Siglec 8 |
| | β-haptoglobin | ↑ tri-antennary | LC | [62] | PHA-E, PHA-L, DBA |
| | β-haptoglobin | ↑ sialic acid | LC | [62] | SNA, TJA-I, MAA, anti-α2-3-linked sialic acid antibody (i.e. HYB4), Siglec 1, Siglec 4 or Siglec 8 |
| | fibronectin | ↑ Galβ1-3GalNAc | PNA | [63] | ABA, Jacalin (DSA) |
| | α₁-acid glycoprotein | ↑ antennary fucose | AAL | [64] | TJA II, UEA-I, LCA, PSL, AAA, LTA |
| | α₁-acid glycoprotein | ↑ sialyl Le^{x} | Antibody against sLe^{x} | [64] | SNA, TJA-I, MAA, anti-α2-3-linked sialic acid antibody (i.e. HYB4), Siglec 1, Siglec 4 or Siglec 8 |
| | α-1-antitrypsin | ↑ antennary fucose | AAL | [65] | TJA II, UEA-I, LCA, PSL, AAA, LTA |
| | α-1-antitrypsin | ↑ β-Gal, Galβ1-4GIcNAc | RCA120 | [65] | RCA, ECL, AlloA |
| | α-1-antitrypsin | ↑ α-Gal and α-GalNAc | BS-I | [65] | DBA, SBA, HPA |
| | α-1-antitrypsin | ↑ (GlcNAc)ₙ | WGA | [65] | LEL |
| | α-1-antitrypsin | ↑ Branched (LacNAc)ₙ | PWM | [65] | |
| | α-1-antitrypsin | ↑ high-mannose, Manα1-3Man | GNA | [65] | Con A, NPA |
| | | | | | |
| **Stomach** | α₁-acid glycoprotein | ↑ bi-antennary glycans | Con A | [66] | NPA, GNA |
| | α₁-acid glycoprotein | ↓ galactose | | [66] | RCA, RCA120, ABA, AlloA, Jacalin (DSA), ECL, PNA |
| | α₁-acid glycoprotein | ↑ Le^{x} | | [66] | LTA |
| | β-haptoglobin | ↑ sialyl Le^{x} (sLe^{x}) | anti-sLe^{x} mouse monoclonal KM93 antibody | [67] | SNA, TJA-I, MAA, anti-α2-3-linked sialic acid antibody (i.e. HYB4), Siglec 1, Siglec 4 or Siglec 8 |
| | β-haptoglobin | ↑ tri-, tetra-antennary glycans | LC/MS | [68] | PHA-E, PHA-L, DBA |
| | β-haptoglobin | ↑ antennary fucose | LC/MS | [68] | AAL, TJA II, UEA-I, LCA, PSL, AAA, LTA |
| | β-haptoglobin | ↑ sialyl-Le^{a} (sLe^{a}) | LC/MS | [68] | Antibody against sLe^{a}, SNA, TJA-I, MAA, anti-α2-3-linked sialic acid antibody (i.e. HYB4), Siglec 1, Siglec 4 or Siglec 8 |
| | β-haptoglobin | ↑ sialyl-Le^{a} (sLe^{a}) | LC/MS | [68] | Antibody against sLe^{a}, SNA, TJA-I, MAA, anti-α2-3-linked sialic acid antibody (i.e. HYB4) |
| | β-haptoglobin | ↑ antennary fucose | AAL | [68] | TJA II, UEA-I, LCA, PSL, AAA, LTA |
| | β-haptoglobin | ↑ (GlcNAc)ₙ | WGA | [68] | LEL |
| | β-haptoglobin | ↓ high mannose | Con A | [68] | NPA, GNA |
| | leucine-rich-α2- | ↑ sialyl Le^{x} ↑ (sLe^{x}) | anti-sLe^{x} mouse monoclonal KM93 antibody | [67] | Antibody against sLe^{a}, SNA, TJA-I, MAA, anti-α2-3-linked sialic acid antibody (i.e. HYB4), Siglec 1, Siglec 4 or Siglec 8 |
| | glycoprotein | | | | |
| | | | | | |
| **Testicular** | Human chorionic gonadotropin-*β* | T fucose | LC-MS | [69] | AAL, TJA II, UEA-I, LCA, PSL, AAA, LTA, PhoSL, AOL |
| | Human chorionic gonadotropin-*β* | ↑ tri-antennary glycans | LC-MS | [69] | PHA-E, PHA-L, DBA |
| | AFP-L3 | ↑ antennary fucose | LCA | [70] | AAL, TJA II, UEA-I, LCA, PSL, AAA, LTA |
| | | | | | |
| **Bladder** | MUC1 | ↑ antennary fucose | AAL | [71, 72] | TJA II, UEA-I, LCA, PSL, AAA, LTA |
| | endoplasmin (HSP90B1) | ↑ antennary fucose | AAL | [71, 72] | TJA II, UEA-I, LCA, PSL, AAA, LTA |
| | Golgi apparatus protein 1 (GLG1) | ↑ antennary fucose | AAL | [71, 72] | TJA II, UEA-I, LCA, PSL, AAA, LTA |
| | prostatic acid phosphatase (ACPP) | ↑ antennary fucose | AAL | [71, 72] | TJA II, UEA-I, LCA, PSL, AAA, LTA |
| | Ig gamma-2 chain C region (IGHG2) | ↑ antennary fucose | AAL | [71, 72] | TJA II, UEA-I, LCA, PSL, AAA, LTA |
| | deoxyribonuclease-2-alpha (DNASE2A) | ↑ antennary fucose | AAL | [71, 72] | TJA II, UEA-I, LCA, PSL, AAA, LTA |
| | integrin | ↑ sialic acid | MS | [73] | SNA, TJA-I, MAA, anti-α2-3-linked sialic acid antibody (i.e. HYB4), Siglec 1, Siglec 4 or Siglec 8 |
| | integrin | ↑ tetra-antennary glycans | MS | [73] | PHA-E, PHA-L, DBA |
| | MUC16 | ↑ sialyl Tn | LC/MS | [74] | SNA, TJA-I, MAA, anti-α2-3-linked sialic acid antibody (i.e. HYB4) |
| | α-1-antitrypsin | T high mannose | Con A | [75] | NPA, GNA |
| | α-1-antitrypsin | ↑ (GlcNAcβ1-4)ₙ | WGA | [75] | LEL |

Due to their superior sensitivity and/or specificity magnetic carriers, anti-glycoprotein antibodies, the antigen binding portions thereof, one or more lectins, compositions, kits and methods and uses based thereon of the present invention are particularly suitable for isoform-specific detection and analysis of glycoproteins (e.g., in diagnostics of cancer).

In some aspects the invention relates to, an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody, preferably anti-PSA antibody) or an antigen binding portion thereof (e.g., a single chain antibody fragment (scAb)) immobilized (e.g., conjugated) on a magnetic carrier (e.g., a magnetic particle or magnetic bead), wherein said magnetic carrier further comprises a polypeptide having peroxidase activity (e.g., polypeptide having EC 1.11.1.7 enzymatic activity, e.g., microperoxidase-11). Further preferred antibodies relating to the present invention are shown in Table 1.

In some aspects the invention relates to, an anti-glycoprotein antibody or the antigen binding portion thereof, wherein said polypeptide having peroxidase activity has a molecular weight of less than 2 kDa (e.g., about 1.5 or about 1.6 or about 1.9 kDa), preferably a molecular weight between 1 and 2 kDa.

In some aspects the invention relates to, an anti-glycoprotein antibody or the antigen binding portion thereof, wherein said polypeptide having peroxidase activity is immobilized (e.g., conjugated) on said magnetic carrier.

In some aspects the invention relates to, an anti-glycoprotein antibody or the antigen binding portion thereof, wherein said anti-glycoprotein antibody or the antigen binding portion thereof specifically binds to a target polypeptide comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) Prostate-specific antigen (PSA), preferably SEQ ID NOs: 1, 2, 3, 4, 5 or 6; further preferably SEQ ID NO: 6; ii) Alpha-fetoprotein (AFP), preferably SEQ ID NOs: 11 or 12; further preferably SEQ ID NO: 12; iii) Mucin-16 (MUC16), preferably SEQ ID NO: 13; iv) WAP four-disulfide core domain protein 2 (WFDC2), preferably SEQ ID NOs: 14, 15, 16, 17, 18 or 19; further preferably SEQ ID NO: 19; v) Mucin-1 (MUC1), preferably SEQ ID NOs: 20, 21, 22, 23, 24, 25, 26, 27, 28, 29, 30, 31, 32, 33, 34, 35, 36 or 37; further preferably SEQ ID NO: 37; vi) Receptor tyrosine-protein kinase erbB-2 (ERBB2), preferably SEQ ID NOs: 38, 39, 40, 41, 42, 43, or 44; further preferably SEQ ID NO: 44; vii) Carcinoembryonic antigen-related cell adhesion molecule 5 (CEACAM5), preferably SEQ ID NOs: 45, 46, or 47; further preferably SEQ ID NO: 47; viii) Galactoside 3(4)-L-fucosyltransferase (FUT3), preferably SEQ ID NO: 48; ix) Thyroglobulin (TG), preferably SEQ ID NOs: 49, 50, or 51; further preferably SEQ ID NO: 51.

In some aspects the invention relates to, an anti-glycoprotein antibody or the antigen binding portion thereof, wherein said target polypeptide is a prostate-specific antigen (PSA) having peptidase activity (e.g., EC 3.4.21.77 enzymatic activity).

In some aspects the invention relates to, an anti-glycoprotein antibody or the antigen binding portion thereof, wherein said anti-glycoprotein antibody or the antigen binding portion thereof specifically binds to a target polypeptide (e.g., PSA) comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 6; preferably said polypeptide has SEQ ID NO: 6; ii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 1; preferably said polypeptide has SEQ ID NO: 1; iii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 2; preferably said polypeptide has SEQ ID NO: 2; iv) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 3; preferably said polypeptide has SEQ ID NO: 3; v) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 4; preferably said polypeptide has SEQ ID NO: 4; vi) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 5; preferably said polypeptide has SEQ ID NO: 5.

In some aspects the invention relates to, an anti-glycoprotein antibody or the antigen binding portion thereof, wherein said target polypeptide is human, rabbit, rat or mouse, preferably said target polypeptide is human.

In some aspects the invention relates to, an antibody or the antigen binding portion thereof, wherein said polypeptide having peroxidase activity comprises a microperoxidase (MP) (e.g., microperoxidase-11).

In some aspects the invention relates to, an antibody or the antigen binding portion thereof, wherein said microperoxidase (MP) is a heme containing peptide portion of cytochrome c (e.g., shown as SEQ ID NO: 10, cytochrome c derived from *Equus caballus,* NCBI Reference Sequence: NP_001157486.1) that retains peroxidase activity (e.g., EC 1.11.1.7 enzymatic activity, e.g., microperoxidase-11).

In some aspects the invention relates to, an antibody or the antigen binding portion thereof, wherein said heme containing peptide portion of cytochrome c is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 7 (MP-11 peptide), SEQ ID NO: 8 (MP-9 peptide) and SEQ ID NO: 9 (MP-8 peptide), preferably said microperoxidase (MP) peptide is selected from the group consisting of: SEQ ID NO: 7 (MP-11 peptide), SEQ ID NO: 8 (MP-9 peptide) and SEQ ID NO: 9 (MP-8 peptide).

In some aspects the invention relates to, an antibody or the antigen binding portion thereof, wherein said magnetic carrier is a magnetic particle or magnetic bead.

In some aspects the invention relates to, an anti-glycoprotein antibody or the antigen binding portion thereof, wherein said anti-glycoprotein antibody or the antigen binding portion thereof is capable of simultaneous binding to said target polypeptide (e.g., PSA, AFP, MUC16, WFDC2, MUC1, ERBB2, CEACAM5, FUT3 or TG) in a sample and generating a detection signal (e.g., by the optical means).

In some aspects the invention relates to, an anti-glycoprotein antibody or the antigen binding portion thereof, wherein said anti-glycoprotein antibody or the antigen binding portion thereof is capable of simultaneous binding and enriching said target polypeptide (e.g., PSA, AFP, MUC16, WFDC2, MUC1, ERBB2, CEACAM5, FUT3 or TG) in a sample and generating a detection signal (e.g., by the optical means).

In some aspects the invention relates to, an anti-glycoprotein antibody or the antigen binding portion thereof, wherein said anti-glycoprotein or the antigen binding portion thereof is capable of detecting said target polypeptide (e.g., PSA, AFP, MUC16, WFDC2, MUC1, ERBB2, CEACAM5, FUT3 or TG) in a sample (e.g., serum sample) having said target polypeptide in an amount corresponding to 0.04 mL or less of undiluted biological sample (e.g., undiluted serum sample), preferably in the range between 0.01-0.04 mL, further preferably in the range between 0.02-0.04 mL, most preferably in the range between 0.02-0.04 mL).

In some aspects the invention relates to, an anti-glycoprotein antibody or the antigen binding portion thereof, wherein said anti-glycoprotein antibody is a monoclonal antibody.

In some aspects the invention relates to, an anti-glycoprotein antibody or the antigen binding portion thereof, wherein said anti-glycoprotein antibody is selected from the group consisting of: chimeric, humanized or human antibody.

In some aspects the invention relates to, a magnetic carrier (e.g., a magnetic particle or magnetic bead) comprising: i) an immobilized (e.g., conjugated) anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody) or an antigen binding portion thereof (e.g., a single chain antibody fragment (scAb)) and ii) a polypeptide having peroxidase activity (e.g., EC 1.11.1.7 enzymatic activity, e.g., microperoxidase-11).

In some aspects the invention relates to, a magnetic carrier, wherein said polypeptide having peroxidase activity has a molecular weight of less than 2 kDa (e.g., about 1.5 or about 1.6 or about 1.9 kDa), preferably a molecular weight between 1 and 2 kDa.

In some aspects the invention relates to, a magnetic carrier, wherein said polypeptide having peroxidase activity is immobilized (e.g., conjugated) on said magnetic carrier.

In some aspects the invention relates to, a magnetic carrier, wherein said anti-glycoprotein antibody or the antigen binding portion thereof specifically binds to a target polypeptide comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) Prostate-specific antigen (PSA), preferably SEQ ID NOs: 1, 2, 3, 4, 5 or 6; further preferably SEQ ID NO: 6; ii) Alpha-fetoprotein (AFP), preferably SEQ ID NOs: 11 or 12; further preferably SEQ ID NO: 12; iii) Mucin-16 (MUC16), preferably SEQ ID NO: 13; iv) WAP four-disulfide core domain protein 2 (WFDC2), preferably SEQ ID NOs: 14, 15, 16, 17, 18 or 19; further preferably SEQ ID NO: 19; v) Mucin-1 (MUC1), preferably SEQ ID NOs: 20, 21, 22, 23, 24, 25, 26, 27, 28, 29, 30, 31, 32, 33, 34, 35, 36 or 37; further preferably SEQ ID NO: 37; vi) Receptor tyrosine-protein kinase erbB-2 (ERBB2), preferably SEQ ID NOs: 38, 39, 40, 41, 42, 43, or 44; further preferably SEQ ID NO: 44; vii) Carcinoembryonic antigen-related cell adhesion molecule 5 (CEACAM5), preferably SEQ ID NOs: 45, 46, or 47; further preferably SEQ ID NO: 47; viii) Galactoside 3(4)-L-fucosyltransferase (FUT3), preferably SEQ ID NO: 48; ix) Thyroglobulin (TG), preferably SEQ ID NOs: 49, 50, or 51; further preferably SEQ ID NO: 51.

In some aspects the invention relates to, a magnetic carrier, said target polypeptide is a prostate-specific antigen (PSA) having peptidase activity (e.g., EC 3.4.21.77 enzymatic activity).

In some aspects the invention relates to, a magnetic carrier, wherein said anti-glycoprotein antibody or the antigen binding portion thereof specifically binds to a target polypeptide (e.g., PSA) comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 6; preferably said polypeptide has SEQ ID NO: 6; ii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 1; preferably said polypeptide has SEQ ID NO: 1; iii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 2; preferably said polypeptide has SEQ ID NO: 2; iv) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 3; preferably said polypeptide has SEQ ID NO: 3; v) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 4; preferably said polypeptide has SEQ ID NO: 4; vi) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 5; preferably said polypeptide has SEQ ID NO: 5.

In some aspects the invention relates to, a magnetic carrier, wherein said target polypeptide is human, rabbit, rat or mouse, preferably said glycoprotein is human.

In some aspects the invention relates to, a magnetic carrier, wherein said polypeptide having peroxidase activity comprises a microperoxidase (MP) (e.g., microperoxidase-11).

In some aspects the invention relates to, a magnetic carrier, wherein said microperoxidase (MP) is a heme containing peptide portion of cytochrome c (e.g., shown as SEQ ID NO: 10, cytochrome c derived from *Equus caballus,* NCBI Reference Sequence: NP_001157486.1) that retains peroxidase activity (e.g., EC 1.11.1.7 enzymatic activity, e.g., microperoxidase-11).

In some aspects the invention relates to, a magnetic carrier, said heme containing peptide portion of cytochrome c is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 7 (MP-11 peptide), SEQ ID NO: 8 (MP-9 peptide) and SEQ ID NO: 9 (MP-8 peptide), preferably said microperoxidase (MP) peptide is selected from the group consisting of: SEQ ID NO: 7 (MP-11 peptide), SEQ ID NO: 8 (MP-9 peptide) and SEQ ID NO: 9 (MP-8 peptide).

In some aspects the invention relates to, a magnetic carrier, wherein said magnetic carrier is a magnetic particle or magnetic bead.

In some aspects the invention relates to, a magnetic carrier, said magnetic carrier is capable of simultaneous binding to said target polypeptide (e.g., PSA, AFP, MUC16, WFDC2, MUC1, ERBB2, CEACAM5, FUT3 or TG) in a sample and generating a detection signal (e.g., by the optical means).

In some aspects the invention relates to, a magnetic carrier, said magnetic carrier is capable of simultaneous binding and enriching said target polypeptide (e.g., PSA, AFP, MUC16, WFDC2, MUC1, ERBB2, CEACAM5, FUT3 or TG) in a sample and generating a detection signal (e.g., by the optical means).

In some aspects the invention relates to, a magnetic carrier, wherein said magnetic carrier is capable of detecting said target polypeptide (e.g., PSA, AFP, MUC16, WFDC2, MUC1, ERBB2, CEACAM5, FUT3 or TG) in a sample (e.g., serum sample) having said target polypeptide in an amount corresponding to 0.04 mL or less of undiluted biological sample (e.g., undiluted serum sample), preferably in the range between 0.01-0.04 mL, further preferably in the range between 0.02-0.04 mL, most preferably in the range between 0.02-0.04 mL).

In some aspects the invention relates to, a magnetic carrier, wherein said anti-glycoprotein antibody is a monoclonal antibody.

In some aspects the invention relates to, a magnetic carrier, wherein said anti-glycoprotein antibody is selected from the group consisting of: chimeric, humanized or human antibody.

In some aspects the invention relates to, a method for producing an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody) or an antigen binding portion thereof, said method comprising simultaneously conjugating a magnetic carrier (e.g., magnetic particle or bead) to: i) an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody) or an antigen binding portion thereof, ii) a polypeptide having peroxidase activity.

In some aspects the invention relates to, a method for producing a magnetic carrier, said method comprising simultaneously conjugating said magnetic carrier (e.g., magnetic particle or bead) to: i) an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody) or an antigen binding portion thereof, ii) a polypeptide having peroxidase activity.

In some aspects the invention relates to, a method comprising: a) providing: i) a magnetic carrier or anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody) or antigen binding portion thereof; ii) one or more lectins, preferably said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL); further preferably said one or more lectins comprising MAA II, most preferably said one or more lectins are two lectins comprising MAA II, further most preferably said one or more lectins are two lectins comprising MAA II in combination with: aa) AAL, preferably said method has sensitivity of about 100% and/or said method has specificity of about 81.3%; or bb) Con A, preferably said method has sensitivity of about 100% and/or said method has specificity of about 93.8%; or cc) SNA-I, preferably said method has sensitivity of about 100% and/or said method has specificity of about 93.8%; b) determining the quantity, presence, or absence of oligosaccharide chains (e.g., glycans) covalently attached to a target polypeptide of said anti-glycoprotein antibody (e.g., target polypeptide, e.g., PSA, AFP, MUC16, WFDC2, MUC1, ERBB2, CEACAM5, FUT3 or TG), preferably said target polypeptide is PSA, further preferably said determining comprises the use of a magnetic carrier and/or an anti-glycoprotein antibody and/or an antigen binding portion thereof and/or one or more lectins and/or a composition and/or kits based thereof.

In some aspects the invention relates to, a method for detection of a glycosylated polypeptide, wherein binding and detection (e.g., by the optical means) of said target polypeptide (e.g., PSA, AFP, MUC16, WFDC2, MUC1, ERBB2, CEACAM5, FUT3 or TG) is carried out simultaneously, preferably said target polypeptide is PSA.

In some aspects the invention relates to, a method for detection of a glycosylated polypeptide, wherein binding, enriching and detection (e.g., by the optical means) of said target polypeptide (e.g., PSA, AFP, MUC16, WFDC2, MUC1, ERBB2, CEACAM5, FUT3 or TG) is carried out simultaneously, preferably said target polypeptide is PSA.

In some aspects the invention relates to, a method for detection of a glycosylated polypeptide, wherein said method is the method for one or more of the following: i) for selective capture and/or enrichment of a target polypeptide (e.g., PSA, AFP, MUC16, WFDC2, MUC1, ERBB2, CEACAM5, FUT3 or TG), preferably said target polypeptide is PSA; ii) for selective capture and/or enrichment of a target polypeptide (e.g., PSA, AFP, MUC16, WFDC2, MUC1, ERBB2, CEACAM5, FUT3 or TG, preferably said target polypeptide is PSA), wherein said method for selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate); iii) for glycoprofiling of a target polypeptide (e.g., PSA, AFP, MUC16, WFDC2, MUC1, ERBB2, CEACAM5, FUT3 or TG, preferably said target polypeptide is PSA); iv) for screening and/or analysing oligosaccharide chains (e.g., glycans) covalently attached to a target polypeptide (e.g., PSA, AFP, MUC16, WFDC2, MUC1, ERBB2, CEACAM5, FUT3 or TG, preferably said target polypeptide is PSA); v) for diagnostics of cancer (e.g., prostate cancer); vi) for positive and/or negative prediction of cancer (e.g., prostate cancer); vii) for determining a clinical stage of cancer (e.g., prostate cancer); viii) for distinguishing between prostate cancer and metastasizing prostate cancer; ix) for identifying prostate cancer likely to metastasize (e.g. likely to metastasize to the bone); x) for distinguishing between benign prostatic hyperplasia (BPH) and prostate cancer; xi) for prevention and/or treatment of cancer (e.g., prostate cancer); xii) for discriminating between significant and insignificant tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xiii) for discriminating between slow growing (e.g., clinically harmless) and fast growing (e.g., clinically relevant) tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xiv) for identifying organ confined and/or potentially curable cancers (e.g., prostate cancer), e.g., by means of glycoprotein-based (e. g., target polypeptide-based, e.g., based on PSA, AFP, MUC16, WFDC2, MUC1, ERBB2, CEACAM5, FUT3 or TG, preferably said target polypeptide is PSA) diagnostics of cancer; xv) for screening compounds.

In some aspects the invention relates to, a method for screening compounds and said method comprising: a) providing: i) a prostate cancer sample with a test compound b) contacting said prostate cancer sample with said test compound; and c) determining the likelihood of said prostate cancer cell to metastasize based on the determined quantity, presence, or absence of oligosaccharide chains (e.g., glycans) covalently attached to a target polypeptide (e.g., PSA, AFP, MUC16, WFDC2, MUC1, ERBB2, CEACAM5, FUT3 or TG, preferably said target polypeptide is PSA) in said prostate cancer sample before and after contacting said prostate cancer sample with said test compound.

In some aspects the invention relates to, a method described herein, wherein said method is the method: i) for positive prediction of prostate cancer and/or ii) for distinguishing between benign prostatic hyperplasia (BPH) and prostate cancer; wherein said one or more lectins comprise MAA II or WFL, preferably said one or more lectins comprise MAA II.

In some aspects the invention relates to, a method described herein, wherein used one or more lectins is MAA II, wherein the sensitivity and/or specificity of said method is about 87.5%.

In some aspects the invention relates to, a method described herein, wherein used one or more lectins are two lectins comprising MAA II in combination with: i) Con A, wherein said method has sensitivity of about 100% and/or said method has specificity of is about 93.8%; or ii) SNA-I, wherein said method has sensitivity of about 100% and/or said method has specificity of is about 93.8%; or iii) AAL, wherein said method has sensitivity of about 100% and/or said method has specificity of is about 81.3%.

In some aspects the invention relates to, a method described herein, wherein used lectin is WFL, wherein the sensitivity of said method is about 50% and/or specificity of said method is about 75%.

In some aspects the invention relates to, a method described herein, wherein said method is the method: i) for negative prediction of prostate cancer and/or ii) for distinguishing between benign prostatic hyperplasia (BPH) and prostate cancer; wherein said lectin is selected from the group consisting of: Con A, AAL and SNA-I.

In some aspects the invention relates to, a method described herein, wherein used lectin is Con A, wherein the sensitivity of said method is about 50% and/or specificity of said method is about 75%.

In some aspects the invention relates to, a method described herein, wherein used lectin is AAL, wherein the sensitivity of said method is about 50% and/or specificity of said method is about 75%.

In some aspects the invention relates to, a method described herein, wherein used lectin is SNA-I, wherein the sensitivity of said method is about 57% and/or specificity of said method is about 75%.

In some aspects the invention relates to, a method described herein, wherein said method comprising the step of determining a treatment course of action based on determined quantity, presence, or absence of oligosaccharide chains (e.g., glycans) covalently attached to a target polypeptide of an anti-glycoprotein antibody (e.g., target polypeptide, e.g., PSA, AFP, MUC16, WFDC2, MUC1, ERBB2, CEACAM5, FUT3 or TG), preferably said target polypeptide is PSA.

In some aspects the invention relates to, a method described herein, wherein said method is the method for distinguishing between prostate cancer and metastasizing prostate cancer in a sample (e.g., in a serum sample), wherein used one or more lectins are selected from the group consisting of: AAL and Con A.

In some aspects the invention relates to, a method described herein, wherein said method is carried out in a sample.

In some aspects the invention relates to, a method described herein, a suitable sample is selected from the group of urine, blood, serum, biopsy and post-surgical tissue sample, preferably a serum sample.

In some aspects the invention relates to, a lectin for use in a method of the present invention, preferably for use in a method: i) for prediction (e.g., positive or negative) of prostate cancer; and/or ii) for distinguishing between benign prostatic hyperplasia (BPH) and prostate cancer; and/or iii) for distinguishing between prostate cancer and metastasizing prostate cancer.

In some aspects the invention relates to, one or more lectins, wherein said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL), preferably said one or more lectins comprising MAA II, further preferably said one or more lectins are two lectins comprising MAA II, most preferably said one or more lectins are two lectins comprising MAA II in combination with AAL, Con A or SNA-I.

In some aspects the invention relates to, one or more lectins, wherein said one or more lectins are immobilized (e.g., in a sample location, e.g., in a microplate, e.g., in an Enzyme-linked Immunosorbent Assay (ELISA), enzyme-linked lectin assay (ELLA) or magnetic enzyme-linked lectin assay (MELLA) microplate).

In some aspects the invention relates to, a composition comprising one or more of the following: i) an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody, preferably anti-PSA antibody) or an antigen binding portion thereof; ii) a magnetic carrier; iii) one or more lectins, preferably said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL); further preferably said one or more lectins comprising MAA II, most preferably said one or more lectins are two lectins comprising MAA II, further most preferably said one or more lectins are two lectins comprising MAA II in combination with AAL, Con A or SNA-I.

In some aspects the invention relates to, a kit comprising an anti-glycoprotein antibody (e.g., anti-PSA, anti-AFP, anti-MUC16, anti-WFDC2, anti-MUC1, anti-ERBB2, anti-CEACAM5, anti-FUT3 or anti-TG), preferably anti-PSA antibody), antigen binding portion thereof, magnetic carrier, one or more lectins or composition.

In some aspects the invention relates to, an anti-glycoprotein antibody (e.g., anti-PSA, anti-AFP, anti-MUC16, anti-WFDC2, anti-MUC1, anti-ERBB2, anti-CEACAM5, anti-FUT3 or anti-TG), preferably anti-PSA antibody), antigen binding portion thereof, magnetic carrier, one or more lectins or composition for use as a medicament.

In some aspects the invention relates to, an anti-glycoprotein antibody (e.g., anti-PSA, anti-AFP, anti-MUC16, anti-WFDC2, anti-MUC1, anti-ERBB2, anti-CEACAM5, anti-FUT3 or anti-TG), preferably anti-PSA antibody), antigen binding portion thereof, magnetic carrier, one or more lectins or composition for use in one or more of the following methods (e.g., in vitro, in vivo or ex vivo methods): i) for selective capture and/or enrichment of a target polypeptide (e.g., PSA, AFP, MUC16, WFDC2, MUC1, ERBB2, CEACAM5, FUT3 or TG), preferably said target polypeptide is PSA; ii) for selective capture and/or enrichment of a target polypeptide (e.g., PSA, AFP, MUC16, WFDC2, MUC1, ERBB2, CEACAM5, FUT3 or TG, preferably said target polypeptide is PSA), wherein said method for selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate); iii) for glycoprofiling of a target polypeptide (e.g., PSA, AFP, MUC16, WFDC2, MUC1, ERBB2, CEACAM5, FUT3 or TG, preferably said target polypeptide is PSA); iv) for screening and/or analysing oligosaccharide chains (e.g., glycans) covalently attached to a target polypeptide (e.g., PSA, AFP, MUC16, WFDC2, MUC1, ERBB2, CEACAM5, FUT3 or TG, preferably said target polypeptide is PSA); v) for diagnostics of cancer (e.g., prostate cancer); vi) for positive and/or negative prediction of cancer (e.g., prostate cancer); vii) for determining a clinical stage of cancer (e.g., prostate cancer); viii) for distinguishing between prostate cancer and metastasizing prostate cancer; ix) for identifying prostate cancer likely to metastasize (e.g. likely to metastasize to the bone); xi) for distinguishing between benign prostatic hyperplasia (BPH) and prostate cancer; xii) for prevention and/or treatment of cancer (e.g., prostate cancer); xiii) for discriminating between significant and insignificant tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xiv) for discriminating between slow growing (e.g., clinically harmless) and fast growing (e.g., clinically relevant) tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xv) for identifying organ confined and/or potentially curable cancers (e.g., prostate cancer), e.g., by means of glycoprotein-based (e. g., target polypeptide-based, e.g., based on PSA, AFP, MUC16, WFDC2, MUC1, ERBB2, CEACAM5, FUT3 or TG, preferably said target polypeptide is PSA) diagnostics of cancer; xvi) for screening compounds; xvii) for use in a method of the present invention.

In some aspects the invention relates to, an anti-glycoprotein antibody (e.g., an anti-PSA, anti-AFP, anti-MUC16, anti-WFDC2, anti-MUC1, anti-ERBB2, anti-CEACAM5, anti-FUT3 or anti-TG antibody, preferably anti-PSA antibody), an antigen binding portion thereof, a magnetic carrier, one or more lectins or composition for one or more of the following: i) for selective capture and/or enrichment of a target polypeptide (e.g., PSA, AFP, MUC16, WFDC2, MUC1, ERBB2, CEACAM5, FUT3 or TG), preferably said target polypeptide is PSA; ii) for selective capture and/or enrichment of a target polypeptide (e.g., PSA, AFP, MUC16, WFDC2, MUC1, ERBB2, CEACAM5, FUT3 or TG, preferably said target polypeptide is PSA), wherein said selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate); iii) for glycoprofiling of a target polypeptide (e.g., PSA, AFP, MUC16, WFDC2, MUC1, ERBB2, CEACAM5, FUT3 or TG, preferably said target polypeptide is PSA); iv) for screening and/or analysing oligosaccharide chains (e.g., glycans) covalently attached to a target polypeptide (e.g., PSA, AFP, MUC16, WFDC2, MUC1, ERBB2, CEACAM5, FUT3 or TG, preferably said target polypeptide is PSA); v) for diagnostics of cancer (e.g., prostate cancer); vi) for positive and/or negative prediction of cancer (e.g., prostate cancer); vii) for determining a clinical stage of cancer (e.g., prostate cancer); viii) for distinguishing between prostate cancer and metastasizing prostate cancer; ix) for identifying prostate cancer likely to metastasize (e.g. likely to metastasize to the bone); x) for distinguishing between benign prostatic hyperplasia (BPH) and prostate cancer; xi) for prevention and/or treatment of cancer (e.g., prostate cancer); xii) for discriminating between significant and insignificant tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xiii) for discriminating between slow growing (e.g., clinically harmless) and fast growing (e.g., clinically relevant) tumours, e.g., by means of glycoprotein-based (e.g., target polypeptide-based, e.g., based on PSA, AFP, MUC16, WFDC2, MUC1, ERBB2, CEACAM5, FUT3 or TG, preferably said target polypeptide is PSA) diagnostics of cancer (e.g. prostate cancer); xiv) for identifying organ confined and/or potentially curable cancers (e.g., prostate cancer), e.g., by means of glycoprotein-based (e. g., target polypeptide-based, e.g., based on PSA, AFP, MUC16, WFDC2, MUC1, ERBB2, CEACAM5, FUT3 or TG, preferably said target polypeptide is PSA) diagnostics of cancer; xv) for screening compounds; xvi) for use in a method of the present invention.

In some aspects the invention relates to, a use of the present invention, wherein said use is an in vitro, ex vivo or in vivo use or combinations thereof.

In some aspects the invention relates to, an anti-glycoprotein antibody, antigen binding portion thereof, magnetic carrier, one or more lectins, kit, composition, use or method of the present invention, wherein said lectin is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 52, 53, 54, 55, 56, 57, 58, 59, lectins with following UniProtKB Accession Numbers: P0DKL3, P02866, P18891, O04366, AOA218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, 024313, Q9SM56, P06750, B9SPG3, Q9BZZ2, P20916, Q9NYZ4, Q96RL6, P05046, P93535, P02876, P10968, P10969, P22972 or P56625, lectins as described in Table 1 herein, wherein said lectin is capable to specifically reacting with glycosidic residue (e.g., terminal glycosidic residue) of other molecule (e.g. cell wall polysaccharide and/or glycoprotein (e.g., target polypeptide according any one of the preceding items or a biomarker selected from the group consisting of biomarkers as described in Table 1 herein).

In some aspects the invention relates to, an anti-glycoprotein antibody, antigen binding portion thereof, magnetic carrier, one or more lectins, kit, composition, use or method of the present invention, wherein said lectin is selected from the group consisting of: SEQ ID NO: 52, 53, 54, 55, 56, 57, 58, 59, lectins with following UniProtKB Accession Numbers: P0DKL3, P02866, P18891, O04366, A0A218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, 024313, Q9SM56, P06750, B9SPG3, Q9BZZ2, P20916, Q9NYZ4, Q96RL6, P05046, P93535, P02876, P10968, P10969, P22972 or P56625, lectins as described in Table 1 herein.

In some aspects the invention relates to, an anti-glycoprotein antibody, antigen binding portion thereof, magnetic carrier, one or more lectins, kit, composition, use or method of the present invention, wherein said lectin is mature.

In some aspects the invention relates to, an anti-glycoprotein antibody, antigen binding portion thereof, magnetic carrier, one or more lectins, kit, composition, use or method of the present invention, wherein said antibody is selected from the group consisting of: antibodies as described in Table 1 herein.

In some aspects the invention relates to, an anti-glycoprotein antibody, antigen binding portion thereof, magnetic carrier, one or more lectins, kit, composition, use or method of the present invention, wherein a biomarker is selected from the group consisting of biomarkers as described in Table 1 herein.

In some aspects the invention relates to, an anti-glycoprotein antibody, antigen binding portion thereof, magnetic carrier, one or more lectins, kit, composition, use or method of the present invention, wherein a cancer is selected from the group consisting of cancers as described in Table 1 herein.

In some aspects the invention relates to, an anti-glycoprotein antibody, antigen binding portion thereof, magnetic carrier, one or more lectins, kit or composition, use or method of the present invention, wherein said antibody, said biomarker, said cancer and said lectin are selected from the group consisting of corresponding antibodies, biomarkers, cancers and lectins as described in Table 1 herein.

In some aspects the invention relates to, an anti-glycoprotein antibody, antigen binding portion thereof, magnetic carrier, one or more lectins, kit, composition, use or method according to any one of preceding items, wherein a corresponding glycan modification (e.g., corresponding change (e.g., detectable change) of a glycan state and/or glycan composition and/or glycan concentration and/or glycan complexing (e.g., dimerization, trimerization, etc.) is selected from the group consisting of glycan modifications as described in Table 1 herein.

References as shown in Table 1 herein are as follows:
[1] C. Ohyama, M. Hosono, K. Nitta, M. Oh-eda, K. Yoshikawa, T. Habuchi, Y. Arai, M. Fukuda, Carbohydrate structure and differential binding of prostate specific antigen to Maackia amurensis lectin between prostate cancer and benign prostate hypertrophy, Glycobiology, 14 (2004) 671-679.
[2] K. Fukushima, T. Satoh, S. Baba, K. Yamashita, α1,2-Fucosylated and β-N-acetylgalactosaminylated prostate-specific antigen as an efficient marker of prostatic cancer, Glycobiology, 20 (2010) 452-460.
[3] T. Ishikawa, T. Yoneyama, Y. Tobisawa, S. Hatakeyama, T. Kurosawa, K. Nakamura, S. Narita, K. Mitsuzuka, W. Duivenvoorden, J.H. Pinthus, Y. Hashimoto, T. Koie, T. Habuchi, Y. Arai, C. Ohyama, An Automated Micro-Total Immunoassay System for Measuring Cancer-Associated 2,3-linked Sialyl N-Glycan-Carrying Prostate-Specific Antigen May Improve the Accuracy of Prostate Cancer Diagnosis, Int. J. Mol. Sci., 18 (2017) 15.
[4] D. Pihikova, P. Kasak, P. Kubanikova, R. Sokol, J. Tkac, Aberrant sialylation of a prostate-specific antigen: Electrochemical label-free glycoprofiling in prostate cancer serum samples, Anal. Chim. Acta, 934 (2016) 72-79.
[5] C. Ohyama, T. Koie, T. Yoneyama, Y. Tobisawa, Quantification of prostate cancer-associated aberrant glycosylation of prostate-specific antigen, Glycoscience: Biology and Medicine, Springer2015, pp. 1373-1377.
[6] E. Llop, M. Ferrer-Batalle, S. Barrabes, P.E. Guerrero, M. Ramirez, R. Saldova, P.M. Rudd, R.N. Aleixandre, J. Comet, R. de Llorens, R. Peracaula, Improvement of Prostate Cancer Diagnosis by Detecting PSA Glycosylation-Specific Changes, Theranostics, 6 (2016) 1190-1204.
[7] M. Ferrer-Batalle, E. Llop, M. Ramirez, R.N. Aleixandre, M. Saez, J. Comet, R. de Llorens, R. Peracaula, Comparative Study of Blood-Based Biomarkers, alpha 2,3-Sialic Acid PSA and PHI, for High-Risk Prostate Cancer Detection, International Journal of Molecular Sciences, 18 (2017) 12.
[8] T. Yoneyama, C. Ohyama, S. Hatakeyama, S. Narita, T. Habuchi, T. Koie, K. Mori, K.I. Hidari, M. Yamaguchi, T. Suzuki, Measurement of aberrant glycosylation of prostate specific antigen can improve specificity in early detection of prostate cancer, Biochem. Biophys. Res. Commun., 448 (2014) 390-396.
[9] N. Idil, I. Percin, V. Karakoc, H. Yavuz, N. Aksoz, A. Denizli, Concanavalin A immobilized magnetic poly(glycidyl methacrylate) beads for prostate specific antigen binding, Colloid Surf. B-Biointerfaces, 134 (2015) 461-468.
[10] M.V. Dwek, A. Jenks, A.J. Leathem, A sensitive assay to measure biomarker glycosylation demonstrates increased fucosylation of prostate specific antigen (PSA) in patients with prostate cancer compared with benign prostatic hyperplasia, Clin. Chim. Acta, 411 (2010) 1935-1939.
[11] K. Hagiwara, Y. Tobisawa, T. Kaya, T. Kaneko, S. Hatakeyama, K. Mori, Y. Hashimoto, T. Koie, Y. Suda, C. Ohyama, T. Yoneyama, Wisteria floribunda Agglutinin and Its Reactive-Glycan-Carrying Prostate-Specific Antigen as a Novel Diagnostic and Prognostic Marker of Prostate Cancer, Int. J. Mol. Sci., 18 (2017) 16.
[12] T. Kaya, T. Kaneko, S. Kojima, Y. Nakamura, Y. Ide, K. Ishida, Y. Suda, K. Yamashita, High-sensitivity immunoassay with surface plasmon field-enhanced fluorescence spectroscopy using a plastic sensor chip: Application to quantitative analysis of total prostate-specific antigen and GalNAcβ1-4GlcNAc-linked prostate-specific antigen for prostate cancer diagnosis, Anal. Chem., 87 (2015) 1797-1803.
[13] Q.K. Li, L. Chen, M.-H. Ao, J.H. Chiu, Z. Zhang, H. Zhang, D.W. Chan, Serum fucosylated prostate-specific antigen (PSA) improves the differentiation of aggressive from non-aggressive prostate cancers, Theranostics, 5 (2015) 267.
[14] K. Fujita, T. Hayashi, K. Matsuzaki, W. Nakata, M. Masuda, A. Kawashima, T. Ujike, A. Nagahara, M. Tsuchiya, Y. Kobayashi, S. Nojima, M. Uemura, E. Morii, E. Miyoshi, N. Nonomura, Decreased fucosylated PSA as a urinary marker for high Gleason score prostate cancer, Oncotarget, 7 (2016) 56643-56649.
[15] S. Takahashi, T. Sugiyama, M. Shimomura, Y. Kamada, K. Fujita, N. Nonomura, E. Miyoshi, M. Nakano, Site-specific and linkage analyses of fucosylated N-glycans on haptoglobin in sera of patients with various types of cancer: possible implication for the differential diagnosis of cancer, Glycoconjugate J., 33 (2016) 471-482.
[16] S. Kazuno, T. Fujimura, T. Arai, T. Ueno, K. Nagao, M. Fujime, K. Murayama, Multi-sequential surface plasmon resonance analysis of haptoglobin-lectin complex in sera of patients with malignant and benign prostate diseases, Anal. Biochem., 419 (2011) 241-249.
[17] S.-J. Yoon, S.-Y. Park, P.-C. Pang, J. Gallagher, J.E. Gottesman, A. Dell, J.-H. Kim, S.-I. Hakomori, N-glycosylation status of β-haptoglobin in sera of patients with prostate cancer vs. benign prostate diseases, Int. J. Oncol., 36 (2010) 193-203.
[18] T. Fujimura, Y. Shinohara, B. Tissot, P.C. Pang, M. Kurogochi, S. Saito, Y. Arai, M. Sadilek, K. Murayama, A. Dell, Glycosylation status of haptoglobin in sera of patients with prostate cancer vs. benign prostate disease or normal subjects, Int. J. Cancer, 122 (2008) 39-49.
[19] K. Fujita, M. Shimomura, M. Uemura, W. Nakata, M. Sato, A. Nagahara, Y. Nakai, S. Takamatsu, E. Miyoshi, N. Nonomura, Serum fucosylated haptoglobin as a novel prognostic biomarker predicting high□Gleason prostate cancer, The Prostate, 74 (2014) 1052-1058.
[20] S. Weiz, M. Wieczorek, C. Schwedler, M. Kaup, E.I. Braicu, J. Sehouli, R. Tauber, V. Blanchard, Acute□phase glycoprotein N□glycome of ovarian cancer patients analyzed by CE□LIF, Electrophoresis, 37 (2016) 1461-1467.
[21] R. Saldova, L. Royle, C.M. Radcliffe, U.M. Abd Hamid, R. Evans, J.N. Arnold, R.E. Banks, R. Hutson, D.J. Harvey, R. Antrobus, Ovarian cancer is associated with changes in glycosylation in both acute-phase proteins and IgG, Glycobiology, 17 (2007) 1344-1356.
[22] G. Turner, M. Goodarzi, S. Thompson, Glycosylation of alpha-1-proteinase inhibitor and haptoglobin in ovarian cancer: evidence for two different mechanisms, Glycoconjugate J., 12 (1995) 211-218.
[23] K. Chen, A. Gentry-Maharaj, M. Burnell, C. Steentoft, L. Marcos-Silva, U. Mandel, I. Jacobs, A. Dawnay, U. Menon, O. Blixt, Microarray Glycoprofiling of CA125 improves differential diagnosis of ovarian cancer, J. Proteome Res., 12 (2013) 1408-1418.
[24] R. Saldova, W.B. Struwe, K. Wynne, G. Elia, M.J. Duffy, P.M. Rudd, Exploring the glycosylation of serum CA125, International journal of molecular sciences, 14 (2013) 15636-15654.
[25] H. Zhuang, J. Gao, Z. Hu, J. Liu, D. Liu, B. Lin, Co-expression of Lewis y antigen with human epididymis protein 4 in ovarian epithelial carcinoma, PLoS One, 8 (2013) e68994.
[26] J. Wu, X. Xie, S. Nie, R.J. Buckanovich, D.M. Lubman, Altered expression of sialylated glycoproteins in ovarian cancer sera using lectin-based ELISA assay and quantitative glycoproteomics analysis, J. Proteome Res., 12 (2013) 3342-3352.
[27] H. Ideo, Y. Hinoda, K. Sakai, I. Hoshi, S. Yamamoto, M. Oka, K. Maeda, N. Maeda, S. Hazama, J. Amano, Expression of mucin 1 possessing a 3'□sulfated core1 in recurrent and metastatic breast cancer, Int. J. Cancer, 137 (2015) 1652-1660.
[28] S.A. Svarovsky, L. Joshi, Cancer glycan biomarkers and their detection-past, present and future, Anal. Methods, 6 (2014) 3918-3936.
[29] S.J. Storr, L. Royle, C.J. Chapman, U.M.A. Hamid, J.F. Robertson, A. Murray, R.A. Dwek, P.M. Rudd, The O-linked glycosylation of secretory/shed MUC1 from an advanced breast cancer patient's serum, Glycobiology, 18 (2008) 456-462.
[30] H.A. Badr, D.M. AlSadek, A.A. Darwish, A.I. ElSayed, B.O. Bekmanov, E.M. Khussainova, X. Zhang, W.C. Cho, L.B. Djansugurova, C.-Z. Li, Lectin approaches for glycoproteomics in FDA-approved cancer biomarkers, Expert review of proteomics, 11 (2014) 227-236.
[31] Y. Taeda, M. Nose, S. Hiraizumi, N. Ohuchi, Expression of L-PHA-binding proteins in breast cancer: Reconstitution and molecular characterization of beta 1-6 branched oligosaccharides in three-dimensional cell culture, Breast Cancer Res. Treat., 38 (1996) 313-324.
[32] S.Y. Park, S.J. Yoon, Y.T. Jeong, J.M. Kim, J.Y. Kim, B. Bernert, T. Ullman, S.H. Itzkowitz, J.H. Kim, S.i. Hakomori, N□glycosylation status of β□haptoglobin in sera of patients with colon cancer, chronic inflammatory diseases and normal subjects, Int. J. Cancer, 126 (2010) 142-155.
[33] Y. Takeda, S. Shinzaki, K. Okudo, K. Moriwaki, K. Murata, E. Miyoshi, Fucosylated haptoglobin is a novel type of cancer biomarker linked to the prognosis after an operation in colorectal cancer, Cancer, 118 (2012) 3036-3043.
[34] S.Y. Park, S.H. Lee, N. Kawasaki, S. Itoh, K. Kang, S. Hee Ryu, N. Hashii, J.M. Kim, J.Y. Kim, J. Hoe Kim, α1□3/4 fucosylation at Asn 241 of β□haptoglobin is a novel marker for colon cancer: A combinatorial approach for development of glycan biomarkers, Int. J. Cancer, 130 (2012) 2366-2376.
[35] S.-Y. Park, S.-J. Yoon, S.-I. Hakomori, J.-M. Kim, J.-Y. Kim, B. Bernert, T. Ullman, S.H. Itzkowitz, J.H. Kim, Dimeric Lea (Lea-on-Lea) status of β-haptoglobin in sera of colon cancer, chronic inflammatory disease and normal subjects, Int. J. Oncol., 36 (2010) 1291-1297.
[36] R.S. Bresalier, J.C. Byrd, D. Tessler, J. Lebel, J. Koomen, D. Hawke, E. Half, K.F. Liu, N. Mazurek, C. Great Lakes-New England, A circulating ligand for galectin-3 glycoprotein elevated in individual is a haptoglobin-related with colon cancer, Gastroenterology, 127 (2004) 741-748.
[37] E. Saeland, A.I. Belo, S. Mongera, I. van Die, G.A. Meijer, Y. van Kooyk, Differential glycosylation of MUC1 and CEACAM5 between normal mucosa and tumour tissue of colon cancer patients, Int. J. Cancer, 131 (2012) 117-128.
[38] Y. Qiu, T.H. Patwa, L. Xu, K. Shedden, D.E. Misek, M. Tuck, G. Jin, M.T. Ruffin, D.K. Turgeon, S. Synal, Plasma glycoprotein profiling for colorectal cancer biomarker identification by lectin glycoarray and lectin blot, J. Proteome Res., 7 (2008) 1693-1703.
[39] C. Li, D.M. Simeone, D.E. Brenner, M.A. Anderson, K.A. Shedden, M.T. Ruffin, D.M. Lubman, Pancreatic cancer serum detection using a lectin/glyco-antibody array method, J. Proteome Res., 8 (2008) 483-492.
[40] J. Zhao, T.H. Patwa, W. Qiu, K. Shedden, R. Hinderer, D.E. Misek, M.A. Anderson, D.M. Simeone, D.M. Lubman, Glycoprotein microarrays with multi-lectin detection: unique lectin binding patterns as a tool for classifying normal, chronic pancreatitis and pancreatic cancer sera, J. Proteome Res., 6 (2007) 1864-1874.
[41] E. Miyoshi, M. Nakano, Fucosylated haptoglobin is a novel marker for pancreatic cancer: detailed analyses of oligosaccharide structures, Proteomics, 8 (2008) 3257-3262.
[42] S. Nie, A. Lo, J. Wu, J. Zhu, Z. Tan, D.M. Simeone, M.A. Anderson, K.A. Shedden, M.T. Ruffin, D.M. Lubman, Glycoprotein biomarker panel for pancreatic cancer discovered by quantitative proteomics analysis, J. Proteome Res., 13 (2014) 1873-1884.
[43] K. Kusama, Y. Okamoto, K. Saito, T. Kasahara, T. Murata, Y. Ueno, Y. Kobayashi, Y. Kamada, E. Miyoshi, Reevaluation of Pholiota squarrosa lectin-reactive haptoglobin as a pancreatic cancer biomarker using an improved ELISA system, Glycoconjugate J., (2017) 1-8.
[44] N. Parker, C. Makin, C. Ching, D. Eccleston, O. Taylor, D. Milton, J.M. Rhodes, A new enzyme□linked lectin/mucin antibody sandwich assay (CAM 17.1/WGA) assessed in combination with CA 1909 and peanut lectin binding assay for the diagnosis of pancreatic cancer, Cancer, 70 (1992) 1062-1068.
[45] J.Y. Yiannakou, P. Newland, F. Calder, A.N. Kingsnorth, J.M. Rhodes, Prospective study of CAM 17 center dot 1/WGA mucin assay for serological diagnosis of pancreatic cancer, Lancet, 349 (1997) 389-392.
[46] T. Yue, I.J. Goldstein, M.A. Hollingsworth, K. Kaul, R.E. Brand, B.B. Haab, The prevalence and nature of glycan alterations on specific proteins in pancreatic cancer patients revealed using antibody-lectin sandwich arrays, Mol. Cel. Proteom., 8 (2009) 1697-1707.
[47] S. Pan, T.A. Brentnall, R. Chen, Glycoproteins and glycoproteomics in pancreatic cancer, World journal of gastroenterology, 22 (2016) 9288.
[48] K. Shimizu, K. Nakamura, S. Kobatake, S. Satomura, M. Maruyama, F. Kameko, J. Tajiri, R. Kato, The clinical utility of Lens culinaris agglutinin-reactive thyroglobulin ratio in serum for distinguishing benign from malignant conditions of the thyroid, Clin. Chim. Acta, 379 (2007) 101-104.
[49] T. Kanai, M. Amakawa, R. Kato, K. Shimizu, K. Nakamura, K.-i. Ito, Y. Hama, M. Fujimori, J. Amano, Evaluation of a new method for the diagnosis of alterations of Lens culinaris agglutinin binding of thyroglobulin molecules in thyroid carcinoma, Clinical chemistry and laboratory medicine, 47 (2009) 1285-1290.
[50] K. YAMAMOTO, T. TSUJI, O. TARUTANI, T. OSAWA, Structural changes of carbohydrate chains of human thyroglobulin accompanying malignant transformations of thyroid glands, The FEBS Journal, 143 (1984) 133-144.
[51] A. Naitoh, Y. Aoyagi, H. Asakura, Highly enhanced fucosylation of serum glycoproteins in patients with hepatocellular carcinoma, Journal of gastroenterology and hepatology, 14 (1999) 436-445.
[52] Y.H. Ahn, P.M. Shin, N.R. Oh, G.W. Park, H. Kim, J.S. Yoo, A lectin-coupled, targeted proteomic mass spectrometry (MRM MS) platform for identification of multiple liver cancer biomarkers in human plasma, J. Proteomics, 75 (2012) 5507-5515.
[53] Y.H. Ahn, P.M. Shin, Y.S. Kim, N.R. Oh, E.S. Ji, K.H. Kim, Y.J. Lee, S.H. Kim, J.S. Yoo, Quantitative analysis of aberrant protein glycosylation in liver cancer plasma by AAL-enrichment and MRM mass spectrometry, Analyst, 138 (2013) 6454-6462.
[54] J.H. Lee, C.H. Cho, S.H. Kim, J.G. Kang, J.S. Yoo, C.L. Chang, J.-H. Ko, Y.-S. Kim, Semiquantitative measurement of a specific glycoform using a DNA-tagged antibody and lectin affinity chromatography for glyco-biomarker development, Mol. Cel. Proteom., 14 (2015) 782-795.
[55] H. Toyoda, T. Kumada, T. Tada, Y. Kaneoka, A. Maeda, F. Kanke, S. Satomura, Clinical utility of highly sensitive Lens culinaris agglutinin□reactive alpha□fetoprotein in hepatocellular carcinoma patients with alpha□fetoprotein< 20 ng/mL, Cancer Sci., 102 (2011) 1025-1031.
[56] X. Yi, S. Yu, Y. Bao, Alpha-fetoprotein-L3 in hepatocellular carcinoma: a meta-analysis, Clin. Chim. Acta, 425 (2013) 212-220.
[57] M.A. Comunale, M. Wang, J. Hafner, J. Krakover, L. Rodemich, B. Kopenhaver, R.E. Long, O. Junaidi, A.M.D. Bisceglie, T.M. Block, Identification and development of fucosylated glycoproteins as biomarkers of primary hepatocellular carcinoma, J. Proteome Res., 8 (2008) 595-602.
[58] Y. Liu, J. He, C. Li, R. Benitez, S. Fu, J. Marrero, D.M. Lubman, Identification and Confirmation of Biomarkers Using an Integrated Platform for Quantitative Analysis of Glycoproteins and Their Glycosylations, J. Proteome Res., 9 (2010) 798-805.
[59] L.F. Hoagland, M.J. Campa, E.B. Gottlin, J.E. Herndon, E.F. Patz, Haptoglobin and posttranslational glycan□modified derivatives as serum biomarkers for the diagnosis of nonsmall cell lung cancer, Cancer, 110 (2007) 2260-2268.
[60] D. Wang, M. Hincapie, T. Rejtar, B.L. Karger, Ultrasensitive characterization of site-specific glycosylation of affinity-purified haptoglobin from lung cancer patient plasma using 10 µm id porous layer open tubular liquid chromatography- linear ion trap collision-induced dissociation/electron transfer dissociation mass spectrometry, Anal. Chem., 83 (2011) 2029-2037.
[61] H.Y. Tsai, K. Boonyapranai, S. Sriyam, C.J. Yu, S.W. Wu, K.H. Khoo, S. Phutrakul, S.T. Chen, Glycoproteomics analysis to identify a glycoform on haptoglobin associated with lung cancer, Proteomics, 11 (2011) 2162-2170.
[62] J.N. Arnold, R. Saldova, M.C. Galligan, T.B. Murphy, Y. Mimura-Kimura, J.E. Telford, A.K. Godwin, P.M. Rudd, Novel Glycan Biomarkers for the Detection of Lung Cancer, J. Proteome Res., 10 (2011) 1755-1764.
[63] Y. Hirao, H. Matsuzaki, J. Iwaki, A. Kuno, H. Kaji, T. Ohkura, A. Togayachi, M. Abe, M. Nomura, M. Noguchi, Glycoproteomics approach for identifying glycobiomarker candidate molecules for tissue type classification of non-small cell lung carcinoma, J. Proteome Res., 13 (2014) 4705-4716.
[64] M. Ferens-Sieczkowska, E.M. Kratz, B. Kossowska, E. Passowicz-Muszynska, R. Jankowska, Comparison of Haptoglobin and Alpha(1)-Acid Glycoprotein Glycosylation in the Sera of Small Cell and Non-Small Cell Lung Cancer Patients, Postep. Hig. Med. Dosw., 67 (2013) 828-836.
[65] Y.Q. Liang, T.R. Ma, A. Thakur, H.J. Yu, L. Gao, P.Y. Shi, X.T. Li, H. Ren, L.Y. Jia, S. Zhang, Z. Li, M.W. Chen, Differentially expressed glycosylated patterns of alpha-1-antitrypsin as serum biomarkers for the diagnosis of lung cancer, Glycobiology, 25 (2015) 331-340.
[66] S.D. Shiayan, V.V. Nasonov, N.V. Bovin, L.I. Novikova, V.A. Aleshkin, A.G. Lutov, STUDIES OF N-LINKED OLIGOSACCHARIDE CHAINS OF ALPHA(1)-ACID GLYCOPROTEIN ISOLATED FROM ASCITIC FLUID OF STOMACH-CANCER PATIENTS AND NORMAL SERUM, Eksperimentalnaya Onkologiya, 15 (1993) 53-61.
[67] J. Bones, J.C. Byrne, N. O'Donoghue, C. McManus, C. Scaife, H. Boissin, A. Nastase, P.M. Rudd, Glycomic and glycoproteomic analysis of serum from patients with stomach cancer reveals potential markers arising from host defense response mechanisms, J. Proteome Res., 10 (2010) 1246-1265.
[68] S.H. Lee, S. Jeong, J. Lee, I.S. Yeo, M.J. Oh, U. Kim, S. Kim, S.H. Kim, S.Y. Park, J.H. Kim, S.H. Park, J.H. Kim, H.J. An, Glycomic profiling of targeted serum haptoglobin for gastric cancer using nano LC/MS and LC/MS/MS, Mol. Biosyst., 12 (2016) 3611-3621.
[69] L. Valmu, H. Alfthan, K. Hotakainen, S. Birken, U.H. Stenman, Site-specific glycan analysis of human chorionic gonadotropin beta-subunit from malignancies and pregnancy by liquid chromatography-electrospray mass spectrometry, Glycobiology, 16 (2006) 1207-1218.
[70] T. Kamoto, S. Satomura, T. Yoshiki, Y. Okada, F. Henmi, H. Nishiyama, T. Kobayashi, A. Terai, T. Habuchi, O. Ogawa, Lectin-reactive alpha-fetoprotein (AFP-L3%) curability and prediction of clinical course after treatment of non-seminomatous germ cell tumors, Jpn. J. Clin. Oncol., 32 (2002) 472-476.
[71] S. Ambrose, N. Gordon, J. Goldsmith, W. Wei, M. Zeegers, N. James, M. Knowles, R. Bryan, D. Ward, Use of Aleuria alantia Lectin Affinity Chromatography to Enrich Candidate Biomarkers from the Urine of Patients with Bladder Cancer, Proteomes, 3 (2015) 266.
[72] R. Azevedo, A. Peixoto, C. Gaiteiro, E. Fernandes, M. Neves, L. Lima, L.L. Santos, J.A. Ferreira, Over forty years of bladder cancer glycobiology: Where do glycans stand facing precision oncology?, Oncotarget, 8 (2017) 91734-91764.
[73] E. Pocheć, A. Lityńska, M. Bubka, A. Amoresano, A. Casbarra, Characterization of the oligosaccharide component of α 3 β 1 integrin from human bladder carcinoma cell line T24 and its role in adhesion and migration, European journal of cell biology, 85 (2006) 47-57.
[74] S. Cotton, R. Azevedo, C. Gaiteiro, D. Ferreira, L. Lima, A. Peixoto, E. Fernandes, M. Neves, D. Neves, T. Amaro, R. Cruz, A. Tavares, M. Rangel, A.M.N. Silva, L.L. Santos, J.A. Ferreira, Targeted O-glycoproteomics explored increased sialylation and identified MUC16 as a poor prognosis biomarker in advanced-stage bladder tumours, Mol. Oncol., 11 (2017) 895-912.
[75] N. Yang, S. Feng, K. Shedden, X.L. Xie, Y.S. Liu, C.J. Rosser, D.M. Lubman, S. Goodison, Urinary Glycoprotein Biomarker Discovery for Bladder Cancer Detection Using LC/MS-MS and Label-Free Quantification, Clin. Cancer Res., 17 (2011) 3349-3359.
[76] Yamashita K, Umetsu K, Suzuki T, Ohkura T (1992) Purification and characterization of a Neu5Ac alpha 2-->6Gal beta 1-->4GlcNAc and HSO3(-)-->6Gal beta 1-->GlcNAc specific lectin in tuberous roots of Trichosanthes japonica. Biochemistry 31 (46):11647-11650.
[77] Debray H, Montreuil J (1989) Aleuria aurantia agglutinin. A new isolation procedure and further study of its specificity towards various glycopeptides and oligosaccharides. Carbohydrate Research 185 (1):15-26.
[78] Kochibe N, Furukawa K (1980) Purification and properties of a novel fucose-specific hemagglutinin of Aleuria aurantia. Biochemistry 19 (13):2841-2846.

**The invention is also characterized by the following items:**
1. An anti-glycoprotein antibody (e.g., monoclonal antibody, chimeric antibody, humanized antibody, human antibody, e.g., an anti-PSA, anti-AFP, anti-MUC16, anti-WFDC2, anti-MUC1, anti-ERBB2, anti-CEACAM5, anti-FUT3 or anti-TG antibody, preferably anti-PSA antibody) or an antigen binding portion thereof (e.g., a single chain antibody fragment (scAb), scFv, Fab, diabody, a DART, domain antibody, or nanobody) immobilized (e.g., conjugated) on a magnetic carrier (e.g., a magnetic particle, magnetic bead, metallic nano- or microparticle, metal oxide nano- or microparticle), wherein said magnetic carrier further comprises a polypeptide having peroxidase activity (e.g., polypeptide having EC 1.11.1.7 enzymatic activity, e.g., microperoxidase-11), preferably said antibody is selected from the group consisting of antibodies as described in Table 1 herein.
2. The anti-glycoprotein antibody or the antigen binding portion thereof according to any one of preceding items, wherein said polypeptide having peroxidase activity has a molecular weight of less than 2 kDa (e.g., about 1.5 or about 1.6 or about 1.9 kDa), preferably a molecular weight between 1 and 2 kDa.
3. The anti-glycoprotein antibody or the antigen binding portion thereof according to any one of preceding items, wherein said polypeptide having peroxidase activity is immobilized (e.g., conjugated) on said magnetic carrier.
4. The anti-glycoprotein antibody or the antigen binding portion thereof according to any one of preceding items, wherein said anti-glycoprotein antibody or the antigen binding portion thereof specifically binds to a target polypeptide (e.g., biomarker) comprising a polypeptide selected from the group consisting of (e.g., target glycoprotein comprises said polypeptide, e.g., anti-glycoprotein antibody is raised against said polypeptide, e.g., antigen glycoprotein comprises said polypeptide):
   i) Prostate-specific antigen (PSA), preferably SEQ ID NOs: 1, 2, 3, 4, 5 or 6; further preferably SEQ ID NO: 6;
   ii) α-fetoprotein (AFP), preferably SEQ ID NOs: 11 or 12; further preferably SEQ ID NO: 12;
   iii) Mucin-16 (MUC16), preferably SEQ ID NO: 13;
   iv) WAP four-disulfide core domain protein 2 (WFDC2), preferably SEQ ID NOs: 14, 15, 16, 17, 18 or 19; further preferably SEQ ID NO: 19;
   v) Mucin-1 (MUC1), preferably SEQ ID NOs: 20, 21, 22, 23, 24, 25, 26, 27, 28, 29, 30, 31, 32, 33, 34, 35, 36 or 37; further preferably SEQ ID NO: 37;
   vi) Receptor tyrosine-protein kinase erbB-2 (ERBB2), preferably SEQ ID NOs: 38, 39, 40, 41, 42, 43, or 44; further preferably SEQ ID NO: 44;
   vii) Carcinoembryonic antigen-related cell adhesion molecule 5 (CEACAM5), preferably SEQ ID NOs: 45, 46, or 47; further preferably SEQ ID NO: 47;
   viii) Galactoside 3(4)-L-fucosyltransferase (FUT3), preferably SEQ ID NO: 48;
   ix) Thyroglobulin (TG), preferably SEQ ID NOs: 49, 50, or 51; further preferably SEQ ID NO: 51
   x) any biomarker as described in Table 1 herein.
5. The anti-glycoprotein antibody or the antigen binding portion thereof according to any one of preceding items, wherein said target polypeptide is a prostate-specific antigen (PSA) having peptidase activity (e.g., EC 3.4.21.77 enzymatic activity).
6. The anti-glycoprotein antibody or the antigen binding portion thereof according to any one of preceding items, wherein said anti-glycoprotein antibody or the antigen binding portion thereof specifically binds to a target polypeptide (e.g., PSA) comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 6; preferably said polypeptide has SEQ ID NO: 6; ii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 1; preferably said polypeptide has SEQ ID NO: 1; iii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 2; preferably said polypeptide has SEQ ID NO: 2; iv) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 3; preferably said polypeptide has SEQ ID NO: 3; v) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 4; preferably said polypeptide has SEQ ID NO: 4; vi) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 5; preferably said polypeptide has SEQ ID NO: 5.
7. The anti-glycoprotein antibody or the antigen binding portion thereof according to any one of preceding items, wherein said target polypeptide is human, rabbit, rat or mouse, preferably said target polypeptide is human.
8. The antibody or the antigen binding portion thereof according to any one of preceding items, wherein said polypeptide having peroxidase activity comprises a microperoxidase (MP) (e.g., microperoxidase-11) and/or horseradish peroxidase (HRP, e.g., Peroxidase C1A having UniProtKB Accession Number: P00433).
9. The antibody or the antigen binding portion thereof according to any one of preceding items, wherein said microperoxidase (MP) is a heme containing peptide portion of cytochrome c (e.g., shown as SEQ ID NO: 10, cytochrome c derived from *Equus caballus,* NCBI Reference Sequence: NP_001157486.1) that retains peroxidase activity (e.g., EC 1.11.1.7 enzymatic activity, e.g., microperoxidase-11).
10. The antibody or the antigen binding portion thereof according to any one of preceding items, wherein said heme containing peptide portion of cytochrome c is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 7 (MP-11 peptide), SEQ ID NO: 8 (MP-9 peptide) and SEQ ID NO: 9 (MP-8 peptide), preferably said microperoxidase (MP) peptide is selected from the group consisting of: SEQ ID NO: 7 (MP-11 peptide), SEQ ID NO: 8 (MP-9 peptide) and SEQ ID NO: 9 (MP-8 peptide).
11. The antibody or the antigen binding portion thereof according to any one of preceding items, wherein said magnetic carrier is a magnetic particle, magnetic bead, metallic nano- or microparticle, metal oxide nano- or microparticle .
12. The anti-glycoprotein antibody or the antigen binding portion thereof according to any one of preceding items, wherein said anti-glycoprotein antibody or the antigen binding portion thereof is capable of simultaneous binding to said target polypeptide (e.g., PSA, AFP, MUC16, WFDC2, MUC1, ERBB2, CEACAM5, FUT3, TG, or a biomarker selected from the group consisting of biomarkers as described in Table 1 herein) in a sample and generating a detection signal (e.g., by the optical means).
13. The anti-glycoprotein antibody or the antigen binding portion thereof according to any one of preceding items, wherein said anti-glycoprotein antibody or the antigen binding portion thereof is capable of simultaneous binding and enriching said target polypeptide (e.g., PSA, AFP, MUC16, WFDC2, MUC1, ERBB2, CEACAM5, FUT3, TG or a biomarker selected from the group consisting of biomarkers as described in Table 1 herein) in a sample and generating a detection signal (e.g., by the optical means).
14. The anti-glycoprotein or the antigen binding portion thereof according to any one of preceding items, wherein said anti-glycoprotein or the antigen binding portion thereof is capable of detecting said target polypeptide (e.g., PSA, AFP, MUC16, WFDC2, MUC1, ERBB2, CEACAM5, FUT3, TG or a biomarker selected from the group consisting of biomarkers as described in Table 1 herein) in a sample (e.g., serum sample) having said target polypeptide in an amount corresponding to 0.04 mL or less of undiluted biological sample (e.g., undiluted serum sample), preferably in the range between 0.01-0.04 mL, further preferably in the range between 0.02-0.04 mL, most preferably in the range between 0.02-0.04 mL).
15. The anti-glycoprotein or the antigen binding portion thereof according to any one of preceding items, wherein said anti-glycoprotein antibody is a monoclonal antibody.
16. The anti-glycoprotein or the antigen binding portion thereof according to any one of preceding items, wherein said anti-glycoprotein antibody is selected from the group consisting of: chimeric, humanized or human antibody.
17. A magnetic carrier (e.g., a magnetic particle, magnetic bead, metallic nano- or microparticle, metal oxide nano- or microparticle ) comprising: i) an immobilized (e.g., conjugated) anti-glycoprotein antibody (e.g., monoclonal antibody, chimeric antibody, humanized antibody, human antibody, e.g., an anti-PSA, anti-AFP, anti-MUC16, anti-WFDC2, anti-MUC1, anti-ERBB2, anti-CEACAM5, anti-FUT3, anti-TG antibody, or an antibody selected from the group consisting of antibodies as described in Table 1 herein) or an antigen binding portion thereof (e.g., a single chain antibody fragment (scAb), scFv, Fab, diabody, a DART, domain antibody, or nanobody) and ii) a polypeptide having peroxidase activity (e.g., EC 1.11.1.7 enzymatic activity, e.g., microperoxidase-11).
18. The magnetic carrier according to any one of preceding items, wherein said polypeptide having peroxidase activity has a molecular weight of less than 2 kDa (e.g., about 1.5 or about 1.6 or about 1.9 kDa), preferably a molecular weight between 1 and 2 kDa.
19. The magnetic carrier according to any one of preceding items, wherein said polypeptide having peroxidase activity is immobilized (e.g., conjugated) on said magnetic carrier.
20. The magnetic carrier according to any one of preceding items, wherein said anti-glycoprotein antibody or the antigen binding portion thereof specifically binds to a target polypeptide comprising a polypeptide selected from the group consisting of (e.g., target glycoprotein comprises said polypeptide, e.g., anti-glycoprotein antibody is raised against said polypeptide, e.g., antigen glycoprotein comprises said polypeptide):
   i) Prostate-specific antigen (PSA), preferably SEQ ID NOs: 1, 2, 3, 4, 5 or 6; further preferably SEQ ID NO: 6;
   ii) α-fetoprotein (AFP), preferably SEQ ID NOs: 11 or 12; further preferably SEQ ID NO: 12;
   iii) Mucin-16 (MUC16), preferably SEQ ID NO: 13;
   iv) WAP four-disulfide core domain protein 2 (WFDC2), preferably SEQ ID NOs: 14, 15, 16, 17, 18 or 19; further preferably SEQ ID NO: 19;
   v) Mucin-1 (MUC1), preferably SEQ ID NOs: 20, 21, 22, 23, 24, 25, 26, 27, 28, 29, 30, 31, 32, 33, 34, 35, 36 or 37; further preferably SEQ ID NO: 37;
   vi) Receptor tyrosine-protein kinase erbB-2 (ERBB2), preferably SEQ ID NOs: 38, 39, 40, 41, 42, 43, or 44; further preferably SEQ ID NO: 44;
   vii) Carcinoembryonic antigen-related cell adhesion molecule 5 (CEACAM5), preferably SEQ ID NOs: 45, 46, or 47; further preferably SEQ ID NO: 47;
   viii) Galactoside 3(4)-L-fucosyltransferase (FUT3), preferably SEQ ID NO: 48;
   ix) Thyroglobulin (TG), preferably SEQ ID NOs: 49, 50, or 51; further preferably SEQ ID NO: 51
   x) any biomarker as described in Table 1 herein.
21. The magnetic carrier according to any one of preceding items, said target polypeptide is a prostate-specific antigen (PSA) having peptidase activity (e.g., EC 3.4.21.77 enzymatic activity).
22. The magnetic carrier according to any one of preceding items, wherein said anti-glycoprotein antibody or the antigen binding portion thereof specifically binds to a target polypeptide (e.g., PSA) comprising a polypeptide selected from the group consisting of (e.g., target glycoprotein comprises said polypeptide, e.g., anti-glycoprotein antibody is raised against said polypeptide, e.g., antigen glycoprotein comprises said polypeptide): i) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 6; preferably said polypeptide has SEQ ID NO: 6; ii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 1; preferably said polypeptide has SEQ ID NO: 1; iii) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 2; preferably said polypeptide has SEQ ID NO: 2; iv) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 3; preferably said polypeptide has SEQ ID NO: 3; v) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 4; preferably said polypeptide has SEQ ID NO: 4; vi) a polypeptide which is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence of SEQ ID NO: 5; preferably said polypeptide has SEQ ID NO: 5.
23. The magnetic carrier according to any one of preceding items, wherein said target polypeptide is human, rabbit, rat or mouse, preferably said glycoprotein is human.
24. The magnetic carrier according to any one of preceding items, wherein said polypeptide having peroxidase activity comprises a microperoxidase (MP) (e.g., microperoxidase-11) and/or horseradish peroxidase (HRP, e.g., Peroxidase C1A having UniProtKB Accession Number: P00433).
25. The magnetic carrier according to any one of preceding items, wherein said microperoxidase (MP) is a heme containing peptide portion of cytochrome c (e.g., shown as SEQ ID NO: 10, cytochrome c derived from *Equus caballus,* NCBI Reference Sequence: NP_001157486.1) that retains peroxidase activity (e.g., EC 1.11.1.7 enzymatic activity, e.g., microperoxidase-11).
26. The magnetic carrier according to any one of preceding items, said heme containing peptide portion of cytochrome c is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 7 (MP-11 peptide), SEQ ID NO: 8 (MP-9 peptide) and SEQ ID NO: 9 (MP-8 peptide), preferably said microperoxidase (MP) peptide is selected from the group consisting of: SEQ ID NO: 7 (MP-11 peptide), SEQ ID NO: 8 (MP-9 peptide) and SEQ ID NO: 9 (MP-8 peptide).
27. The magnetic carrier according to any one of preceding items, wherein said magnetic carrier is a magnetic particle, magnetic bead, metallic nano- or microparticle, metal oxide nano- or microparticle .
28. The magnetic carrier according to any one of preceding items, said magnetic carrier is capable of simultaneous binding to said target polypeptide (e.g., PSA, AFP, MUC16, WFDC2, MUC1, ERBB2, CEACAM5, FUT3, TG or a biomarker selected from the group consisting of biomarkers as described in Table 1 herein) in a sample and generating a detection signal (e.g., by the optical means).
29. The magnetic carrier according to any one of preceding items, said magnetic carrier is capable of simultaneous binding and enriching said target polypeptide (e.g., PSA, AFP, MUC16, WFDC2, MUC1, ERBB2, CEACAM5, FUT3, TG or a biomarker selected from the group consisting of biomarkers as described in Table 1 herein) in a sample and generating a detection signal (e.g., by the optical means).
30. The magnetic carrier according to any one of preceding items, wherein said magnetic carrier is capable of detecting said target polypeptide (e.g., PSA, AFP, MUC16, WFDC2, MUC1, ERBB2, CEACAM5, FUT3, TG or a biomarker selected from the group consisting of biomarkers as described in Table 1 herein) in a sample (e.g., serum sample) having said target polypeptide in an amount corresponding to 0.04 mL or less of undiluted biological sample (e.g., undiluted serum sample), preferably in the range between 0.01-0.04 mL, further preferably in the range between 0.02-0.04 mL, most preferably in the range between 0.02-0.04 mL).
31. The magnetic carrier according to any one of preceding items, wherein said anti-glycoprotein antibody is a monoclonal antibody.
32. The magnetic carrier according to any one of preceding items, wherein said anti-glycoprotein antibody is selected from the group consisting of: chimeric, humanized or human antibody.
33. A method for producing an anti-glycoprotein antibody (e.g., an anti-glycoprotein antibody according any one of preceding items, e.g., an anti-PSA, anti-AFP, anti-MUC16, anti-WFDC2, anti-MUC1, anti-ERBB2, anti-CEACAM5, anti-FUT3, anti-TG antibody, or an antibody selected from the group of antibodies as described in Table 1 herein) or an antigen binding portion thereof (e.g., an antigen binding portion according to any one of preceding items), said method comprising simultaneously conjugating a magnetic carrier (e.g., a magnetic particle, magnetic bead, metallic nano- or microparticle, metal oxide nano- or microparticle) to:
   i) an anti-glycoprotein antibody (e.g., an anti-PSA, anti-AFP, anti-MUC16, anti-WFDC2, anti-MUC1, anti-ERBB2, anti-CEACAM5, anti-FUT3, anti-TG antibody or an antibody selected from the group of antibodies as described in Table 1 herein) or an antigen binding portion thereof according to any one of preceding items,
   ii) a polypeptide having peroxidase activity according to any one of preceding items.
34. A method for producing a magnetic carrier (e.g., a magnetic carrier according to any one of preceding items), said method comprising simultaneously conjugating said magnetic carrier (e.g., a magnetic particle, magnetic bead, metallic nano- or microparticle, metal oxide nano- or microparticle) to:
   i) an anti-glycoprotein antibody (e.g., an anti-glycoprotein antibody according any one of preceding items, e.g., an anti-PSA, anti-AFP, anti-MUC16, anti-WFDC2, anti-MUC1, anti-ERBB2, anti-CEACAM5, anti-FUT3, anti-TG antibody or an antibody selected from the group of antibodies as described in Table 1 herein) or an antigen binding portion thereof (e.g., an antigen binding portion thereof according to any one of preceding items),
   ii) a polypeptide having peroxidase activity according to any one of preceding items.
35. A method comprising:
   a) providing:
      i) magnetic carrier according to any one of preceding items or anti-glycoprotein antibody (e.g., an anti-PSA, anti-AFP, anti-MUC16, anti-WFDC2, anti-MUC1, anti-ERBB2, anti-CEACAM5, anti-FUT3, anti-TG antibody or an antibody selected from the group of antibodies as described in Table 1 herein) or antigen binding portion thereof according to any one of preceding items;
      ii) one or more lectins (e.g., a lectin selected from the group of lectins as described in Table 1 herein), preferably said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL); further preferably said one or more lectins comprising MAA II, most preferably said one or more lectins are two lectins comprising MAA II, further most preferably said one or more lectins are two lectins comprising MAA II in combination with:
         aa) AAL, preferably said method has sensitivity of about 100% and/or said method has specificity of about 81.3%; or
         bb) Con A, preferably said method has sensitivity of about 100% and/or said method has specificity of about 93.8%; or
         cc) SNA-I, preferably said method has sensitivity of about 100% and/or said method has specificity of about 93.8%;
   b) determining the quantity, presence, or absence of oligosaccharide chains (e.g., glycans) covalently attached to a target polypeptide (e.g., biomarker) of said anti-glycoprotein antibody (e.g., target polypeptide according to any one of preceding items, e.g., PSA, AFP, MUC16, WFDC2, MUC1, ERBB2, CEACAM5, FUT3, TG, or a biomarker selected from the group of biomarkers as described in Table 1 herein), preferably said target polypeptide is PSA, further preferably said determining comprises the use of magnetic carrier and/or anti-glycoprotein antibody and/or the antigen binding portion thereof and/or one or more lectins and/or composition and/or kit, e.g., magnetic carrier and/or anti-glycoprotein antibody and/or the antigen binding portion thereof and/or one or more lectins and/or composition and/or kit according to any one of preceding or following items.
36. The method according to any one of preceding items, wherein binding and detection (e.g., by the optical means) of said target polypeptide (e.g., PSA, AFP, MUC16, WFDC2, MUC1, ERBB2, CEACAM5, FUT3, TG a biomarker selected from the group of biomarkers as described in Table 1 herein) is carried out simultaneously, preferably said target polypeptide is PSA.
37. The method according to any one of preceding items, wherein binding, enriching and detection (e.g., by the optical means) of said target polypeptide (e.g., PSA, AFP, MUC16, WFDC2, MUC1, ERBB2, CEACAM5, FUT3, TG, or a biomarker selected from the group of biomarkers as described in Table 1 herein) is carried out simultaneously, preferably said target polypeptide is PSA.
38. The method according to any one of preceding items, wherein said method is the method for one or more of the following:
   i) for selective capture and/or enrichment of a target polypeptide (e.g., biomarker) according to any one of preceding items (e.g., PSA, AFP, MUC16, WFDC2, MUC1, ERBB2, CEACAM5, FUT3, TG, or a biomarker selected from the group of biomarkers as described in Table 1 herein), preferably said target polypeptide is PSA;
   ii) for selective capture and/or enrichment of a target polypeptide according to any one of preceding items (e.g., PSA, AFP, MUC16, WFDC2, MUC1, ERBB2, CEACAM5, FUT3, TG, or a biomarker selected from the group of biomarkers as described in Table 1 herein, preferably said target polypeptide is PSA), wherein said method for selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate);
   iii) for glycoprofiling of a target polypeptide (e.g., biomarker) according to any one of preceding items (e.g., PSA, AFP, MUC16, WFDC2, MUC1, ERBB2, CEACAM5, FUT3, TG, or a biomarker selected from the group of biomarkers as described in Table 1 herein, preferably said target polypeptide is PSA);
   iv) for screening and/or analysing oligosaccharide chains (e.g., glycans) covalently attached to a target polypeptide (e.g., biomarker) according to any one of preceding items (e.g., PSA, AFP, MUC16, WFDC2, MUC1, ERBB2, CEACAM5, FUT3, TG, or a biomarker selected from the group of biomarkers as described in Table 1 herein, preferably said target polypeptide is PSA);
   v) for diagnostics of cancer (e.g., prostate cancer or a cancer selected from the group of cancers as described in Table 1 herein);
   vi) for positive and/or negative prediction of cancer (e.g., prostate cancer or a cancer selected from the group of cancers as described in Table 1 herein);
   vii) for determining a clinical stage of cancer (e.g., prostate cancer or a cancer selected from the group of cancers as described in Table 1 herein);
   viii) for distinguishing between prostate cancer and metastasizing prostate cancer;
   ix) for identifying prostate cancer likely to metastasize (e.g. likely to metastasize to the bone), preferably a cancer is selected from the group of cancers as described in Table 1 herein;
   x) for distinguishing between benign prostatic hyperplasia (BPH) and prostate cancer;
   xi) for prevention and/or treatment of cancer (e.g., prostate cancer or a cancer selected from the group of cancers as described in Table 1 herein);
   xii) for discriminating between significant and insignificant tumours, e.g., by means of glycoprotein-based (e.g., target polypeptide (e.g., biomarker) -based, e.g., based on PSA, AFP, MUC16, WFDC2, MUC1, ERBB2, CEACAM5, FUT3, TG, or a biomarker selected from the group of biomarkers as described in Table 1 herein, preferably said target polypeptide is PSA) diagnostics of cancer (e.g. prostate cancer or a cancer selected from the group of cancers as described in Table 1 herein);
   xiii) for discriminating between slow growing (e.g., clinically harmless) and fast growing (e.g., clinically relevant) tumours, e.g., by means of glycoprotein-based (e.g., target polypeptide (e.g., biomarker) -based, e.g., based on PSA, AFP, MUC16, WFDC2, MUC1, ERBB2, CEACAM5, FUT3, TG, or a biomarker selected from the group of biomarkers as described in Table 1 herein, preferably said target polypeptide is PSA) diagnostics of cancer (e.g. prostate cancer or a cancer selected from the group of cancers as described in Table 1 herein);
   xiv) for identifying organ confined and/or potentially curable cancers (e.g., prostate cancer or a cancer selected from the group of cancers as described in Table 1 herein), e.g., by means of glycoprotein-based (e. g., target polypeptide (e.g., biomarker) -based, e.g., based on PSA, AFP, MUC16, WFDC2, MUC1, ERBB2, CEACAM5, FUT3, TG or a biomarker selected from the group of biomarkers as described in Table 1 herein, preferably said target polypeptide is PSA) diagnostics of cancer;
   xv) for screening compounds.
39. The method according to any one of preceding items, wherein said method is the method for screening compounds and said method further comprising:
   c) providing:
      iii) a cancer sample (e.g., prostate cancer sample or a sample of a cancer selected from the group of cancers as described in Table 1 herein) with a test compound
   d) contacting said prostate cancer sample with said test compound; and
   e) determining the likelihood of said prostate cancer cell to metastasize based on the determined quantity, presence, or absence of oligosaccharide chains (e.g., glycans) covalently attached to a target polypeptide (e.g., according to any one of preceding items, e.g., PSA, AFP, MUC16, WFDC2, MUC1, ERBB2, CEACAM5, FUT3, TG, or a biomarker selected from the group of biomarkers as described in Table 1 herein, preferably said target polypeptide is PSA) in said prostate cancer sample before and after contacting said prostate cancer sample with said test compound.
40. The method according to any one of preceding items, wherein said method is the method:
   i) for positive prediction of prostate cancer and/or ii) for distinguishing between benign prostatic hyperplasia (BPH) and prostate cancer; wherein said one or more lectins comprise MAA II or WFL, preferably said one or more lectins comprise MAA II.
41. The method according to any one of preceding items, wherein said one or more lectins is MAA II, wherein the sensitivity and/or specificity of said method is about 87.5%.
42. The method according to any one of preceding items, wherein said one or more lectins are two lectins comprising MAA II in combination with: i) Con A, wherein said method has sensitivity of about 100% and/or said method has specificity of is about 93.8%; or ii) SNA-I, wherein said method has sensitivity of about 100% and/or said method has specificity of about 93.8%; or iii) AAL, wherein said method has sensitivity of about 100% and/or said method has specificity of about 81.3%.
43. The method according to any one of preceding items, wherein said lectin is WFL, wherein the sensitivity of said method is about 50% and/or specificity of said method is about 75%.
44. The method according to any one of preceding items, wherein said method is the method: i) for negative prediction of prostate cancer and/or ii) for distinguishing between benign prostatic hyperplasia (BPH) and prostate cancer; wherein said lectin is selected from the group consisting of: Con A, AAL and SNA-I.
45. The method according to any one of preceding items, wherein said lectin is Con A, wherein the sensitivity of said method is about 50% and/or specificity of said method is about 75%.
46. The method according to any one of preceding items, wherein said lectin is AAL, wherein the sensitivity of said method is about 50% and/or specificity of said method is about 75%.
47. The method according to any one of preceding items, wherein said lectin is SNA-I, wherein the sensitivity of said method is about 57% and/or specificity of said method is about 75%.
48. The method according to any one of preceding items, wherein said method further comprising the step of determining a treatment course of action based on determined quantity, presence, or absence of oligosaccharide chains (e.g., glycans) covalently attached to a target polypeptide of said anti-glycoprotein antibody (e.g., target polypeptide according to any one of preceding items, e.g., PSA, AFP, MUC16, WFDC2, MUC1, ERBB2, CEACAM5, FUT3, TG, or a biomarker selected from the group of biomarkers as described in Table 1 herein), preferably said target polypeptide is PSA.
49. The method according to any one of preceding items, wherein said method is the method for distinguishing between prostate cancer and metastasizing prostate cancer in a sample (e.g., in a serum sample), wherein said one or more lectins are selected from the group consisting of: AAL, Con A, MAA II and SNA-I, preferably selected from the group consisting of: AAL, Con A and MAA II, further preferably selected from the group consisting of: AAL and Con A.
50. The method according to any one of preceding items, wherein said method is carried out in a sample.
51. The method according to any one of preceding items, wherein said sample is selected from the group of urine, blood, serum, biopsy and post-surgical tissue sample, preferably a serum sample.
52. A method of determining the glycoprofile of a protein, comprising:
   (a) contacting a sample comprising said protein with an antibody directed against said protein to form an antibody-protein complex;
   (b) isolating the antibody-protein complex obtained in step (a); and
   (c) contacting the antibody-protein complex with: one or more lectins to determine the glycoprofile of said protein.
53. The method according to any one of preceding items, wherein said antibody of step (a) is not immobilized, preferably not immobilized on a solid surface.
54. The method according to any one of preceding items, further comprising step (d) comparing the glycoprofile of said protein with a control glycoprofile of said protein (e.g., a glycoprofile of said protein under conditions not associated with disease) to determine whether the glycoprofile of said protein may deviate from the glycoprofile of said control glycoprofile.
55. The method according to any one of preceding items, wherein said protein is a cancer biomarker protein, an autoimmune disease biomarker protein or an inflammatory disease biomarker protein, preferably said biomarker is selected from the group consisting of biomarkers as described in Table 1 herein.
56. The method according to any one of preceding items, wherein said cancer biomarker protein is an ovarian cancer biomarker protein, breast cancer biomarker protein, colorectal cancer biomarker protein, pancreatic cancer biomarker protein, prostate cancer biomarker protein, thyroid cancer biomarker protein, liver cancer biomarker protein, lung cancer biomarker protein, stomach cancer biomarker protein, testicular cancer biomarker protein or bladder cancer biomarker protein, preferably said biomarker is selected from the group consisting of biomarkers as described in Table 1 herein.
57. The method according to any one of preceding items, wherein ovarian cancer biomarker protein is α1-acid glycoprotein (UniProtKB Accession Number: P02763 and P19652), C1 esterase inhibitor (UniProtKB Accession Number: P05155, P00736 and P09871), 2HS glycoprotein (UniProtKB Accession Number: P02765), α1-antichymotrypsin (UniProtKB Accession Number: P01011), α1-antitrypsin (UniProtKB Accession Number: P01009), transferrin (UniProtKB Accession Number: P02787), cancer antigen CA125 (CA16) (UniProtKB Accession Number: Q8WXI7), cancer antigen CA 15-3 (MUC 1) (UniProtKB Accession Number: P15941), β-haptoglobin (UniProtKB Accession Number: P00738), human epididymis protein 4 (HE4) = WFDC2, hemopexin (UniProtKB Accession Number: P02790), clusterin (UniProtKB Accession Number: P10909), leucine-rich α-2-glycoprotein (UniProtKB Accession Number: P02750) or IgG (immunoglobulin G, antibody), preferably said biomarker is selected from the group consisting of biomarkers as described in Table 1 herein.
58. The method according to any one of preceding items, wherein breast cancer biomarker protein is cancer antigen CA 15-3, cancer antigen CA 27.29 (MUC 1, other epitope than CA 15-3) (UniProtKB Accession Number: P15941), CEA (CEACAM5), galectin 3 binding protein (UniProtKB Accession Number: Q08380) or HER2/neu (Receptor tyrosine-protein kinase erbB-2) (UniProtKB Accession Number: P04626), preferably said biomarker is selected from the group consisting of biomarkers as described in Table 1 herein.
59. The method according to any one of preceding items, wherein colorectal cancer biomarker protein is β-haptoglobin, CA19-9 (MUC 1, other epitope than CA 15-3 and CA 27.29) (UniProtKB Accession Number: P15941), CEA (CEACAM5), complement C3 (UniProtKB Accession Number: P01024), histidine rich glycoprotein (UniProtKB Accession Number: P04196) or kininogen-1 (UniProtKB Accession Number: P01042), preferably said biomarker is selected from the group consisting of biomarkers as described in Table 1 herein.
60. The method according to any one of preceding items, wherein pancreatic cancer biomarker protein is α1-β-glycoprotein (UniProtKB Accession Number: P04217), β-2-glycoprotein 1 (UniProtKB Accession Number: P02749), hemopexin, antithrombin-!!! (UniProtKB Accession Number: P01008), β-haptoglobin, haptoglobin-related protein (UniProtKB Accession Number: P00739), amyloid p-component (UniProtKB Accession Number: P02743), serum amyloid P-component= amyloid p-component, clusterin, plasma protease C1 inhibitor = C1 esterase inhibitor, α-1-antichymotrypsin, α-1-antitrypsin, thrombospondin-1 (UniProtKB Accession Number: P07996), MUC1, mucin 4 (CAM 17.1) (UniProtKB Accession Number: Q99102), MUC5ac (UniProtKB Accession Number: P98088), MUC16 = CA125 (CA16), or kininogen-1, preferably said biomarker is selected from the group consisting of biomarkers as described in Table 1 herein.
61. The method according to any one of preceding items, wherein prostate cancer biomarker protein is β-haptoglobin, metalloproteinase inhibitor 1 (TIMP-1) (UniProtKB Accession Number: P01033), PSA or tPSA (total or compexed PSA, making complex with α1-antichymotrypsin and α2-macroglobulin (UniProtKB Accession Number: P01023)), preferably said biomarker is selected from the group consisting of biomarkers as described in Table 1 herein.
62. The method according to any one of preceding items, wherein thyroid cancer biomarker protein is thyroglobulin (TG), preferably said biomarker is selected from the group consisting of biomarkers as described in Table 1 herein.
63. The method according to any one of preceding items, wherein liver cancer biomarker protein is α1-antitrypsin, α₁-antichymotrypsin, α-1-acid glycoprotein 1, α-fetoprotein (AFP) (UniProtKB Accession Number: P02771), α-fetoprotein fraction L3 (AFP-L3 fraction of AFP detected by LCA lectin), transferrin, ceruloplasmin (UniProtKB Accession Number: P00450), α-2-HS-glycoprotein, fetuin A = α-2-HS-glycoprotein = 2HS glycoprotein, C3 complement = complement C3, histidine rich glycoprotein, monocyte differentiation antigen CD14 (UniProtKB Accession Number: P08571), hepatocyte growth factor activator (UniProtKB Accession Number: Q04756), hemopexin, α-2-macroglobulin, preferably said biomarker is selected from the group consisting of biomarkers as described in Table 1 herein.
64. The method according to any one of preceding items, wherein lung cancer biomarker protein is β-haptoglobin, fibronectin (UniProtKB Accession Number: P02751), a₁-acid glycoprotein or α-1-antitrypsin, preferably said biomarker is selected from the group consisting of biomarkers as described in Table 1 herein.
65. The method according to any one of preceding items, wherein stomach cancer biomarkers is α1-acid glycoprotein, β-haptoglobin or leucine rich α-2-glycoprotein, preferably said biomarker is selected from the group consisting of biomarkers as described in Table 1 herein.
66. The method according to any one of preceding items, wherein testicular cancer biomarkers is α-fetoprotein-L3 or human chorionic gonadotropin-β (UniProtKB Accession Number: P0DN86), preferably said biomarker is selected from the group consisting of biomarkers as described in Table 1 herein.
67. The method according to any one of preceding items, wherein bladder cancer biomarker is MUC 1, MUC 16, α-1-antitrypsin, endoplasmin (UniProtKB Accession Number: P14625), Golgi apparatus protein 1 (UniProtKB Accession Number: Q92896), prostatic acid phosphatase (UniProtKB Accession Number: P15309), Ig gamma-2 chain C region (UniProtKB Accession Number: P01859), deoxyribonuclease-2-alpha (UniProtKB Accession Number: 000115) or integrin (UniProtKB Accession Number: P16144 and Q9UKX5), preferably said biomarker is selected from the group consisting of biomarkers as described in Table 1 herein.
68. The method according to any one of preceding items, wherein said autoimmune disease biomarker protein is IgG (immunoglobulin G), preferably said biomarker is selected from the group consisting of biomarkers as described in Table 1 herein.
69. The method according to any one of preceding items, wherein said inflammatory disease biomarker protein IgG (immunoglobulin G), IgA (immunoglobulin A) or C-reactive protein (CRP) (UniProtKB Accession Number: P02741), preferably said biomarker is selected from the group consisting of biomarkers as described in Table 1 herein.
70. The method according to any one of preceding items, wherein said antibody is not directed against a glycan attached to said protein (e.g. target polypeptide).
71. The method according to any one of preceding items, wherein said antibody is an antibody or fragment thereof selected from the group consisting of a monoclonal antibody, chimeric antibody, humanized antibody, human antibody, scFv, scAb, Fab, diabody, a DART, domain antibody, or nanobody.
72. The method according to any one of preceding items, wherein said antibody is a FN3 scaffold, adnectin, affibody, anticalin, avimer, a bicyclic peptide, DARPin, a Kunitz domain, an Obody or an aptamer, such as a DNA, RNA or peptide aptamer.
73. The method according to any one of preceding items, wherein said protein is not released from said antibody while performing the method.
74. The method according to any one of preceding items, wherein said antibody comprises a bead which allows the isolation of said antibody.
75. The method according to any one of preceding items, wherein said bead is an agarose bead, latex bead, metallic nano- or microparticle, metal oxide nano- or microparticle or magnetic bead.
76. The method according to any one of preceding items, wherein said antibody comprises a detectable label.
77. The method according to any one of preceding items, wherein said detectable label comprises a fluorophore, an enzyme, a radioisotope, a fluorescent protein, a fluorescent dye, or a tag.
78. The method according to any one of preceding items, wherein said enzyme is a peroxidase (e.g., having EC 1.11.1.7 activity), preferably microperoxidase 11 (MP-11), (MP-9) or (MP-8), alkaline phosphatase (from varous sources, preferentially from bovine intestine (UniProtKB Accession Number: P09487 and P19111)), β-galactosidase (from varous sources, preferentially from *Escherichia coli* (UniProtKB Accession Number: P00722, A7ZI91, B1J0T5, B7UJI9, B5Z2P7, Q8X685, A1A831, Q8FKG6, A7ZWZ1, B7N8Q1, B1LIM9, Q1RFJ2, Q0TKT1, Q8VNN2 and P06864)) or luciferase (from varous sources, preferentially from bovine intestine (UniProtKB Accession Number: P08659)).
79. The method according to any one of preceding items, wherein said one or more lectins are specific for core fucose, antennary fucose, Fucα1-6GlcNAc-*N*-Asn containing *N*-linked oligosaccharides, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GlcNAc, branched *N*-linked hexa-saccharide, Manα1-3Man, α-D-Man, (GlcNAcβ1-4)₂₋₄, Galβ1-4GlcNAc, GlcNAcα1-4Galβ1-4GlcNAc, (GlcNAcβ1-4)₂₋₅, Neu5Ac, Galβ1-3GalNAc-serine/threonine, Galα1-3GalNAc, Galβ1-6Gal, Galβ1-4GlcNAc, Galβ1-3GalNAc, GalNAcα1-3GalNAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAca1-3GαlNAc, GalNAcβ1-3/4Gal, GaINAc-Ser/Thr (Tn antigen), Galβ1-3GalNAc-Ser/Thr (T antigen), GalNAcβ1-4GlcNAc (LacdiNAc), α2,3Neu5Ac, α2,6Neu5Ac, α2,8Neu5Ac, sialic acid (α2,3Neu5Ac, α2,6Neu5Ac or α2,8Neu5Ac), Neu5Acα4/9-O-Ac-Neu5Ac, Neu5Acα2-3Galβ1-4Glc/GlcNAc, Neu5Acα2-6Gal/GalNAc, *N*-linked bi-antennary, N-linked tri/tetra-antennary, branched β1-6GlcNAc, Galα1-3(Fucα1-2)Galβ1-3/4GlcNAc, Galβ1-3(Fucα1-4)GlcNAc, NeuAcα,2-3Galβ1-3(Fucα1-4)GlcNAc, Fucα1-2Galβ1-3(Fucα1-4)GlcNAc, Galβ1-4(Fucα1-3)GlcNAc, NeuAcα2-3Galβ1-4(Fucα1-3)GlcNAc, Fucα1-2Galβ1-4(Fucα1-3)GlcNAc, high mannose, sialyl Lewis^{a} antigen (sialyl Le^{a}), sialyl Lewis^{x} antigen (sialyl Le^{x}), Lewis^{x} antigen (Le^{x}), sialyl Tn antigen, sialyl T antigen, Lewis^{y} antigen (Le^{y}), sulfated core1 glycan, Tn antigen, T antigen, core 2 glycan, Lewis^{a} antigen (Le^{a}), (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, branched (LacNAc)ₙ.
80. The method according to any one of preceding items, wherein the glycoprofile of PSA, and/or tPSA is determined by lectins specific for α2-3Neu5Ac (α2-3 linked sialic acid), α2-6Neu5Ac (α2-6 linked sialic acid), core fucosylation, antennary fucose, bi-antennary glycans, high mannose glycans, tri-/tetra-antennary glycans, LacdiNAc, GalNAc and/or polysialic acid (Neu5Acα2-8Neu5Ac).
81. The method according to any one of preceding items, wherein
   - an increase of α2-3Neu5Ac (α2-3 linked sialic acid),
   - a decrease of α2-6Neu5Ac (α2-6 linked sialic acid),
   - a decrease of core fucose,
   - an increase of antennary fucose,
   - an increase of core fucose,
   - an increase of fucose,
   - a decrease of bi-antennary glycans
   - a decrease of high mannose glycans,
   - an increase of tri-/tetra-antennary glycans,
   - an increase of LacdiNAc,
   - an increase of GalNAc and/or
   - presence of polysialic acid (Neu5Acα2-8Neu5Ac).
   is indicative of prostate cancer.
82. The method according to any one of preceding items, wherein
   - an increase of α2-3Neu5Ac (α2-3 linked sialic acid) is determined by *Maackia amurensis* agglutinin, anti-α2-3-linked Neu5Ac (α2-3 linked sialic acid) monoclonal antibody, Siglec 1, Siglec 4 or Siglec 8;
   - a decrease of α2-6Neu5Ac (α2-6 linked sialic acid) is determined by *Sambucus nigra* agglutinin or *Trichosanthes japonica* agglutinin I;
   - a decrease of core fucose is determined by *Pholiota squarrosa* lectin or *Aspergillus oryzae* agglutinin;
   - an increase of antennary fucose is determined by *Trichosanthes japonica* agglutinin-II, *Aleuria aurantia* agglutinin, *Ulex europaeus* I agglutinin, *Lens culinaris* agglutinin, *Pisum sativum* agglutinin, *Anguilla anguilla* agglutinin, *Lotus tetragonolobus* agglutinin;
   - a decrease of bi- antennary glycans is determined by Concanavalin A, *Galanthus nivalis* agglutinin, *Narcissus pseudonarcissus* (Daffodil) lectin;
   - and decrease of high mannose glycans is determined by Concanavalin A, *Galanthus nivalis* agglutinin, *Narcissus pseudonarcissus* (Daffodil) lectin;
   - an increase of bi- tri-/tetra-antennary glycans is determined by *Datura stramonium* agglutinin, *Phaseolus vulgaris* erythroagglutinin or *Phaseolus vulgaris* leukoagglutinin;
   - an increase of LacdiNAc is determined by *Wisteria floribunda* agglutinin
   - an increase of GalNAc is determined by *Wisteria floribunda* agglutinin;
   - presence of polysialic acid (Neu5Acα2-8Neu5Ac) is determined by a specific antibody, Siglec-7 or Siglec-11.
83. A method for diagnosing whether a subject may be at a risk or may suffer from cancer, comprising
   (a) contacting a sample obtained from said subject, said sample comprising a cancer biomarker protein, with an antibody directed against said cancer biomarker protein to form an antibody-cancer biomarker protein complex; and
   (b) isolating the antibody-protein complex obtained in step (a); and
   (c) contacting the antibody-cancer biomarker protein complex with one or more lectins to determine the glycoprofile of said cancer biomarker protein,
   wherein a deviation of said glycoprofile from the healthy glycoprofile of said cancer biomarker protein is indicative that said subject may be at a risk or may suffer from cancer, preferably said biomarker is selected from the group consisting of biomarkers as described in Table 1 herein.
84. The method according to any one of preceding items, wherein said cancer biomarker protein is one of those as defined according to any one of preceding items, preferably said biomarker is selected from the group consisting of biomarkers as described in Table 1 herein.
85. A method for diagnosing whether a subject may be at a risk or may suffer from an autoimmune disease, comprising
   (a) contacting a sample obtained from said subject, said sample comprising an autoimmune disease biomarker protein, with an antibody directed against said autoimmune disease biomarker protein to form an antibody-autoimmune disease biomarker protein complex; and
   (b) isolating the antibody-protein complex obtained in step (a); and
   (c) contacting the antibody-autoimmune disease biomarker protein complex with one or more lectins to determine the glycoprofile of said autoimmune disease biomarker protein,
   wherein a deviation of said glycoprofile from the healthy glycoprofile of said autoimmune disease biomarker protein is indicative that said subject may be at a risk or may suffer from an autoimmune disease, preferably said biomarker is selected from the group consisting of biomarkers as described in Table 1 herein.
86. The method according to any one of preceding items, wherein said autoimmune disease biomarker protein is defined according to any one of preceding items, preferably said biomarker is selected from the group consisting of biomarkers as described in Table 1 herein.
87. A method for diagnosing whether a subject may be at a risk or may suffer from an inflammatory disease, comprising
   (a) contacting a sample obtained from said subject, said sample comprising an inflammatory disease biomarker protein, with an antibody directed against said inflammatory disease biomarker protein to form an antibody-inflammatory biomarker protein complex; and
   (b) isolating the antibody-protein complex obtained in step (a); and
   (c) contacting the antibody-inflammatory biomarker protein complex with one or more lectins to determine the glycoprofile of said inflammatory disease biomarker protein,
   wherein a deviation of said glycoprofile from the healthy glycoprofile of said inflammatory disease biomarker protein is indicative that said subject may be at a risk or may suffer from an inflammatory disease, preferably said biomarker is selected from the group consisting of biomarkers as described in Table 1 herein.
88. The method according to any one of preceding items, wherein said inflammatory disease biomarker protein is defined according to any one of preceding items, preferably said biomarker is selected from the group consisting of biomarkers as described in Table 1 herein.
89. The method according to any one of preceding items (e.g., relating to PSA and prostate cancer), wherein the glycoprofile of PSA is determined by lectins specific for α2-3Neu5Ac (α2-3 linked sialic acid), α2-6Neu5Ac (α2-6 linked sialic acid), core fucosylation, antennary fucose, bi-antennary glycans, high mannose glycans, tri-/tetra-antennary glycans, LacdiNAc, GalNAc and/or polysialic acid (Neu5Acα2-8Neu5Ac).
90. The method according to any one of preceding items (e.g., relating to PSA and prostate cancer), wherein
   - an increase of α2-3Neu5Ac (α2-3 linked sialic acid),
   - a decrease of α2-6Neu5Ac (α2-6 linked sialic acid),
   - a decrease of core fucose,
   - an increase of antennary fucose,
   - an increase of fucose,
   - a decrease of bi-antennary glycans
   - a decrease of high mannose glycans,
   - an increase of tri-/tetra-antennary glycans,
   - an increase of LacdiNAc,
   - an increase of GalNAc and/or
   - presence of polysialic acid (Neu5Acα2-8Neu5Ac)
   is indicative of prostate cancer.
91. The method according to any one of preceding items (e.g., relating to PSA and prostate cancer), wherein
   - an increase of α2-3Neu5Ac (α2-3 linked sialic acid) is determined by *Maackia amurensis* agglutinin, anti-α2-3-linked Neu5Ac (α2-3 linked sialic acid) monoclonal antibody, Siglec 1, Siglec 4 and/or Siglec 8;
   - a decrease of α2-6Neu5Ac (α2-6 linked sialic acid) is determined by *Sambucus nigra* agglutinin and/or *Trichosanthes japonica* agglutinin I;
   - a decrease of core fucose is determined by *Pholiota squarrosa* lectin and/or *Aspergillus oryzae* agglutinin;
   - an increase of antennary fucose is determined by *Trichosanthes japonica* agglutinin-II, *Aleuria aurantia* agglutinin, *Ulex europaeus* I agglutinin, *Lens culinaris* agglutinin, *Pisum sativum* agglutinin, *Anguilla anguilla* agglutinin, and/or *Lotus tetragonolobus* agglutinin;
   - a decrease of bi- antennary glycans is determined by Concanavalin A, *Galanthus nivalis* agglutinin, *Narcissus pseudonarcissus* (Daffodil) lectin;
   - and decrease of high mannose glycans is determined by Concanavalin A, *Galanthus nivalis* agglutinin, *Narcissus pseudonarcissus* (Daffodil) lectin;
   - an increase of bi- tri-/tetra-antennary glycans is determined by *Datura stramonium* agglutinin, *Phaseolus vulgaris* erythroagglutinin and/or *Phaseolus vulgaris* leukoagglutinin;
   - an increase of LacdiNAc is determined by *Wisteria floribunda* agglutinin;
   - an increase of GalNAc is determined by *Wisteria floribunda* agglutinin; and/or
   - presence of polysialic acid (Neu5Acα2-8Neu5Ac) is determined by a specific antibody, Siglec-7 and/or Siglec-11.
92. The method according to any one of preceding items (e.g., relating to β-haptoglobin and prostate cancer), wherein the glycoprofile of β-haptoglobin is determined by lectins specific for α2-6Neu5Ac (α2-6 linked sialic acid), core fucosylation, antennary fucose, tri-/tetra-antennary glycans, sialyl Lewis^{a} glycan and/or sialyl Lewis" glycan.
93. The method according to any one of preceding items (e.g., relating to β-haptoglobin and prostate cancer), wherein
   - an increase of α2-6Neu5Ac (α2-6 linked sialic acid),
   - an increase of core fucosylation,
   - an increase of antennary fucose,
   - an increase of tri-/tetra-antennary glycans,
   - an increase of sialyl Lewis^{a} glycan and/or
   - an increase of sialyl Lewis^{x} glycan
   is indicative of prostate cancer.
94. The method according to any one of preceding items (e.g., relating to β-haptoglobin and prostate cancer), wherein
   - an increase of α2-6NeuAc (α2-6 linked sialic acid) is determined by *Sambucus nigra* agglutinin and/or *Trichosanthes japonica* agglutinin I;
   - an increase of core fucosylation is determined by *Pholiota squarrosa* lectin and/or *Aspergillus oryzae* agglutinin;
   - an increase of antennary fucose is determined by *Aleuria aurantia* agglutinin, *Trichosanthes japonica* agglutinin-II, *Ulex europaeus* I agglutinin, *Lens culinaris* agglutinin, *Pisum sativum* agglutinin, *Anguilla anguilla* agglutinin and/or *Lotus tetragonolobus* agglutinin;
   - an increase of tri-/tetra-antennary glycans is determined by *Phaseolus vulgaris* leukoagglutinin, *Datura stramonium* agglutinin and/or *Phaseolus vulgaris* erythroagglutinin; and/or
   - an increase of sialyl Lewis^{a} glycan or sialyl Lewis" glycans is determined using anti-sialyl Lewis^{a} glycan antibody, anti-sialyl Lewis" glycan antibody, *Sambucus nigra* agglutinin, *Trichosanthes japonica* agglutinin I, *Maackia amurensis* agglutinin, anti-α2-3-linked Neu5Ac (α2-3 linked sialic acid) monoclonal antibody, Siglec 1, Siglec 4 and/or Siglec 8.
95. The method according to any one of preceding items (e.g., relating to TIMP1 and prostate cancer), wherein the glycoprofile of TIMP1 is determined by lectins specific for α1-3/6 fucose.
96. The method according to any one of preceding items (e.g., relating to TIMP1 and prostate cancer), wherein
   - an increase of α1-3/6 fucose
   is indicative of prostate cancer.
97. The method according to any one of preceding items (e.g., relating to TIMP1 and prostate cancer), wherein
   - an increase of α1-3/6 fucose is determined by AOL, PhoSL, TJA II, UEA-I, LCA, PSL, AAA and/or LTA.
98. The method according to any one of preceding items (e.g., relating to α₁-acid glycoprotein and ovarian cancer), wherein the glycoprofile of α₁-acid glycoprotein is determined by lectins specific for tri-/tetra-antennary glycan, core fucosylation, α2-6Neu5Ac (α2-6 linked sialic acid), sialyl Lewis" glycan, and/or α2-3Neu5Ac (α2-3 linked sialic acid).
99. The method according to any one of preceding items (e.g., relating to α₁-acid glycoprotein and ovarian cancer), wherein
   - an increase of tri-/tetra-antennary glycan,
   - an increase of core fucosylation,
   - an increase of α2-6Neu5Ac (α2-6 linked sialic acid),
   - an increase of sialyl Lewis^{x} glycan, and/or
   - a decrease of α2-3Neu5Ac (α2-3 linked sialic acid)
   is indicative of ovarian cancer.
100. The method according to any one of preceding items (e.g., relating to α₁-acid glycoprotein and ovarian cancer), wherein
   - an increase of tri-/tetra-antennary glycan is determined by PHA-I, PHA-E and/or DSA;
   - an increase of core fucosylation is determined by PhoSL and/or AOL;
   - an increase of α2-6Neu5Ac (α2-6 linked sialic acid) is determined by TJA-I and/or SNA;
   - an increase of sialyl Lewis^{a} glycan is determined by an antibody against sLe^{x}, SNA, TJA-I, MAA, anti-α2-3-linked Neu5Ac (α2-3 linked sialic acid) antibody (e.g., HYB4), Siglec 1, Siglec 4 and/or Siglec 8; and/or
   - a decrease of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 and/or Siglec 8.
101. The method according to any one of preceding items (e.g., relating to C1 esterase inhibitor and ovarian cancer), wherein the glycoprofile of C1 esterase inhibitor is determined by lectins specific for Le^{x} and/or tri-antennary glycans.
102. The method according to any one of preceding items (e.g., relating to C1 esterase inhibitor and ovarian cancer), wherein
   - an increase of Le^{x} and/or
   - an increase of tri-antennary glycans
   is indicative of ovarian cancer.
103. The method according to any one of preceding items (e.g., relating to C1 esterase inhibitor and ovarian cancer), wherein
   - an increase of Le^{x} is determined by an antibody against Le^{x} and/or LTA, and/or
   - an increase of tri-antennary glycans is determined by DBA, PHA-E and/or PHA-L.
104. The method according to any one of preceding items (e.g., relating to 2-HS glycoprotein and ovarian cancer), wherein the glycoprofile of 2-HS glycoprotein is determined by lectins specific for tri-tetra-antennary glycans.
105. The method according to any one of preceding items (e.g., relating to 2-HS glycoprotein and ovarian cancer), wherein
   - an increase of tri-tetra-antennary glycans
   is indicative of ovarian cancer.
106. The method according to any one of preceding items (e.g., relating to 2-HS glycoprotein and ovarian cancer), wherein
   - an increase of tri-/tetra-antennary glycans is determined by DBA, PHA-E and/or PHA-L.
107. The method according to any one of preceding items (e.g., relating to β-haptoglobin and ovarian cancer), wherein the glycoprofile of β-haptoglobin is determined by lectins specific for tri-, tetra-antennary glycans, Le^{x}, sialyl Le^{x}, α2-3Neu5Ac (α2-3 linked sialic acid), α2-6Neu5Ac (α2-6 linked sialic acid), antennary fucose and/or bi-antennary glycans.
108. The method according to any one of preceding items (e.g., relating to β-haptoglobin and ovarian cancer), wherein
   - an increase of tri-/tetra-antennary glycans,
   - an increase of Le^{x},
   - an increase of sialyl Le^{x},
   - an increase of α2-6Neu5Ac (α2-6 linked sialic acid),
   - a decrease of α2-3Neu5Ac (α2-3 linked sialic acid),
   - an increase of α2-3Neu5Ac (α2-3 linked sialic acid),
   - a decrease of α2-6Neu5Ac (α2-6 linked sialic acid),
   - an increase of antennary fucose and/or
   - a decrease of bi-antennary glycans
   is indicative of ovarian cancer.
109. The method according to any one of preceding items (e.g., relating to β-haptoglobin and ovarian cancer), wherein
   - an increase of tri-/tetra-antennary glycans is determined by DBA, PHA-E and/or PHA-L;
   - an increase of Le^{x} is determined by an antibody against Le^{x} and/or LTA;
   - an increase of sialyl Le^{x} is determined by an antibody against sLe^{x}, SNA, TJA-I, MAA, anti- α2-3-linked Neu5Ac (α2-3 linked sialic acid) antibody (e.g., HYB4), Siglec 1, Siglec 4 and/or Siglec 8;
   - an increase of α2-6Neu5Ac (α2-6 linked sialic acid) is determined by SNA and/or TJA-I;
   - a decrease of α2-3Neu5Ac is determined by MAA, anti-(2-3-linked Neu5Ac (α2-3 linked sialic acid) antibody (e.g., HYB4), Siglec 1, Siglec 4 and/or Siglec 8;
   - a decrease of α2-6Neu5Ac (α2-6 linked sialic acid) is determined by SNA and/or TJA-I;
   - an increase of antennary fucose is determined by TJA II, AAL, UEA-I, LCA, PSL, AAA and/or AAL;
   - a decrease of bi-antennary glycans is determined by Con A and/or GNA and/or
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 and/or Siglec 8.
110. The method according to any one of preceding items (e.g., relating to α-1-antitrypsin and ovarian cancer), wherein the glycoprofile of α-1-antitrypsin is determined by lectins specific for tetra-antennary glycans, Le^{x}, tri-, tetra-antennary glycans, α2-3Neu5Ac (α2-3 linked sialic acid), α2-6Neu5Ac (α2-6 linked sialic acid), core fucose and/or bi-antennary glycans.
111. The method according to any one of preceding items (e.g., relating to α-1-antitrypsin and ovarian cancer), wherein
   - an increase of tetra-antennary glycans,
   - an increase of Le^{x},
   - a decrease of tri-, tetra-antennary glycans,
   - a decrease of α2-3Neu5Ac (α2-3 linked sialic acid),
   - an increase of α2-6Neu5Ac (α2-6 linked sialic acid),
   - an increase of core fucose and/or
   - an increase of bi-antennary glycans,
   is indicative of ovarian cancer.
112. The method according to any one of preceding items (e.g., relating to α-1-antitrypsin and ovarian cancer), wherein
   - an increase of tetra-antennary glycans is determined by DBA, PHA-E and/or PHA-L;
   - an increase of Le^{x} is determined by Antibody against Le^{x} and/or LTA;
   - a decrease of tri-, tetra-antennary glycans is determined by DBA, PHA-E and/or PHA-L;
   - a decrease of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 and/or Siglec 8;
   - an increase of α2-6Neu5Ac (α2-6 linked sialic acid) is determined by SNA and/or TJA-I;
   - an increase of core fucose is determined by AOL and/or PhoSL and/or
   - an increase of bi-antennary is determined by Con A, NPA and/or GNA.
113. The method according to any one of preceding items (e.g., relating to α-1-antichymotrypsin and ovarian cancer), wherein the glycoprofile of α-1-antichymotrypsin is determined by lectins specific for tetra-antennary glycans, Le^{x,}, sialyl Le^{x}, α2-6Neu5Ac (α2-6 linked sialic acid) and/or α2-3Neu5Ac (α2-3 linked sialic acid).
114. The method according to any one of preceding items (e.g., relating to α-1-antichymotrypsin and ovarian cancer), wherein
   - an increase of tetra-antennary glycans,
   - an increase of Le^{x},
   - an increase of sialyl Le^{x},
   - an increase of α2-6Neu5Ac (α2-6 linked sialic acid), and/or
   - a decrease of α2-3Neu5Ac (α2-3 linked sialic acid)
   is indicative of ovarian cancer.
115. The method according to any one of preceding items (e.g., relating to α-1-antichymotrypsin and ovarian cancer), wherein
   - an increase of tetra-antennary glycans is determined by DBA, PHA-E and/or PHA-L;
   - an increase of Le^{x} is determined by an antibody against Le^{x} and/or LTA;
   - an increase of sialyl Le^{x} is determined by an antibody against sLe^{x}, SNA, TJA-I, MAA, anti- α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I; and/or
   - a decrease of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 and/or Siglec 8.
116. The method according to any one of preceding items (e.g., relating to transferrin and ovarian cancer), wherein the glycoprofile of transferrin is determined by lectins specific for tri-antennary glycans.
117. The method according to any one of preceding items (e.g., relating to transferrin and ovarian cancer), wherein
   - a decrease of tri-antennary glycans
   is indicative of ovarian cancer.
118. The method according to any one of preceding items (e.g., relating to transferrin and ovarian cancer), wherein
   - a decrease of tri-antennary glycans is determined by DBA, PHA-E and/or PHA-L.
119. The method according to any one of preceding items (e.g., relating to hemopexin and ovarian cancer), wherein the glycoprofile of hemopexin is determined by lectins specific for Le^{x}.
120. The method according to any one of preceding items (e.g., relating to hemopexin and ovarian cancer), wherein
   - an increase of Le^{x}
   is indicative of ovarian cancer.
121. The method according to any one of preceding items (e.g., relating to hemopexin and ovarian cancer), wherein
   - an increase of Le^{x} is determined by an antibody against Le^{x} and/or LTA.
122. The method according to any one of preceding items (e.g., relating to IgG and ovarian cancer), wherein the glycoprofile of IgG is determined by lectins specific for galactose and/or sialic acid.
123. The method according to any one of preceding items (e.g., relating to IgG and ovarian cancer), wherein
   - a decrease of galactose and/or
   - a decrease of sialic acid
   is indicative of ovarian cancer.
124. The method according to any one of preceding items (e.g., relating to IgG and ovarian cancer), wherein
   - a decrease of galactose is determined by RCA, RCA120, ABA, Jacalin (DSA), AlloA, ECL and/or PNA and/or
   - a decrease of sialic acid is determined by SNA, TJA-I, MAA, anti-α2-3-linked Neu5Ac (sialic acid) antibody (e.g., HYB4), Siglec 1, Siglec 4 or Siglec 8.
125. The method according to any one of preceding items (e.g., relating to CA125 (MUC16) and ovarian cancer), wherein the glycoprofile of CA125 (MUC16) is determined by lectins specific for sialyl Tn antigen and/or sialyl T antigen.
126. The method according to any one of preceding items (e.g., relating to CA125 (MUC16) and ovarian cancer), wherein
   - an increase of sialyl Tn antigen and/or
   - an increase of sialyl T antigen
   is indicative of ovarian cancer.
127. The method according to any one of preceding items (e.g., relating to CA125 (MUC16) and ovarian cancer), wherein
   - an increase of sialyl Tn antigen is determined by VVA lectin after sialidase treatment, SNA, TJA-I, MAA, anti-α2-3-linked Neu5Ac (α2-3 linked sialic acid) antibody (e.g., HYB4), Siglec 1, Siglec 4 or Siglec 8 and/or
   - an increase of sialyl T antigen is determined by anticarbohydrate IgM antibodies 3C9 after sialidase detection, SNA, TJA-I, MAA, anti-α2-3-linked Neu5Ac (α2-3 linked sialic acid) antibody (e.g., HYB4), Siglec 1, Siglec 4 and/or Siglec 8.
128. The method according to any one of preceding items (e.g., relating to CA15-3 (MUC1) and ovarian cancer), wherein the glycoprofile of CA15-3 (MUC1) is determined by lectins specific for sialyl Tn antigen, core fucose, bi-antennary glycans, tri-/tetra-antennary glycans and/or antennary fucose.
129. The method according to any one of preceding items (e.g., relating to CA15-3 (MUC1) and ovarian cancer), wherein
   - an increase of sialyl Tn antigen,
   - an increase of core fucose,
   - an increase of bi-antennary glycans,
   - a decrease of tri-/tetra-antennary glycans and/or
   - an increase of antennary fucose
   is indicative of ovarian cancer.
130. The method according to any one of preceding items (e.g., relating to CA15-3 (MUC1) and ovarian cancer), wherein
   - an increase of sialyl Tn antigen is determined by VVA lectin after sialidase detection, SNA, TJA-I, MAA, anti-α2-3-linked Neu5Ac (α2-3 linked sialic acid) antibody (e.g., HYB4), Siglec 1, Siglec 4 or Siglec 8;
   - an increase of core fucose is determined by PhoSL and/or AOL;
   - an increase of bi-antennary glycans is determined by Con A;
   - a decrease of tri-/tetra-antennary glycans is determined by PHA-E, PHA-L and/or DBA and/or
   - an increase of antennary fucose is determined by AAL, TJA II, AAL, UEA-I, LCA, PSL, AAA and/or LTA.
131. The method according to any one of preceding items (e.g., relating to human epididymis protein 4 (HE4) and ovarian cancer), wherein the glycoprofile of human epididymis protein 4 (HE4) is determined by lectins specific for Le^{y} antigen.
132. The method according to any one of preceding items (e.g., relating to human epididymis protein 4 (HE4) and ovarian cancer), wherein
   - an increase of Le^{y} antigen
   is indicative of ovarian cancer.
133. The method according to any one of preceding items (e.g., relating to human epididymis protein 4 (HE4) and ovarian cancer), wherein
   - an increase of Le^{y} antigen is determined by an antibody against Lewis^{y} glycan and/or UEA-I.
134. The method according to any one of preceding items (e.g., relating to clusterin and ovarian cancer), wherein the glycoprofile of Clusterin is determined by lectins specific for α2-6Neu5Ac (α2-6 linked sialic acid).
135. The method according to any one of preceding items (e.g., relating to clusterin and ovarian cancer), wherein
   - an increase of α2-6Neu5Ac (α2-6 linked sialic acid)
   is indicative of ovarian cancer.
136. The method according to any one of preceding items (e.g., relating to clusterin and ovarian cancer), wherein
   - an increase of α2-6Neu5Ac (α2-6 linked sialic acid) is determined by SNA and/or TJA-I.
137. The method according to any one of preceding items (e.g., relating to leucine-rich α-2-glycoprotein and ovarian cancer), wherein the glycoprofile of leucine-rich α-2-glycoprotein is determined by lectins specific for α2-6Neu5Ac (α2-6 linked sialic acid).
138. The method according to any one of preceding items (e.g., relating to leucine-rich α-2-glycoprotein and ovarian cancer), wherein
   - an increase of α2-6Neu5Ac (α2-6 linked sialic acid)
   is indicative of ovarian cancer.
139. The method according to any one of preceding items (e.g., relating to leucine-rich α-2-glycoprotein and ovarian cancer), wherein
   - an increase of α2-6Neu5Ac (α2-6 linked sialic acid) is determined by SNA and/or TJA-I.
140. The method according to any one of preceding items (e.g., relating to CA15-3 (MUC1) and breast cancer), wherein the glycoprofile of CA15-3 (MUC1) is determined by lectins specific for sulfated core1 glycan, Tn, sialyl Tn antigens, sialyl T, T antigens, α2-8Neu5Ac (α2-8 linked sialic acid), sialylation and/or core 2 glycan.
141. The method according to any one of preceding items (e.g., relating to CA15-3 (MUC1) and breast cancer), wherein
   - an increase of sulfated core1 glycan,
   - an increase of Tn or sialyl Tn antigens,
   - presence of sialyl T or T antigens,
   - presence of α2-8Neu5Ac (α2-8 linked sialic acid),
   - presence of sialylation and/or
   - presence of core 2 glycan
   is indicative of breast cancer.
142. The method according to any one of preceding items (e.g., relating to CA15-3 (MUC1) and breast cancer), wherein
   - an increase of sulfated core1 glycan is determined by Galectin 4, SBA, ABA, VVA, Jacalin (DSA), BPL, PNA, GSL1 and/or SJA;
   - an increase of Tn or sialyl Tn antigens is determined by SBA, DBA, VVA, SNA, SNA, TJA-I, MAA and/or anti-α2-3-linked Neu5Ac (α2-3 linked sialic acid) antibody (e.g., HYB4);
   - presence of sialyl Tn or T antigens is determined by SNA, TJA-I, MAA, anti-α2-3-linked Neu5Ac (α2-3 linked sialic acid) antibody (e.g., HYB4), Siglec 1, Siglec 4 or Siglec 8; SBA, ABA, VVA, BPL, Jacalin and/or PNA;
   - presence of α2-8Neu5Ac (α2-8 linked sialic acid) is determined by antibody against poly(sialic acid), Siglec 7 and/or Siglec 11;
   - presence of sialylation is determined by SNA, TJA-I, TVA, MAA, anti-α2-3-linked Neu5Ac (α2-3 linked sialic acid) antibody (e.g., HYB4), Siglec 1, Siglec 4 and/or Siglec 8 and/or
   - presence of core 2 glycan is determined by RCA, RCA120, ABA, Jacalin (DSA), PNA and/or WGA.
143. The method according to any one of preceding items (e.g., relating to CA27.29 and breast cancer), wherein the glycoprofile of CA27.29 is determined by lectins specific for sialylation.
144. The method according to any one of preceding items (e.g., relating to CA27.29 and breast cancer), wherein a presence of sialylation is indicative of breast cancer.
145. The method according to any one of preceding items (e.g., relating to CA27.29 and breast cancer), wherein presence of sialylation is determined by SNA, TJA-I, MAA, anti-α2-3-linked Neu5Ac (α2-3 linked sialic acid) antibody (e.g., HYB4), Siglec 1, Siglec 4 and/or Siglec 8.
146. The method according to any one of preceding items (e.g., relating to HER2 and breast cancer), wherein the glycoprofile of HER2 is determined by lectins specific for antennary fucose and/or sialylation.
147. The method according to any one of preceding items (e.g., relating to HER2 and breast cancer), wherein
   - a presence of antennary fucose and/or
   - a presence of sialylation
   is indicative of breast cancer.
148. The method according to any one of preceding items (e.g., relating to HER2 and breast cancer), wherein
   - a presence of antennary fucose is determined by UEA, TJA II, AAL, LCA, PSL, AAA and/or LTA and/or
   - a presence of sialylation is determined by SNA, TJA-I, TVA, MAA, anti-α2-3-linked Neu5Ac (α2-3 linked sialic acid) antibody (e.g., HYB4), Siglec 1, Siglec 4 and/or Siglec 8.
149. The method according to any one of preceding items (e.g., relating to CEA and breast cancer), wherein the glycoprofile of CEA is determined by lectins specific for tri-, tetra-antennary glycans.
150. The method according to any one of preceding items (e.g., relating to CEA and breast cancer), wherein a presence of tri-, tetra-antennary glycans is indicative of breast cancer.
151. The method according to any one of preceding items (e.g., relating to CEA and breast cancer), wherein a presence of tri-, tetra-antennary glycans is determined by PHA-E, PHA-L and/or DBA.
152. The method according to any one of preceding items (e.g., relating to β-haptoglobin and colorectal cancer), wherein the glycoprofile of β-haptoglobin is determined by lectins specific for antennary fucose, bi-antennary glycans, antennary/core fucose, dimer: Le^{a} on Le^{a} and/or Gal-β-4-GlcNAc.
153. The method according to any one of preceding items (e.g., relating to β-haptoglobin and colorectal cancer), wherein
   - an increase of antennary fucose,
   - an increase of bi-antennary glycans,
   - an increase of antennary/core fucose,
   - an increase of dimer: Le^{a} on Le^{a} and/or
   - an increase of Gal-β1-4-GIcNAc
   is indicative of colorectal cancer.
154. The method according to any one of preceding items (e.g., relating to β-haptoglobin and colorectal cancer), wherein
   - an increase of antennary fucose is determined by AAL, TJA II, UEA-I, LCA, PSL, AAA and/or LTA;
   - an increase of bi-antennary glycans is determined by PHA-E, Con A, PHA-L and/or DBA;
   - an increase of antennary/core fucose is determined by AAL, AOL, LTA, TJA II, UEA-I, LCA, PSL, AAA and/or PhoSL;
   - an increase of dimer: Le^{a} on Le^{a} is determined by mouse monoclonal antibody NCC-ST-421 and/or
   - an increase of Gal-β1-4-GIcNAc is determined by Galectin 3, ECA and/or AlloA.
155. The method according to any one of preceding items (e.g., relating to Carcinoembryonic antigen (CEA) and colorectal cancer), wherein the glycoprofile of Carcinoembryonic antigen (CEA) is determined by lectins specific for Le^{x}, Le^{y}, α2-3Neu5Ac (α2-3 linked sialic acid), α-D-Man, tri-, tetra-antennary glycans, mannose, fucose, terminal GalNAc and/or Gal-β1-4-GIcNAc.
156. The method according to any one of preceding items (e.g., relating to Carcinoembryonic antigen (CEA) and colorectal cancer), wherein
   - an increase of Le^{x},
   - an increase of Le^{y},
   - an increase of α2-3Neu5Ac (α2-3 linked sialic acid),
   - an increase of α-D-Man,
   - an increase of tri-, tetra-antennary glycans,
   - an increase of mannose, fucose,
   - a decrease of terminal GalNAc and/or
   - an increase of Galβ1-4-GlcNAc
   is indicative of colorectal cancer.
157. The method according to any one of preceding items (e.g., relating to Carcinoembryonic antigen (CEA) and colorectal cancer), wherein
   - an increase of Le^{x} is determined by LTA and/or an antibody against sialyl Lewis^{x} glycan;
   - an increase of Le^{y} is determined by UEA-I and/or an antibody against sialyl Lewis^{y} glycan;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α-D-Man is determined by NPA, Con A and/or GNA;
   - an increase of tri-, tetra-antennary glycans is determined by PHA-L, PHA-E and/or DBA
   - an increase of mannose, fucose is determined by DC-SIGN, NPA, Con A, GNA, AAL, TJA II, UEA-I, LCA, PSL, AAA, LTA, AOL and/or PhoSL;
   - a decrease of terminal GalNAc is determined by MGBL, DBA, SBA, VVA, HPA and/or WFA and/or
   - an increase of Gal-β1-4-GIcNAc is determined by Galectin 3.
158. The method according to any one of preceding items (e.g., relating to CA 19-9 (MUC1) and colorectal cancer), wherein the glycoprofile of CA 19-9 (MUC1) is determined by lectins specific for T antigen, Galβ-1,3GalNAc, antennary fucose, α2-3Neu5Ac (α2-3 linked sialic acid), α2-6Neu5Ac (α2-6 linked sialic acid), tri-, tetra-antennary branching and/or terminal GaINAc.
159. The method according to any one of preceding items (e.g., relating to CA 19-9 (MUC1) and colorectal cancer), wherein
   - an increase of T antigen,
   - an increase of Galβ1-3GaINAc,
   - an increase of antennary fucose,
   - an increase of α2-3Neu5Ac (α2-3 linked sialic acid),
   - an increase of α2-6Neu5Ac (α2-6 linked sialic acid),
   - a decrease of tri-, tetra-antennary branching and/or
   - an increase of terminal GalNAc
   is indicative of colorectal cancer.
160. The method according to any one of preceding items (e.g., relating to CA 19-9 (MUC1) and colorectal cancer), wherein
   - an increase of T antigen is determined by SBA and/or ABA;
   - an increase of Galβ1-3GaINAc is determined by PNA, ABA and/or Jacalin;
   - an increase of antennary fucose is determined by UEA, TJA II, AAL, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - a decrease of tri-, tetra-antennary branching is determined by PHA-E, PHA-L and/or DBA and/or
   - an increase of terminal GalNAc is determined by MGBL, DBA, SBA, HPA and/or WFA.
161. The method according to any one of preceding items (e.g., relating to Complement C3 (UniProtKB: P01024) and colorectal cancer), wherein the glycoprofile of Complement C3 (UniProtKB: P01024) is determined by lectins specific for antennary fucose, Galβ1-3GaINAc, α2-3Neu5Ac (α2-3 linked sialic acid) and/or α2-6Neu5Ac (α2-6 linked sialic acid).
162. The method according to any one of preceding items (e.g., relating to Complement C3 (UniProtKB: P01024) and colorectal cancer), wherein
   - an increase of antennary fucose,
   - an increase of Galβ1-3GaINAc,
   - an increase of α2-3Neu5Ac (α2-3 linked sialic acid) and/or
   - an increase of α2-6Neu5Ac (α2-6 linked sialic acid)
   is indicative of colorectal cancer.
163. The method according to any one of preceding items (e.g., relating to Complement C3 (UniProtKB: P01024) and colorectal cancer), wherein
   - an increase of antennary fucose is determined by AAL, TJA II, UEA-I, LCA, PSL, AAA and/or LTA.
   - an increase of Galβ1-3GaINAc is determined by PNA, ABA and/or Jacalin;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8 and/or
   - an increase of α2-6Neu5Ac (α2-6 linked sialic acid) is determined by SNA and/or TJA-I.
164. The method according to any one of preceding items (e.g., relating to Kininogen-I (UniProtKB: P01042) and colorectal cancer), wherein the glycoprofile of Kininogen-I (UniProtKB: P01042) is determined by lectins specific for high mannose, antennary fucose, Galβ1-3GaINAc, α2-3Neu5Ac (α2-3 linked sialic acid) and/or α2-6Neu5Ac (α2-6 linked sialic acid).
165. The method according to any one of preceding items (e.g., relating to Kininogen-I (UniProtKB: P01042) and colorectal cancer), wherein
   - an increase of high mannose,
   - an increase of antennary fucose,
   - an increase of Galβ1-3GalNAc,
   - an increase of α2-3Neu5Ac (α2-3 linked sialic acid) and/or
   - an increase of α2-6Neu5Ac (α2-6 linked sialic acid)
   is indicative of colorectal cancer.
166. The method according to any one of preceding items (e.g., relating to Kininogen-I (UniProtKB: P01042) and colorectal cancer), wherein
   - an increase of high mannose is determined by Con A, NPA and/or GNA;
   - an increase of antennary fucose is determined by AAL, TJA II, UEA-I, LCA, PSL, AAA and/or LTA;
   - an increase of Galβ1-3GaINAc is determined by PNA, ABA and/or Jacalin;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 and/or Siglec 8 and/or
   - an increase of α2-6Neu5Ac (α2-6 linked sialic acid) is determined by SNA and/or TJA-I.
167. The method according to any one of preceding items (e.g., relating to Histidine-rich glycoprotein (UniProtKB: P04196) and colorectal cancer), wherein the glycoprofile of Histidine-rich glycoprotein (UniProtKB: P04196) is determined by lectins specific for antennary fucose and/or α2-6Neu5Ac (α2-6 linked sialic acid).
168. The method according to any one of preceding items (e.g., relating to Histidine-rich glycoprotein (UniProtKB: P04196) and colorectal cancer), wherein
   - an increase of antennary fucose and/or
   - an increase of α2-6Neu5Ac (α2-6 linked sialic acid)
   is indicative of colorectal cancer.
169. The method according to any one of preceding items (e.g., relating to Histidine-rich glycoprotein (UniProtKB: P04196) and colorectal cancer), wherein
   - an increase of antennary fucose is determined by TJA II, AAL, UEA-I, LCA, PSL, AAA and/or LTA and/or
   - an increase of α2-6Neu5Ac (α2-6 linked sialic acid) is determined by SNA and/or TJA-I.
170. The method according to any one of preceding items (e.g., relating to α₁-β-glycoprotein and pancreatic cancer), wherein the glycoprofile of α₁-β-glycoprotein is determined by lectins specific for Neu5Ac.
171. The method according to any one of preceding items (e.g., relating to α₁-β-glycoprotein and pancreatic cancer), wherein an increase of Neu5Ac is indicative of pancreatic cancer.
172. The method according to any one of preceding items (e.g., relating to α₁-β-glycoprotein and pancreatic cancer), wherein an increase of Neu5Ac is determined by SNA, TJA-I, anti-α2-3-linked Neu5Ac (α2-3 linked sialic acid) antibody (e.g., HYB4), Siglec 1, Siglec 4 and/or Siglec 8.
173. The method according to any one of preceding items (e.g., relating to α Amyloid p-component and pancreatic cancer), wherein the glycoprofile of Amyloid p-component is determined by lectins specific for Neu5Ac.
174. The method according to any one of preceding items (e.g., relating to α Amyloid p-component and pancreatic cancer), wherein an increase of Neu5Ac is indicative of pancreatic cancer.
175. The method according to any one of preceding items (e.g., relating to α Amyloid p-component and pancreatic cancer), wherein an increase of Neu5Ac is determined by SNA, TJA-I, anti-α2-3-linked Neu5Ac (α2-3 linked sialic acid) antibody (e.g., HYB4), Siglec 1, Siglec 4 and/or Siglec 8.
176. The method according to any one of preceding items (e.g., relating to β-2-glycoprotein 1 (UniProtKB: P02749) and pancreatic cancer), wherein the glycoprofile of β-2-glycoprotein 1 (UniProtKB: P02749) is determined by lectins specific for antennary fucose, α2-3Neu5Ac (α2-3 linked sialic acid), α2-6Neu5Ac (α2-6 linked sialic acid), high mannose and/or Galβ-1,3GalNAc.
177. The method according to any one of preceding items (e.g., relating to β-2-glycoprotein 1 (UniProtKB: P02749) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid),
   - an increase of high mannose and/or
   - an increase of Galβ1-3GaINAc
   is indicative of pancreatic cancer.
178. The method according to any one of preceding items (e.g., relating to β-2-glycoprotein 1 (UniProtKB: P02749) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - an increase of high mannose is determined by Con A, NPA and/or GNA and/or
   - an increase of Galβ-1,3GaINAc is determined by PNA, ABA and/or Jacalin.
179. The method according to any one of preceding items (e.g., relating to hemopexin (UniProtKB: P02790) and pancreatic cancer), wherein the glycoprofile of hemopexin (UniProtKB: P02790) is determined by lectins specific for antennary fucose, α2-3Neu5Ac (α2-3 linked sialic acid), α2-6Neu5Ac (α2-6 linked sialic acid) and/or high mannose.
180. The method according to any one of preceding items (e.g., relating to hemopexin (UniProtKB: P02790) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid) and/or
   - an increase of high mannose
   is indicative of pancreatic cancer.
181. The method according to any one of preceding items (e.g., relating to hemopexin (UniProtKB: P02790) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I and/or
   - an increase of high mannose is determined by Con A, NPA and/or GNA.
182. The method according to any one of preceding items (e.g., relating to haptoglobin-related protein (UniProtKB: P00739) and pancreatic cancer), wherein the glycoprofile of haptoglobin-related protein (UniProtKB: P00739) is determined by lectins specific for antennary fucose, α2-3Neu5Ac (α2-3 linked sialic acid), α2-6Neu5Ac (α2-6 linked sialic acid), high mannose and/or Galβ-1,3GaINAc.
183. The method according to any one of preceding items (e.g., relating to haptoglobin-related protein (UniProtKB: P00739) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid),
   - an increase of high mannose and/or
   - an increase of Galβ1-3GaINAc
   is indicative of pancreatic cancer.
184. The method according to any one of preceding items (e.g., relating to haptoglobin-related protein (UniProtKB: P00739) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - an increase of high mannose is determined by Con A, NPA and/or GNA and/or
   - an increase of Galβ1-3GaINAc is determined by PNA, ABA and/or Jacalin.
185. The method according to any one of preceding items (e.g., relating to serum amyloid P-component (UniProtKB: P02743) and pancreatic cancer), wherein the glycoprofile of serum amyloid P-component (UniProtKB: P02743) is determined by lectins specific for antennary fucose, α2-3Neu5Ac (α2-3 linked sialic acid), α2-6Neu5Ac (α2-6 linked sialic acid), high mannose and/or Galβ1-3GaINAc.
186. The method according to any one of preceding items (e.g., relating to serum amyloid P-component (UniProtKB: P02743) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid),
   - an increase of high mannose and/or
   - an increase of Galβ1-3GaINAc
   is indicative of pancreatic cancer.
187. The method according to any one of preceding items (e.g., relating to serum amyloid P-component (UniProtKB: P02743) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - an increase of high mannose is determined by Con A, NPA and/or GNA and/or
   - an increase of Galβ-1,3GaINAc is determined by PNA, ABA and/or Jacalin (DSA).
188. The method according to any one of preceding items (e.g., relating to clusterin (UniProtKB: P10909) and pancreatic cancer), wherein the glycoprofile of clusterin (UniProtKB: P10909) is determined by lectins specific for antennary fucose, α2-3Neu5Ac (α2-3 linked sialic acid), α2-6Neu5Ac (α2-6 linked sialic acid) and/or Galβ1-3GaINAc.
189. The method according to any one of preceding items (e.g., relating to clusterin (UniProtKB: P10909) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid) and/or
   - an increase of Galβ1-3GaINAc
   is indicative of pancreatic cancer.
190. The method according to any one of preceding items (e.g., relating to clusterin (UniProtKB: P10909) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I and/or
   - an increase of Galβ1-3GaINAc is determined by PNA, ABA and/or Jacalin.
191. The method according to any one of preceding items (e.g., relating to antithrombin-III (UniProtKB: P01008) and pancreatic cancer), wherein the glycoprofile of antithrombin-III (UniProtKB: P01008) is determined by lectins specific for antennary fucose, α2-3Neu5Ac (α2-3 linked sialic acid), α2-6Neu5Ac α2-6 linked (sialic acid), high mannose and/or Galβ1-3GaINAc.
192. The method according to any one of preceding items (e.g., relating to antithrombin-III (UniProtKB: P01008) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid),
   - an increase of high mannose and/or
   - an increase of Galβ1-3GaINAc
   is indicative of pancreatic cancer.
193. The method according to any one of preceding items (e.g., relating to antithrombin-III (UniProtKB: P01008) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - an increase of high mannose is determined by Con A, NPA and/or GNA and/or
   - an increase of Galβ1-3GaINAc is determined by PNA, ABA and/or Jacalin (DSA).
194. The method according to any one of preceding items (e.g., relating to kininogen-1 (UniProtKB: P01042) and pancreatic cancer), wherein the glycoprofile of kininogen-1 (UniProtKB: P01042) is determined by lectins specific for antennary fucose, α2-3Neu5Ac (α2-3 linked sialic acid), α2-6Neu5Ac (α2-6 linked sialic acid), high mannose and/or Galβ1-3GaINAc.
195. The method according to any one of preceding items (e.g., relating to kininogen-1 (UniProtKB: P01042) and pancreatic cancer), wherein
   - an increase of antennary fucose,
   - an increase of α2-3Neu5Ac (α2-3 linked sialic acid),
   - an increase of α2-6Neu5Ac (α2-6 linked sialic acid),
   - an increase of high mannose and/or
   - an increase of Galβ1-3GaINAc
   is indicative of pancreatic cancer.
196. The method according to any one of preceding items (e.g., relating to kininogen-1 (UniProtKB: P01042) and pancreatic cancer), wherein
   - an increase of antennary fucose is determined by AAL, TJA II, UEA-I, LCA, PSL, AAA and/or LTA;
   - an increase of α2-3Neu5Ac (α2-3 linked sialic acid) is determined by MAA, anti-α2-3-linked Neu5Ac (α2-3 linked sialic acid) antibody (e.g., HYB4), Siglec 1, Siglec 4 or Siglec 8;
   - an increase of α2-6Neu5Ac (α2-6 linked sialic acid) is determined by SNA and/or TJA-I;
   - an increase of high mannose is determined by Con A, NPA and/or GNA and/or
   - an increase of Galβ1-3GaINAc is determined by PNA, ABA and/or Jacalin (DSA).
197. The method according to any one of preceding items (e.g., relating to plasma protease C1 inhibitor (UniProtKB: P05155) and pancreatic cancer), wherein the glycoprofile of plasma protease C1 inhibitor (UniProtKB: P05155) is determined by lectins specific for α2-6Neu5Ac (α2-6 linked sialic acid).
198. The method according to any one of preceding items (e.g., relating to plasma protease C1 inhibitor (UniProtKB: P05155) and pancreatic cancer), wherein an increase of α2-6Neu5Ac (α2-6 linked sialic acid) is indicative of pancreatic cancer.
199. The method according to any one of preceding items (e.g., relating to plasma protease C1 inhibitor (UniProtKB: P05155) and pancreatic cancer), wherein an increase of α2-6Neu5Ac (α2-6 linked sialic acid) is determined by SNA and/or TJA-I.
200. The method according to any one of preceding items (e.g., relating to β-haptoglobin and pancreatic cancer), wherein the glycoprofile of β-haptoglobin is determined by lectins specific for antennary fucose and/or core fucose.
201. The method according to any one of preceding items (e.g., relating to β-haptoglobin and pancreatic cancer), wherein
   - an increase of antennary fucose and/or
   - an increase of core fucose
   is indicative of pancreatic cancer.
202. The method according to any one of preceding items (e.g., relating to β-haptoglobin and pancreatic cancer), wherein
   - an increase of antennary fucose is determined by AAL, TJA II, UEA-I, LCA, PSL, AAA and/or LTA and/or
   - an increase of core fucose is determined by AOL and/or PhoSL.
203. The method according to any one of preceding items (e.g., relating α-1-antichymotrypsin and pancreatic cancer), wherein the glycoprofile of α-1-antichymotrypsin is determined by lectins specific for antennary fucose.
204. The method according to any one of preceding items (e.g., relating α-1-antichymotrypsin and pancreatic cancer), wherein an increase of antennary fucose is indicative of pancreatic cancer.
205. The method according to any one of preceding items (e.g., relating α-1-antichymotrypsin and pancreatic cancer), wherein an increase of antennary fucose is determined by AAL, TJA II, UEA-I, LCA, PSL, AAA and/or LTA.
206. The method according to any one of preceding items (e.g., relating thrombospondin-1 and pancreatic cancer), wherein the glycoprofile of thrombospondin-1 is determined by lectins specific for antennary fucose.
207. The method according to any one of preceding items (e.g., relating thrombospondin-1 and pancreatic cancer), wherein an increase of antennary fucose is indicative of pancreatic cancer.
208. The method according to any one of preceding items (e.g., relating thrombospondin-1 and pancreatic cancer), wherein an increase of antennary fucose is determined by AAL, TJA II, UEA-I, LCA, PSL, AAA and/or LTA.
209. The method according to any one of preceding items (e.g., relating α-1-antitrypsin and pancreatic cancer), wherein the glycoprofile of α-1-antitrypsin is determined by lectins specific for antennary fucose.
210. The method according to any one of preceding items (e.g., relating α-1-antitrypsin and pancreatic cancer), wherein an increase of antennary fucose is indicative of pancreatic cancer.
211. The method according to any one of preceding items (e.g., relating α-1-antitrypsin and pancreatic cancer), wherein an increase of antennary fucose is determined by AAL, TJA II, UEA-I, LCA, PSL, AAA and/or LTA.
212. The method according to any one of preceding items (e.g., relating Mucin (CAM 17.1) and pancreatic cancer), wherein the glycoprofile of Mucin (CAM 17.1) is determined by lectins specific for β-D-GlcNAc and/or Neu5Ac.
213. The method according to any one of preceding items (e.g., relating Mucin (CAM 17.1) and pancreatic cancer), wherein an increase of β-D-GlcNAc and/or Neu5Ac is indicative of pancreatic cancer.
214. The method according to any one of preceding items (e.g., relating Mucin (CAM 17.1) and pancreatic cancer), wherein an increase of β-D-GlcNAc and/or Neu5Ac is determined by DSA, LEL, WGA; SNA and/or TJA-I.
215. The method according to any one of preceding items (e.g., relating MUC16 and pancreatic cancer), wherein the glycoprofile of MUC16 is determined by lectins specific for antennary fucose, T antigen, Gal-GIcNAc, GaINAc, GlcNAc and/or mannose.
216. The method according to any one of preceding items (e.g., relating MUC16 and pancreatic cancer), wherein
   - an increase of antennary fucose,
   - a decrease of T antigen,
   - a decrease of Gal-GIcNAc,
   - a decrease of GaINAc,
   - a decrease of GlcNAc and/or
   - a decrease of mannose
   is indicative of pancreatic cancer.
217. The method according to any one of preceding items (e.g., relating MUC16 and pancreatic cancer), wherein
   - an increase of antennary fucose is determined by AAL, TJA II, UEA-I, LCA, PSL, AAA and/or LTA;
   - a decrease of T antigen is determined by BPL, Jacalin (DSA), PNA, SBA, VVA, ABA, GSL1 and/or SJA;
   - a decrease of Gal-GIcNAc is determined by ECL, PHA-L, PHA-E, AlloA and/or ECA;
   - a decrease of GalNAc is determined by DBA, GSL1, SBA, VVL, SJA, ABA, BPL and/or PNA;
   - a decrease of GlcNAc is determined by GSL2, STL, DSA, LEL and/or WGA and/or
   - a decrease of mannose is determined by Con A, GNA and/or NPA.
218. The method according to any one of preceding items (e.g., relating MUC5ac and pancreatic cancer), wherein the glycoprofile of MUC5ac is determined by lectins specific for T antigen, antennary fucose, Gal-GIcNAc, GalNAc and/or GIcNAc.
219. The method according to any one of preceding items (e.g., relating MUC5ac and pancreatic cancer), wherein
   - an increase of T antigen,
   - an increase of antennary fucose,
   - a decrease of Gal-GIcNAc,
   - a decrease of GaINAc and/or
   - a decrease of GlcNAc
   is indicative of pancreatic cancer.
220. The method according to any one of preceding items (e.g., relating MUC5ac and pancreatic cancer), wherein
   - an increase of T antigen is determined by Jacalin (DSA), SBA, ABA, VVA, BPL, PNA, GSL1 and/or SJA;
   - an increase of antennary fucose is determined by TJA II, AAL, EA-I, LCA, PSL, AAA and/or LTA;
   - a decrease of Gal-GIcNAc is determined by ECA, PHA-L, RCA120, PHA-E and/or RCA;
   - a decrease of GalNAc is determined by DBA, VVA, SJA, GSL1, SBA, ABA, BPL and/or PNA and/or
   - a decrease of GlcNAc is determined by DSA, LEL, WGA, GSL2 and/or STL.
221. The method according to any one of preceding items (e.g., relating MUC1 and pancreatic cancer), wherein the glycoprofile of MUC1 is determined by lectins specific for Gal-GIcNAc, tetra-antennary glycans, T antigen, GaINAc, Gala -1,3Gal and/or GIcNAc.
222. The method according to any one of preceding items (e.g., relating MUC1 and pancreatic cancer), wherein
   - a decrease of Gal-GIcNAc, tetra-antennary glycans,
   - a decrease of T antigen,
   - a decrease of GalNAc,
   - an increase of Galα1-3Gal and/or
   - a decrease of GlcNAc
   is indicative of pancreatic cancer.
223. The method according to any one of preceding items (e.g., relating MUC1 and pancreatic cancer), wherein
   - a decrease of Gal-GIcNAc, tetra-antennary glycans is determined by ECA, PHA-L, RCA120, PHA-E, RCA and/or DBA;
   - a decrease of T antigen is determined by Jacalin (DSA), SBA, ABA, VVA, BPL, PNA, GSL1 and/or SJA;
   - a decrease of GalNAc is determined by DBA, VVA, SJA, GSL1, SBA, ABA, BPL and/or PNA;
   - an increase of Galα1-3Gal is determined by GSL 1and/or
   - a decrease of GlcNAc is determined by DSA, LEL, WGA, GSL2 and/or STL.
224. The method according to any one of preceding items (e.g., relating Thyroglobulin (TG) and thyroid cancer), wherein the glycoprofile of Thyroglobulin (TG) is determined by lectins specific for antennary fucose, terminal galactose, Gal-GIcNAc, tri-antennary glycans and/or mannose.
225. The method according to any one of preceding items (e.g., relating Thyroglobulin (TG) and thyroid cancer), wherein
   - a decrease of antennary fucose,
   - an increase of terminal galactose,
   - an increase of Gal-GIcNAc,
   - an increase of tri-antennary glycans,
   - an increase of antennary fucose and/or
   - an increase of mannose
   is indicative of thyroid cancer.
226. The method according to any one of preceding items (e.g., relating Thyroglobulin (TG) and thyroid cancer), wherein
   - a decrease of antennary fucose is determined by LCA, TJA II, AAL, UEA-I, PSL, AAA and/or LTA;
   - an increase of terminal galactose is determined by RCA, RCA120, ABA, AlloA, Jacalin (DSA), ECL and/or PNA;
   - an increase of Gal-GIcNAc is determined by ECA, PHA-L, RCA120, PHA-E and/or RCA;
   - an increase of, tri-antennary glycans is determined by PHA-E, PHA-L and/or DBA;
   - an increase of antennary fucose is determined by TJA II, AAL, UEA-I, LCA, PSL, AAA and/or LTA and/or
   - an increase of mannose is determined by Con A, NPA and/or GNA.
227. The method according to any one of preceding items (e.g., relating α₁-antitrypsin (AAT) and liver cancer), wherein the glycoprofile of α₁-antitrypsin (AAT) is determined by lectins specific for antennary fucose.
228. The method according to any one of preceding items (e.g., relating α₁-antitrypsin (AAT) and liver cancer), wherein an increase of antennary fucose is indicative of liver cancer.
229. The method according to any one of preceding items (e.g., relating α₁-antitrypsin (AAT) and liver cancer), wherein an increase of antennary fucose is determined by LCA, TJA II, UEA-I, AAL, PSL, AAA and/or LTA.
230. The method according to any one of preceding items (e.g., relating α-fetoprotein (AFP) and liver cancer), wherein the glycoprofile of α-fetoprotein (AFP) is determined by lectins specific for antennary fucose.
231. The method according to any one of preceding items (e.g., relating α-fetoprotein (AFP) and liver cancer), wherein an increase of antennary fucose is indicative of liver cancer.
232. The method according to any one of preceding items (e.g., relating α-fetoprotein (AFP) and liver cancer), wherein an increase of antennary fucose is determined by LCA, TJA II, UEA-I, AAL, PSL, AAA and/or LTA.
233. The method according to any one of preceding items (e.g., relating AFP-L3 (AFP) and liver cancer), wherein the glycoprofile of AFP-L3 is determined by lectins specific for antennary fucose.
234. The method according to any one of preceding items (e.g., relating AFP-L3 (AFP) and liver cancer), wherein an increase of antennary fucose is indicative of liver cancer.
235. The method according to any one of preceding items (e.g., relating AFP-L3 (AFP) and liver cancer), wherein an increase of antennary fucose is determined by LCA, TJA II, UEA-I, PSL, AAA and/or LTA.
236. The method according to any one of preceding items (e.g., relating transferrin and liver cancer), wherein the glycoprofile of transferrin (AFP) is determined by lectins specific for antennary fucose.
237. The method according to any one of preceding items (e.g., relating transferrin and liver cancer), wherein an increase of antennary fucose is indicative of liver cancer.
238. The method according to any one of preceding items (e.g., relating transferrin and liver cancer), wherein an increase of antennary fucose is determined by LCA, TJA II, UEA-I, PSL, AAA and/or LTA.
239. The method according to any one of preceding items (e.g., relating α₁-antichymotrypsin (AAT) and liver cancer), wherein the glycoprofile of α₁-antichymotrypsin (AAT) is determined by lectins specific for antennary fucose.
240. The method according to any one of preceding items (e.g., relating α₁-antichymotrypsin (AAT) and liver cancer), wherein an increase of antennary fucose is indicative of liver cancer.
241. The method according to any one of preceding items (e.g., relating α₁-antichymotrypsin (AAT) and liver cancer), wherein an increase of antennary fucose is determined by AAL, TJA II, UEA-I, LCA, PSL, AAA and/or LTA.
242. The method according to any one of preceding items (e.g., relating α-1-acid glycoprotein 1 and liver cancer), wherein the glycoprofile of α-1-acid glycoprotein 1 is determined by lectins specific for antennary fucose.
243. The method according to any one of preceding items (e.g., relating α-1-acid glycoprotein 1 and liver cancer), wherein an increase of antennary fucose is indicative of liver cancer.
244. The method according to any one of preceding items (e.g., relating α-1-acid glycoprotein 1 and liver cancer), wherein an increase of antennary fucose is determined by AAL, TJA II, UEA-I, LCA, PSL, AAA and/or LTA.
245. The method according to any one of preceding items (e.g., relating ceruloplasmin and liver cancer), wherein the glycoprofile of ceruloplasmin is determined by lectins specific for antennary fucose.
246. The method according to any one of preceding items (e.g., relating ceruloplasmin and liver cancer), wherein an increase of antennary fucose is indicative of liver cancer.
247. The method according to any one of preceding items (e.g., relating ceruloplasmin and liver cancer), wherein an increase of antennary fucose is determined by AAL, LCA, TJA II, UEA-I, LCA, PSL, AAA and/or LTA.
248. The method according to any one of preceding items (e.g., relating α-2-macroglobulin and liver cancer), wherein the glycoprofile of α-2-macroglobulin is determined by lectins specific for antennary fucose.
249. The method according to any one of preceding items (e.g., relating α-2-macroglobulin and liver cancer), wherein an increase of antennary fucose is indicative of liver cancer.
250. The method according to any one of preceding items (e.g., relating α-2-macroglobulin and liver cancer), wherein an increase of antennary fucose is determined by AAL, LCA, TJA II, UEA-I, PSL, AAA and/or LTA.
251. The method according to any one of preceding items (e.g., relating α-2-HS-glycoprotein and liver cancer), wherein the glycoprofile of α-2-HS-glycoprotein is determined by lectins specific for antennary fucose.
252. The method according to any one of preceding items (e.g., relating α-2-HS-glycoprotein and liver cancer), wherein an increase of antennary fucose is indicative of liver cancer.
253. The method according to any one of preceding items (e.g., relating α-2-HS-glycoprotein and liver cancer), wherein an increase of antennary fucose is determined by AAL, TJA II, UEA-I, LCA, PSL, AAA and/or LTA.
254. The method according to any one of preceding items (e.g., relating Fetuin A and liver cancer), wherein the glycoprofile of Fetuin A is determined by lectins specific for antennary fucose.
255. The method according to any one of preceding items (e.g., relating Fetuin A and liver cancer), wherein an increase of antennary fucose is indicative of liver cancer.
256. The method according to any one of preceding items (e.g., relating Fetuin A and liver cancer), wherein an increase of antennary fucose is determined by AAL, TJA II, UEA-I, LCA, PSL, AAA and/or LTA.
257. The method according to any one of preceding items (e.g., relating hemopexin and liver cancer), wherein the glycoprofile of hemopexin is determined by lectins specific for antennary fucose.
258. The method according to any one of preceding items (e.g., relating hemopexin and liver cancer), wherein an increase of antennary fucose is indicative of liver cancer.
259. The method according to any one of preceding items (e.g., relating hemopexin and liver cancer), wherein an increase of antennary fucose is determined by AAL, TJA II, UEA-I, LCA, PSL, AAA and/or LTA.
260. The method according to any one of preceding items (e.g., relating C3 complement and liver cancer), wherein the glycoprofile of C3 complement is determined by lectins specific for antennary fucose.
261. The method according to any one of preceding items (e.g., relating C3 complement and liver cancer), wherein an increase of antennary fucose is indicative of liver cancer.
262. The method according to any one of preceding items (e.g., relating C3 complement and liver cancer), wherein an increase of antennary fucose is determined by AAL, LCA, TJA II, UEA-I, PSL, AAA and/or LTA.
263. The method according to any one of preceding items (e.g., relating Histidine rich glycoprotein and liver cancer), wherein the glycoprofile of Histidine rich glycoprotein is determined by lectins specific for antennary fucose.
264. The method according to any one of preceding items (e.g., relating Histidine rich glycoprotein and liver cancer), wherein an increase of antennary fucose is indicative of liver cancer.
265. The method according to any one of preceding items (e.g., relating Histidine rich glycoprotein and liver cancer), wherein an increase of antennary fucose is determined by AAL, LCA, TJA II, UEA-I, PSL, AAA and/or LTA.
266. The method according to any one of preceding items (e.g., relating Monocyte differentiation antigen CD14 and liver cancer), wherein the glycoprofile of Monocyte differentiation antigen CD14 is determined by lectins specific for antennary fucose.
267. The method according to any one of preceding items (e.g., relating Monocyte differentiation antigen CD14 and liver cancer), wherein an increase of antennary fucose is indicative of liver cancer.
268. The method according to any one of preceding items (e.g., relating Monocyte differentiation antigen CD14 and liver cancer), wherein an increase of antennary fucose is determined by AAL, LCA, TJA II, UEA-I, PSL, AAA and/or LTA.
269. The method according to any one of preceding items (e.g., relating Hepatocyte growth factor activator and liver cancer), wherein the glycoprofile of Hepatocyte growth factor activator is determined by lectins specific for antennary fucose.
270. The method according to any one of preceding items (e.g., relating Hepatocyte growth factor activator and liver cancer), wherein an increase of antennary fucose is indicative of liver cancer.
271. The method according to any one of preceding items (e.g., relating Hepatocyte growth factor activator and liver cancer), wherein an increase of antennary fucose is determined by AAL, LCA, TJA II, UEA-I, PSL, AAA and/or LTA.
272. The method according to any one of preceding items (e.g., relating β-haptoglobin and lung cancer), wherein the glycoprofile of β-haptoglobin is determined by lectins specific for antennary fucose, core fucose, tri-, tetra-antennary glycans, α2-6Neu5Ac (α2-6 linked sialic acid), sialyl Le", tri-antennary glycans and/or sialic acid.
273. The method according to any one of preceding items (e.g., relating β-haptoglobin and lung cancer), wherein
   - an increase of antennary fucose,
   - an increase of core fucose,
   - an increase of tri-, tetra-antennary glycans,
   - an increase of α2-6Neu5Ac (α2-6 linked sialic acid),
   - an increase of sialyl Le",
   - an increase of tri-antennary glycans and/or
   - an increase of sialic acid
   is indicative of lung cancer.
274. The method according to any one of preceding items (e.g., relating β-haptoglobin and lung cancer), wherein
   - an increase of antennary fucose is determined by AAL, TJA II, UEA-I, PSL, AAA, LCA and/or LTA;
   - an increase of core fucose is determined by AOL and/or PhoSL;
   - an increase of tri-, tetra-antennary glycans is determined by PHA-E, PHA-L and/or DBA;
   - an increase of α2-6Neu5Ac (α2-6 linked sialic acid) is determined by SNA and/or TJA-I;
   - an increase of sialyl Le" is determined by SNA, TJA-I, MAA, anti-α2-3-linked Neu5Ac (α2-3 linked sialic acid) antibody (e.g., HYB4), Siglec 1, Siglec 4 and/or Siglec 8;
   - an increase of sialic acid is determined by SNA, TJA-I, MAA, anti-α2-3-linked Neu5Ac (α2-3 linked sialic acid) antibody (e.g., HYB4), Siglec 1, Siglec 4 and/or Siglec 8.
275. The method according to any one of preceding items (e.g., relating fibronectin and lung cancer), wherein the glycoprofile of fibronectin is determined by lectins specific for Galβ1-3GaINAc.
276. The method according to any one of preceding items (e.g., relating fibronectin and lung cancer), wherein an increase of Galβ1-3GaINAc is indicative of lung cancer.
277. The method according to any one of preceding items (e.g., relating fibronectin and lung cancer), wherein an increase of Galβ1-3GaINAc is determined by PNA, ABA and/or Jacalin (DSA).
278. The method according to any one of preceding items (e.g., relating α₁-acid glycoprotein and lung cancer), wherein the glycoprofile of α₁-acid glycoprotein is determined by lectins specific for antennary fucose and/or sialyl Le".
279. The method according to any one of preceding items (e.g., relating α₁-acid glycoprotein and lung cancer), wherein
   - an increase of antennary fucose and/or
   - an increase of sialyl Le"
   is indicative of lung cancer.
280. The method according to any one of preceding items (e.g., relating α₁-acid glycoprotein and lung cancer), wherein
   - an increase of antennary fucose is determined by TJA II, AAL, UEA-I, LCA, PSL, AAA and/or LTA and/or
   - an increase of sialyl Le" is determined by an antibody against sLe", SNA, TJA-I, MAA, anti-α2-3-linked Neu5Ac (α2-3 linked sialic acid) antibody (e.g., HYB4), Siglec 1, Siglec 4 and/or Siglec 8.
281. The method according to any one of preceding items (e.g., relating α-1-antitrypsin and lung cancer), wherein the glycoprofile of α-1-antitrypsin is determined by lectins specific for antennary fucose, β-Gal, Galβ1-4GIcNAc, α-Gal and α-GalNAc, (GlcNAc)ₙ, branched (LacNAc)ₙ and/or high-mannose, Manα1-3Man.
282. The method according to any one of preceding items (e.g., relating α-1-antitrypsin and lung cancer), wherein
   - an increase of antennary fucose,
   - an increase of β-Gal, Galβ1-4GlcNAc,
   - an increase of α-Gal and α-GalNAc,
   - an increase of (GlcNAc)ₙ,
   - an increase of branched (LacNAc)ₙ and/or
   - an increase of high-mannose, Manα1-3Man
   is indicative of lung cancer.
283. The method according to any one of preceding items (e.g., relating α-1-antitrypsin and lung cancer), wherein
   - an increase of antennary fucose is determined by AAL, TJA II, UEA-I, LCA, PSL, AAA and/or LTA;
   - an increase of β-Gal, Galβ1-4GIcNAc is determined by RCA120, RCA, ECL and/or AlloA;
   - an increase of α-Gal and α-GalNAc is determined by BS-I, DBA, SBA and/or HPA;
   - an increase of (GlcNAc)ₙ is determined by WGA and/or LEL;
   - an increase of branched (LacNAc)ₙ is determined by PWM and/or
   - an increase of high-mannose, Manα1-3Man is determined by GNA, Con A and/or N PA.
284. The method according to any one of preceding items (e.g., relating α₁-acid glycoprotein and stomach cancer), wherein the glycoprofile of α₁-acid glycoprotein is determined by lectins specific for bi-antennary glycans, galactose and/or Le".
285. The method according to any one of preceding items (e.g., relating α₁-acid glycoprotein and stomach cancer), wherein
   - an increase of bi-antennary glycans,
   - a decrease of galactose and/or
   - an increase of Le"
   is indicative of stomach cancer.
286. The method according to any one of preceding items (e.g., relating α₁-acid glycoprotein and stomach cancer), wherein
   - an increase of bi-antennary glycans is determined by Con A, NPA and/or GNA;
   - a decrease of galactose is determined by RCA, RCA120, ABA, AlloA, Jacalin (DSA), ECL and/or PNA and/or
   - an increase of Le" is determined by LTA.
287. The method according to any one of preceding items (e.g., relating β-haptoglobin and stomach cancer), wherein the glycoprofile of β-haptoglobin is determined by lectins specific for sialyl Le^{x} (sLe^{x}), tri-, tetra-antennary glycans, antennary fucose, sialyl-Le^{a} (sLe^{a}), (GlcNAc)ₙ and/or high mannose.
288. The method according to any one of preceding items (e.g., relating β-haptoglobin and stomach cancer), wherein
   - an increase of sialyl Le^{x}(sLe^{x}),
   - an increase of tri-, tetra-antennary glycans,
   - an increase of antennary fucose,
   - an increase of sialyl-Le^{a} (sLe^{a}),
   - an increase of (GlcNAc)ₙ and/or
   - a decrease of high mannose
   is indicative of stomach cancer.
289. The method according to any one of preceding items (e.g., relating β-haptoglobin and stomach cancer), wherein
   - an increase of sialyl Le^{x} (sLe^{x}) is determined by anti-sLe^{X} mouse monoclonal KM93 antibody, SNA, TJA-I, MAA, anti-α2-3-linked Neu5Ac (α2-3 linked sialic acid) antibody (e.g., HYB4), Siglec 1, Siglec 4 or Siglec 8;
   - an increase of tri-, tetra-antennary glycans is determined by PHA-E, PHA-L and/or DBA;
   - an increase of antennary fucose is determined by AAL, TJA II, UEA-I, LCA, PSL, AAA and/or LTA;
   - an increase of sialyl-Le^{a} (sLe^{a}) is determined by an antibody against sLe^{a}, SNA, TJA-I, MAA, anti-α2-3-linked Neu5Ac (α2-3 linked sialic acid) antibody (e.g., HYB4), Siglec 1, Siglec 4 and/or Siglec 8;
   - an increase of (GlcNAc)ₙ is determined by WGA and/or LEL and/or
   - a decrease of high mannose is determined by Con A, NPA and/or GNA.
290. The method according to any one of preceding items (e.g., relating leucine-rich-α2-glycoprotein and stomach cancer), wherein the glycoprofile of leucine-rich-α2-glycoprotein is determined by lectins specific for sialyl-Le^{x}(sLe^{x}).
291. The method according to any one of preceding items (e.g., relating leucine-rich-α2-glycoprotein and stomach cancer), wherein an increase of sialyl-Le^{x}(sLe^{x}) is indicative of stomach cancer.
292. The method according to any one of preceding items (e.g., relating leucine-rich-α2-glycoprotein and stomach cancer), wherein an increase of sialyl-Le^{x} (sLe^{x}) is determined by anti-sLe^{X} mouse monoclonal KM93 antibody, an antibody against sLe^{a}, SNA, TJA-I, MAA, anti-α2-3-linked Neu5Ac (α2-3 linked sialic acid) antibody (e.g., HYB4), Siglec 1, Siglec 4 and/or Siglec 8.
293. The method according to any one of preceding items (e.g., relating Human chorionic gonadotropin-*β* and testicular cancer), wherein the glycoprofile of Human chorionic gonadotropin-*β* is determined by lectins specific for fucose and/or tri-antennary glycans.
294. The method according to any one of preceding items (e.g., relating Human chorionic gonadotropin-*β* and testicular cancer), wherein
   - an increase of fucose and/or
   - an increase of tri-antennary glycans
   is indicative of testicular cancer.
295. The method according to any one of preceding items (e.g., relating Human chorionic gonadotropin-*β* and testicular cancer), wherein
   - an increase of fucose is determined by AAL, TJA II, UEA-I, LCA, PSL, AAA, LTA, PhoSL and/or AOL and/or
   - an increase of tri-antennary glycans is determined by PHA-E, PHA-L and/or DBA.
296. The method according to any one of preceding items (e.g., relating AFP-L3 and testicular cancer), wherein the glycoprofile of AFP-L3 is determined by lectins specific for antennary fucose.
297. The method according to any one of preceding items (e.g., relating AFP-L3 and testicular cancer), wherein an increase of antennary fucose is indicative of testicular cancer.
298. The method according to any one of preceding items (e.g., relating AFP-L3 and testicular cancer), wherein an increase of antennary fucose is determined by AAL, TJA II, UEA-I, LCA, PSL, AAA and/or LTA.
299. The method according to any one of preceding items (e.g., relating MUC1 and bladder cancer), wherein the glycoprofile of MUC1 is determined by lectins specific for antennary fucose.
300. The method according to any one of preceding items (e.g., relating MUC1 and bladder cancer), wherein an increase of antennary fucose is indicative of bladder cancer.
301. The method according to any one of preceding items (e.g., relating MUC1 and bladder cancer), wherein an increase of antennary fucose is determined by AAL, TJA II, UEA-I, LCA, PSL, AAA and/or LTA.
302. The method according to any one of preceding items (e.g., relating endoplasmin (HSP90B1) and bladder cancer), wherein the glycoprofile of endoplasmin (HSP90B1) is determined by lectins specific for antennary fucose.
303. The method according to any one of preceding items (e.g., relating endoplasmin (HSP90B1) and bladder cancer), wherein an increase of antennary fucose is indicative of bladder cancer.
304. The method according to any one of preceding items (e.g., relating endoplasmin (HSP90B1) and bladder cancer), wherein an increase of antennary fucose is determined by AAL, TJA II, UEA-I, LCA, PSL, AAA and/or LTA.
305. The method according to any one of preceding items (e.g., relating Golgi apparatus protein 1 (GLG1) and bladder cancer), wherein the glycoprofile of Golgi apparatus protein 1 (GLG1) is determined by lectins specific for antennary fucose.
306. The method according to any one of preceding items (e.g., relating Golgi apparatus protein 1 (GLG1) and bladder cancer), wherein an increase of antennary fucose is indicative of bladder cancer.
307. The method according to any one of preceding items (e.g., relating Golgi apparatus protein 1 (GLG1) and bladder cancer), wherein an increase of antennary fucose is determined by AAL, TJA II, UEA-I, LCA, PSL, AAA and/or LTA.
308. The method according to any one of preceding items (e.g., relating prostatic acid phosphatase (ACPP) and bladder cancer), wherein the glycoprofile of prostatic acid phosphatase (ACPP) is determined by lectins specific for antennary fucose.
309. The method according to any one of preceding items (e.g., relating prostatic acid phosphatase (ACPP) and bladder cancer), wherein an increase of antennary fucose is indicative of bladder cancer.
310. The method according to any one of preceding items (e.g., relating prostatic acid phosphatase (ACPP) and bladder cancer), wherein an increase of antennary fucose is determined by AAL, TJA II, UEA-I, LCA, PSL, AAA and/or LTA.
311. The method according to any one of preceding items (e.g., relating Ig gamma-2 chain C region (IGHG2) and bladder cancer), wherein the glycoprofile of Ig gamma-2 chain C region (IGHG2) is determined by lectins specific for antennary fucose.
312. The method according to any one of preceding items (e.g., relating Ig gamma-2 chain C region (IGHG2) and bladder cancer), wherein an increase of antennary fucose is indicative of bladder cancer.
313. The method according to any one of preceding items (e.g., relating Ig gamma-2 chain C region (IGHG2) and bladder cancer), wherein an increase of antennary fucose is determined by AAL, TJA II, UEA-I, LCA, PSL, AAA and/or LTA.
314. The method according to any one of preceding items (e.g., relating deoxyribonuclease-2-alpha (DNASE2A) and bladder cancer), wherein the glycoprofile of deoxyribonuclease-2-alpha (DNASE2A) is determined by lectins specific for antennary fucose.
315. The method according to any one of preceding items (e.g., relating deoxyribonuclease-2-alpha (DNASE2A) and bladder cancer), wherein an increase of antennary fucose is indicative of bladder cancer.
316. The method according to any one of preceding items (e.g., relating deoxyribonuclease-2-alpha (DNASE2A) and bladder cancer), wherein an increase of antennary fucose is determined by AAL, TJA II, UEA-I, LCA, PSL, AAA and/or LTA.
317. The method according to any one of preceding items (e.g., relating integrin and bladder cancer), wherein the glycoprofile of integrin is determined by lectins specific for sialic acid and/or tetra-antennary glycans.
318. The method according to any one of preceding items (e.g., relating integrin and bladder cancer), wherein
   - an increase of sialic acid and/or
   - an increase of tetra-antennary glycans
   is indicative of bladder cancer.
319. The method according to any one of preceding items (e.g., relating integrin and bladder cancer), wherein
   - an increase of sialic acid is determined by SNA, TJA-I, MAA, anti-α2-3-linked Neu5Ac (α2-3 linked sialic acid) antibody (e.g., HYB4), Siglec 1, Siglec 4 or Siglec 8 and/or
   - an increase of tetra-antennary glycans is determined by PHA-E, PHA-L and/or DBA.
320. The method according to any one of preceding items (e.g., relating MUC16 and bladder cancer), wherein the glycoprofile of MUC16 is determined by lectins specific for sialyl Tn.
321. The method according to any one of preceding items (e.g., relating MUC16 and bladder cancer), wherein an increase of sialyl Tn is indicative of bladder cancer.
322. The method of according to any one of preceding items (e.g., relating MUC16 and bladder cancer), wherein an increase of sialyl Tn is determined by SNA, TJA-I, MAA and/or anti-α2-3-linked Neu5Ac (α2-3 linked sialic acid) antibody (e.g., HYB4).
323. The method according to any one of preceding items (e.g., relating α-1-antitrypsin and bladder cancer), wherein the glycoprofile of α-1-antitrypsin is determined by lectins specific for high mannose and/or (GlcNAcβ1-4)ₙ.
324. The method according to any one of preceding items (e.g., relating α-1-antitrypsin and bladder cancer), wherein
   - an increase of high mannose and/or
   - an increase of (GlcNAcβ1-4)ₙ
   is indicative of bladder cancer.
325. The method according to any one of preceding items (e.g., relating α-1-antitrypsin and bladder cancer), wherein
   - an increase of high mannose is determined by Con A, NPA and/or GNA and/or
   - an increase of (GlcNAcβ1-4)ₙ is determined by WGA and/or LEL.
326. A lectin for use in a method according to any one of preceding items, preferably for use in a method selected from a group consisting of:
   i) for prediction (e.g., positive or negative) of prostate cancer; preferably said lectin is selected from the group consisting of: MAA II, AAL, Con A, SNA-I and WFA; further preferably said lectin comprises MAA II; most preferably said lectin are two lectins comprising MAA II; further most preferably said lectin comprises MAA II in combination with:
      aa) AAL, or
      bb) Con A, or
      cc) SNA-I;
   ii) for distinguishing between benign prostatic hyperplasia (BPH) and prostate cancer; preferably said lectin is selected from the group consisting of: MAA II, AAL, Con A, SNA-I and WFA; further preferably said lectin comprises MAA II; most preferably said lectin are two lectins comprising MAA II; further most preferably said lectin comprises MAA II in combination with:
      aa1) AAL, or
      bb1) Con A, or
      cc1) SNA-I;
   iii) for distinguishing between prostate cancer and metastasizing prostate cancer; preferably said lectin is selected from the group consisting of: AAL, Con A, MAA II and SNA-I; further preferably selected from the group consisting of: AAL, Con A and MAA II; most preferably selected from the group consisting of: AAL and Con A.
327. One or more lectins according to any one of preceding items, wherein said one or more lectins are selected from the group consisting of: i) *Maackia amurensis* lectin II (MAA II); ii) Concanavalin A (Con A) lectin; iii) *Aleuria aurantia* lectin (AAL); iv) *Sambucus nigra* (SNA-I) lectin; v) *Wisteria floribunda* lectin (WFL); vi) any lectin as described in Table 1 herein; preferably said one or more lectins comprising MAA II, further preferably said one or more lectins are two lectins comprising MAA II, most preferably said one or more lectins are two lectins comprising MAA II in combination with AAL, Con A or SNA-I.
328. One or more lectins according to any one of preceding items, wherein said one or more lectins are immobilized (e.g., in a sample location, e.g., in a microplate, e.g., in an Enzyme-linked Immunosorbent Assay (ELISA), enzyme-linked lectin assay (ELLA) or magnetic enzyme-linked lectin assay (MELLA) microplate).
329. A composition comprising one or more of the following:
   i) an anti-glycoprotein antibody (e.g., anti-PSA antibody or any antibody as described in Table 1 herein) or an antigen binding portion thereof according to any one of preceding items;
   ii) a magnetic carrier according to any one of preceding items;
   iii) one or more lectins (e.g., any lectin as described in Table 1 herein), preferably said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL); further preferably said one or more lectins comprising MAA II, most preferably said one or more lectins are two lectins comprising MAA II, further most preferably said one or more lectins are two lectins comprising MAA II in combination with AAL, Con A or SNA-I, further most preferably said one or more lectins are one or more lectins according to any one of preceding items.
330. The composition according to any one of preceding items comprising:
   i) magnetic carrier according to any one of preceding items or anti-glycoprotein antibody (e.g., anti-PSA, anti-AFP, anti-MUC16, anti-WFDC2, anti-MUC1, anti-ERBB2, anti-CEACAM5, anti-FUT3, anti-TG or any antibody as described in Table 1 herein), preferably said target polypeptide is anti-PSA antibody) or antigen binding portion thereof according to any one of preceding items;
   ii) one or more lectins (e.g., any lectin as described in Table 1 herein), preferably said one or more lectins are selected from the group consisting of: *Maackia amurensis* lectin II (MAA II); Concanavalin A (Con A) lectin; *Aleuria aurantia* lectin (AAL); *Sambucus nigra* (SNA-I) lectin; *Wisteria floribunda* lectin (WFL); further preferably said one or more lectins comprising MAA II, most preferably said one or more lectins are two lectins comprising MAA II, further most preferably said one or more lectins are two lectins comprising MAA II in combination with AAL, Con A or SNA-I, further most preferably said one or more lectins are one or more lectins according to any one of preceding items.
331. A kit comprising anti-glycoprotein antibody (e.g., anti-PSA, anti-AFP, anti-MUC16, anti-WFDC2, anti-MUC1, anti-ERBB2, anti-CEACAM5, anti-FUT3, anti-TG or any antibody as described in Table 1 herein, preferably anti-PSA antibody), antigen binding portion thereof, magnetic carrier, one or more lectins or composition according to any one of preceding items.
332. A kit for performing the method according to any one of preceding items, comprising an antibody specific for a cancer biomarker protein as defined according to any one of preceding items and one or more lectins as defined according to any one of preceding items, preferably said cancer biomarker is selected from the group consisting of biomarkers as described in Table 1 herein.
333. A kit for performing the method according to any one of preceding items, comprising an antibody specific for an autoimmune disease biomarker protein as defined according to any one of preceding items and one or more lectins as defined according to any one of preceding items, preferably said biomarker is selected from the group consisting of biomarkers as described in Table 1 herein.
334. A kit for performing the method according to any one of preceding items, comprising an antibody specific for an inflammatory biomarker protein as defined according to any one of preceding items and one or more lectins as defined according to any one of preceding items, preferably said biomarker is selected from the group consisting of biomarkers as described in Table 1 herein.
335. The anti-glycoprotein antibody (e.g., anti-PSA, anti-AFP, anti-MUC16, anti-WFDC2, anti-MUC1, anti-ERBB2, anti-CEACAM5, anti-FUT3, anti-TG or any antibody as described in Table 1 herein, preferably anti-PSA antibody), antigen binding portion thereof, magnetic carrier, one or more lectins or composition according to any one of preceding items for use as a medicament.
336. The anti-glycoprotein antibody (e.g., anti-PSA, anti-AFP, anti-MUC16, anti-WFDC2, anti-MUC1, anti-ERBB2, anti-CEACAM5, anti-FUT3, anti-TG or any antibody as described in Table 1 herein, preferably anti-PSA antibody), antigen binding portion thereof, magnetic carrier, one or more lectins or composition according to any one of preceding items for use in one or more of the following methods (e.g., in vitro, in vivo or ex vivo methods):
   i) for selective capture and/or enrichment of a target polypeptide according to any one of preceding items (e.g., PSA, AFP, MUC16, WFDC2, MUC1, ERBB2, CEACAM5, FUT3, TG, or any biomarker as described in Table 1 herein), preferably said target polypeptide is PSA;
   ii) for selective capture and/or enrichment of a target polypeptide according to any one of preceding items (e.g., PSA, AFP, MUC16, WFDC2, MUC1, ERBB2, CEACAM5, FUT3, TG or any biomarker as described in Table 1 herein, preferably said target polypeptide is PSA), wherein said method for selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate);
   iii) for glycoprofiling of a target polypeptide according to any one of preceding items (e.g., PSA, AFP, MUC16, WFDC2, MUC1, ERBB2, CEACAM5, FUT3, TG, or any biomarker as described in Table 1 herein, preferably said target polypeptide is PSA);
   iv) for screening and/or analysing oligosaccharide chains (e.g., glycans) covalently attached to a target polypeptide according to any one of preceding items (e.g., PSA, AFP, MUC16, WFDC2, MUC1, ERBB2, CEACAM5, FUT3, TG, or any biomarker as described in Table 1 herein, preferably said target polypeptide is PSA);
   v) for diagnostics of cancer (e.g., prostate cancer or any cancer as described in Table 1 herein);
   vi) for positive and/or negative prediction of cancer (e.g., prostate cancer or any cancer as described in Table 1 herein);
   vii) for determining a clinical stage of cancer (e.g., prostate cancer or any cancer as described in Table 1 herein);
   viii) for distinguishing between prostate cancer and metastasizing prostate cancer;
   ix) for identifying prostate cancer likely to metastasize (e.g. likely to metastasize to the bone), preferably any cancer as described in Table 1 herein;
   x) for distinguishing between benign prostatic hyperplasia (BPH) and prostate cancer;
   xi) for prevention and/or treatment of cancer (e.g., prostate cancer or any cancer as described in Table 1 herein);
   xii) for discriminating between significant and insignificant tumours, e.g., by means of glycoprotein-based (e.g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer (e.g. prostate cancer or any cancer as described in Table 1 herein);
   xiii) for discriminating between slow growing (e.g., clinically harmless) and fast growing (e.g., clinically relevant) tumours, e.g., by means of glycoprotein-based (e.g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer (e.g. prostate cancer or any cancer as described in Table 1 herein);
   xiv) for identifying organ confined and/or potentially curable cancers (e.g., prostate cancer or any cancer as described in Table 1 herein), e.g., by means of glycoprotein-based (e. g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer;
   xv) for screening compounds.
   xvi) for use in a method according to any one of preceding items.
337. Use of anti-glycoprotein antibody (e.g., anti-PSA antibody), antigen binding portion thereof, magnetic carrier, one or more lectins or composition according to any one of preceding items for one or more of the following:
   i) for selective capture and/or enrichment of a target polypeptide according to any one of preceding items (e.g., PSA, AFP, MUC16, WFDC2, MUC1, ERBB2, CEACAM5, FUT3, TG, or any biomarker as described in Table 1 herein), preferably said target polypeptide is PSA;
   ii) for selective capture and/or enrichment of a target polypeptide according to any one of preceding items (e.g., PSA, AFP, MUC16, WFDC2, MUC1, ERBB2, CEACAM5, FUT3, TG or any biomarker as described in Table 1 herein, preferably said target polypeptide is PSA), wherein said selective capture and/or enrichment comprises use of one or more lectins (e.g., immobilized lectins); preferably said one or more lectins are immobilized in a sample location (e.g., an Enzyme-linked Immunosorbent Assay (ELISA), enzyme-linked lectin assay (ELLA) or magnetic enzyme-linked lectin assay (MELLA) microplate);
   iii) for glycoprofiling of a target polypeptide according to any one of preceding items (e.g., PSA, AFP, MUC16, WFDC2, MUC1, ERBB2, CEACAM5, FUT3, TG, or any biomarker as described in Table 1 herein, preferably said target polypeptide is PSA);
   iv) for screening and/or analysing oligosaccharide chains (e.g., glycans) covalently attached to a target polypeptide according to any one of preceding items (e.g., PSA, AFP, MUC16, WFDC2, MUC1, ERBB2, CEACAM5, FUT3, TG, or any biomarker as described in Table 1 herein, preferably said target polypeptide is PSA);
   v) for diagnostics of cancer (e.g., prostate cancer or any cancer as described in Table 1 herein);
   vi) for positive and/or negative prediction of cancer (e.g., prostate cancer or any cancer as described in Table 1 herein);
   vii) for determining a clinical stage of cancer (e.g., prostate cancer or any cancer as described in Table 1 herein);
   viii) for distinguishing between prostate cancer and metastasizing prostate cancer;
   ix) for identifying prostate cancer likely to metastasize (e.g. likely to metastasize to the bone), preferably any cancer as described in Table 1 herein;
   x) for distinguishing between benign prostatic hyperplasia (BPH) and prostate cancer;
   xi) for prevention and/or treatment of cancer (e.g., prostate cancer or any cancer as described in Table 1 herein);
   xii) for discriminating between significant and insignificant tumours, e.g., by means of glycoprotein-based (e.g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer (e.g. prostate cancer or any cancer as described in Table 1 herein);
   xiii) for discriminating between slow growing (e.g., clinically harmless) and fast growing (e.g., clinically relevant) tumours, e.g., by means of glycoprotein-based (e.g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer (e.g. prostate cancer or any cancer as described in Table 1 herein);
   xiv) for identifying organ confined and/or potentially curable cancers (e.g., prostate cancer or any cancer as described in Table 1 herein), e.g., by means of glycoprotein-based (e. g., target polypeptide-based, e.g., based on PSA, AFP, MUC16, WFDC2, MUC1, ERBB2, CEACAM5, FUT3, TG, or any biomarker as described in Table 1 herein, preferably said target polypeptide is PSA) diagnostics of cancer;
   xv) for screening compounds
   xvi) for use in a method according to any one of preceding items.
338. The use according to any one of preceding items, wherein said use is an in vitro, ex vivo or in vivo use or combinations thereof.
339. The anti-glycoprotein antibody, antigen binding portion thereof, magnetic carrier, one or more lectins, kit, composition, use or method according to any one of preceding items, wherein said lectin is at least 60% or more (e.g., at least 65%, at least 70%, at least 75%, at least 80%, at least 85%, at least 90%, at least 95%, at least 96%, at least 97%, at least 98%, at least 99% or 100%) identical to a polypeptide sequence selected from the group consisting of: SEQ ID NO: 52, 53, 54, 55, 56, 57, 58, 59, lectins with following UniProtKB Accession Numbers: P0DKL3, P02866, P18891, O04366, A0A218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, 024313, Q9SM56, P06750, B9SPG3, Q9BZZ2, P20916, Q9NYZ4, Q96RL6, P05046, P93535, P02876, P10968, P10969, P22972 or P56625, lectins as described in Table 1 herein, wherein said lectin is capable to specifically reacting with glycosidic residue of other molecule (e.g. cell wall polysaccharide and/or glycoprotein (e.g., target polypeptide according any one of the preceding items or a biomarker selected from the group consisting of biomarkers as described in Table 1 herein)).
340. The anti-glycoprotein antibody, antigen binding portion thereof, magnetic carrier, one or more lectins, kit, composition, use or method according to any one of preceding items, wherein said lectin is selected from the group consisting of: SEQ ID NO: 52, 53, 54, 55, 56, 57, 58, 59, lectins with following UniProtKB Accession Numbers: P0DKL3, P02866, P18891, O04366, A0A218PFP3, Q945S3, Q00022, Q6YNX3, Q71QF2, P02872, P18670, Q2UNX8, Q8L5H4, A0A089ZWN7, P05045, P19588, P83410, P17931, P56470, P24146, Q41263, Q39990, Q2F1K8, G9M5T0, B3XYC5, P02870, P19664, P0DKL3, P49300, A9XX86, Q40423, P16300, P05088, P05087, Q9AVB0, P02867, 024313, Q9SM56, P06750, B9SPG3, Q9BZZ2, P20916, Q9NYZ4, Q96RL6, P05046, P93535, P02876, P10968, P10969, P22972 or P56625, lectins as described in Table 1 herein.
341. The anti-glycoprotein antibody, antigen binding portion thereof, magnetic carrier, one or more lectins, kit, composition, use or method according to any one of preceding items, wherein said lectin is mature.
342. The anti-glycoprotein antibody, antigen binding portion thereof, magnetic carrier, one or more lectins, kit, composition, use or method according to any one of preceding items, wherein said antibody is selected from the group consisting of: antibodies as described in Table 1 herein.
343. The anti-glycoprotein antibody, antigen binding portion thereof, magnetic carrier, one or more lectins, kit, composition, use or method according to any one of preceding items, wherein said biomarker is selected from the group consisting of biomarkers as described in Table 1 herein.
344. The anti-glycoprotein antibody, antigen binding portion thereof, magnetic carrier, one or more lectins, kit, composition, use or method according to any one of preceding items, wherein said cancer is selected from the group consisting of cancers as described in Table 1 herein.
345. The anti-glycoprotein antibody, antigen binding portion thereof, magnetic carrier, one or more lectins, kit or composition, use or method according to any one of preceding items, wherein said antibody, said biomarker, said cancer and said lectin are selected from the group consisting of corresponding antibodies, biomarkers, cancers and lectins as described in Table 1 herein.
346. The anti-glycoprotein antibody, antigen binding portion thereof, magnetic carrier, one or more lectins, kit, composition, use or method according to any one of preceding items, wherein a corresponding glycan modification (e.g., corresponding change (e.g., detectable change) of a glycan state and/or glycan composition and/or glycan concentration and/or glycan complexing (e.g., dimerization, trimerization, branching, etc.) is selected from the group consisting of glycan modifications as described in Table 1 herein).

### Examples of the invention

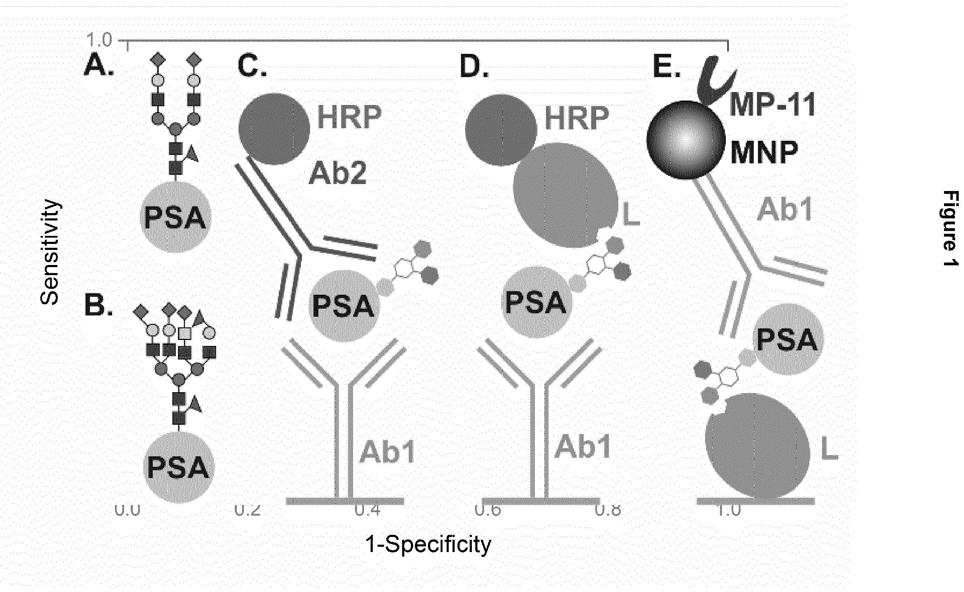

In the course of the present invention the innovation potential of changed glycans (complex carbohydrates) of PSA rather than PSA level in human serum (current standard clinical practice) as a potential novel biomarker for improved prostate cancer (PCa) diagnosis has been investigated (e.g., Figure 1). A typical glycan composition on PSA from healthy individual is shown in **Fig. 1A** and from PCa patient in **Fig. 1B**, indicating glycan changes detectable by lectins (glycan binding proteins) for PCa diagnostics. PSA level in serum sample is usually detected in a sandwich configuration using sensing antibody (Ab1) and detecting antibody (Ab2) (**Fig. 1C**). Glycoprofiling of PSA is performed in a sandwich configuration (**Fig. 1E**) with a single chain antibody fragment (scAb) (or an antibody) and microperoxidase-11 (MP-11) immobilised on magnetic particles applied for selective capture of PSA from serum with lectins immobilised on an Enzyme-Linked ImmunoSorbent Assays (ELISA) microplate (**Fig. 1E**). Magnetic particles with MP-11 and antibodies have a dual role - PSA enrichment from a serum sample and MP-11 applied for optical signal generation (**Fig. 1E**). The method of the present invention for PSA glycoprofiling significantly differs from the methods described in the prior art (e.g., as shown in **Fig. 1D**, but this method involves many steps and requires at least 1.5 mL of serum), for example, among other differences in that it implements a dual role of magnetic particles with immobilised MP-11 and antibody fragment what significantly enhance sensitivity of detection, reducing required amount of sample needed to 0.04 mL and shortens analysis time by using magnetic particles for PSA enrichment and signal generation.

Furthermore, prior art methods are silent on glycoprofiling of the investigated analytes. Moreover, in the course of the present invention a microperoxidase-11 (MP-11) enzyme has been implemented instead of frequently applied and much larger horseradish (HRP) (1.9 kDa vs. 44 kDa respectively) has been used. Additionally, an antigen binding portion of the anti-PSA antibody (e.g., 8x4x3.5 nm) has been implemented in the method of the present invention instead of much larger full-length anti-PSA antibody (15x7x3.5 nm). Said antigen binding portion of the anti PSA antibody was generated as described by Andris-Widhopf et al. (2000).

The method of the present invention works for example in ELLA or MELLA format thus, specialised instrumentation for running surface plasmon fluorescent spectrometry, a microchip capillary electrophoresis employing lectins, a suspension array system and flow cytometry is not needed, and the assay can be performed in ELLA or MELLA (e.g., ELISA-like) format, which is fully compatible with current clinical practise.

The method of the present invention is the first one describing dual role of modified magnetic particles for glycoprofiling of any protein (e.g., a cancer biomarker).

### Materials and methods of Examples 1 and 2:

### Magnetic ELLA (MELLA) for PSA:

At first, 30 µg of equimolar ligand mixture (38 131 Da scAb and 1822 Da MP-11, i.e., 28.6 µg of scAb and 1.4 µg of MP-11) per mg of magnetic particles (MNPs) was conjugated overnight. 100 µl of 10 µg/m! lectin solution in PB (0.1 M, pH = 7.4, filtered using 0.2 µm sterile filter) was added to the each well of a Maxisorp plate (Thermo Scientific, US) and incubated 1 h at room temperature (RT) or at 4°C overnight. After a washing procedure (3x with 200 µl of phosphate buffer (PB)), the wells were blocked using 100 µl of carbofree blocking solution (Vector Labs, US) for 1 hour (h). After the washing step, plates are ready for MELLA analysis.

Human serum samples were aliquoted and kept at -80°C. Prior to analysis, all samples were diluted to the same PSA level, to make the results comparable (in PB 0.1 M, pH = 7.4, filtered using 0.2 µm sterile filter), e.g., 0.7 ng/ml in this case. Normalized sera were mixed in 1+1 ratio (usually 20+20 µl) with MNPs, further diluted 5x using PB (final volume 200 µl) and incubated at RT for 1h with shaking. MNPs were subsequently washed 3x using PB, resuspended in 500 µl of PB and added into the plate wells (100 µl/well). After 10 min of incubation with shaking, a gentle washing step using multichannel pipette and PB was applied. To create a colorimetric signal, 10 mg/ml OPD (o-phenylenediamine dihydrochloride) solution in citrate (0.05 M)-phosphate (0.2 M) buffer (pH 4.6) was used with an addition of 27 µl/20 ml 30% hydrogen peroxide. The reaction was stopped using 3.6 M sulphuric acid (100 µl to the 100 µl of OPD solution in the wells) after 15 min incubation at RT in dark. The signal was measured at 490 nm and RT immediately.

### Example 1:

In this example, BPH (benign prostatic hyperplasia) samples and PCa samples were analysed by the method of the present invention.

### Results

Firstly, single biomarkers were analysed by the method of the present invention.

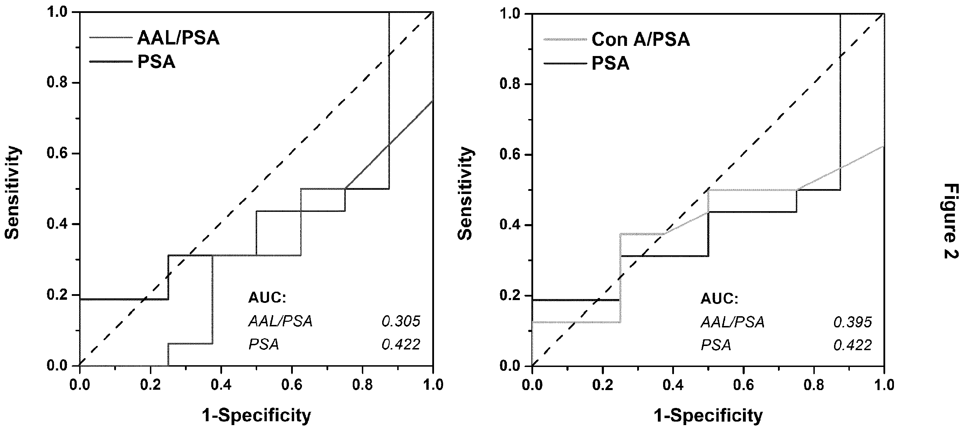

For that five different lectins (MAA-II, Con A, AAL, SNA-I and WFA, purchased from Vector Labs, USA) were applied for glycoprofiling of PSA in a magnetic ELLA format by the method of the present invention (e.g., **Fig. 1E**) to find out the best lectins applicable in PSA glycoprofiling (e.g., **Fig. 2****,** **3** **and** **4**) and compare the results to a conventional PSA-based test. From all these lectins tested only MAA-II was a positive predictor of prostate cancer (PCa) with Area Under the Curve (AUC)=0.871 and other three lectins were a negative predictor of PCA with AUC=0.305 for AAL, AUC=0.395 for Con A and AUC=0.356 for SNA-I. When receiver operating characteristic (ROC) curves were reversed into a positive predictor AUC values for AAL of 0.695, Con A of 0.605, and SNA-I of 0.645 were found (e.g., **Fig. 2**). Total number of samples in the examples was as follows: 8 BPH (e.g., Example 1), 8 PCa serum samples without (e.g., Examples 1 and 2) and 8 PCa serum samples with metastasis (e.g., Example 2). Serum samples were not pretreated. The results are summarised in the **Table 2** below, showing AUC, sensitivity and specificity values and are also shown in **Fig. 2****,** **3** **and** **4** below.

**Table 2: Performance of the tested single biomarkers.**

| **Biomarker** | **Predictor** | **AUC** | **Sensitivity** | **Specificity** |
|---|---|---|---|---|
| **PSA** | + | 0.422 | - | - |
| **AAL** | - | 0.695 | 50% | 75% |
| **Con A** | - | 0.605 | 50% | 75% |
| **MAA-II** | + | 0.871 | 87.5% | 87.5% |
| **SNA-I** | - | 0.645 | 57% | 75% |
| **WFA** | + | 0.602 | 50% | 75% |

Secondly, double biomarkers were analysed by the method of the present invention.

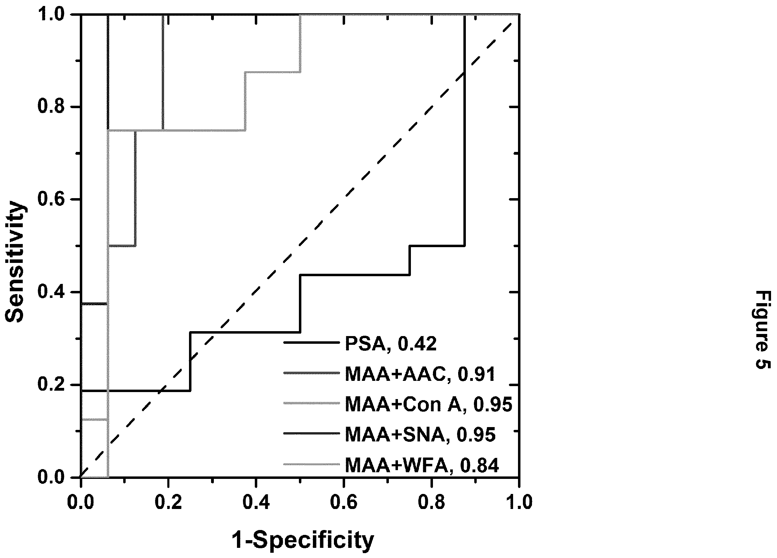

Since MAA lectin showed the best performance (see above) by the method of the present invention (e.g., **Fig. 1E**) combinations of MAA lectin with other lectins was further analysed for their respective predictive properties and compared to the results of a conventional PSA-based test. Results showed that three different combinations of MAA with other lectin, i.e., MAA+AAL (AUC=0.91), MAA+Con A (AUC=0.95) and MAA+SNA (AUC=0.95) (e.g., s. **Table 3** below) showed better performance than MAA alone (AUC=0.87) (e.g., s. **Table 2** above). Combination MAA+WFA (AUC=0.84) (e.g., **Table 3**) showed a slightly lower performance compared to single MAA biomarker (AUC=0.87) (e.g., **Table 2**). The results are also shown in **Fig. 5** below.

**Table 3: Performance of the tested double biomarkers.**

| **Biomarker** | **AUC** | **Sensitivity** | **Specificity** |
|---|---|---|---|
| **PSA** | 0.42 | - | - |
| **MAA+AAL** | 0.91 | 100% | 81.3% |
| **MAA+Con A** | 0.95 | 100% | 93.8% |
| **MAA+SNA-I** | 0.95 | 100% | 93.8% |
| **MAA+WFA** | 0.84 | 75% | 93.8% |

Thirdly, triple biomarkers were analysed by the method of the present invention.

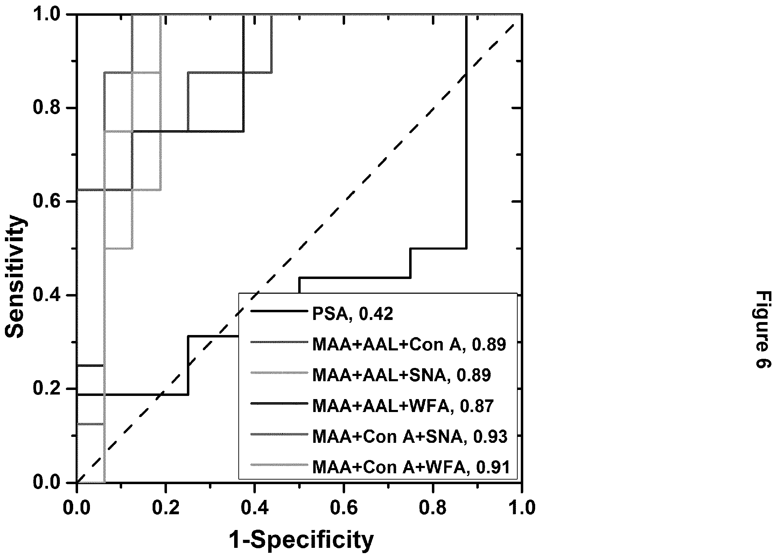

Here combinations of three different lectins were tested to see if it would be possible to better predict PCa patients by the method of the present invention (e.g., **Fig. 1E**) and compare the results to a conventional PSA-based test. Results showed that by using combination of three lectins AUC was in the range 0.87-0.93 (e.g., **Table 4** below), what was worse result compared to double biomarkers MAA+Con A (AUC=0.95) and MAA+SNA (AUC=0.95) (e.g., **Table 3** above) and it was concluded that it was slightly better to use double biomarkers instead of triple biomarkers. The results are also shown in **Fig. 6** below.

**Table 4: Performance of the tested triple biomarkers.**

| **Biomarker** | **AUC** | **Sensitivity** | **Specificity** |
|---|---|---|---|
| **PSA** | 0.42 | - | - |
| **MAA+AAL+Con A** | 0.89 | 87.5% | 75% |
| **MAA+AAL+SNA** | 0.89 | 100% | 81.3% |
| **MAA+AAL+WFA** | 0.87 | 75% | 93.8% |
| **MAA+Con A+SNA** | 0.93 | 100% | 87.5% |
| **MAA+Con A+WFA** | 0.91 | 100% | 81.3% |

Fourthly, quadruple and pentadruple biomarkers were analysed by the method of the present invention.

Here combinations of four and five different lectins were tested to see if it would be possible to better predict PCa patients by the method of the present invention (e.g., **Fig. 1E**) and compare the results to a conventional PSA-based test. Results showed that by using combination of three lectins AUC was in the range 0.89-0.92 (e.g., **Table 5** below), what was slightly worse compared to double biomarkers MAA+Con A (AUC=0.95) and MAA+SNA (AUC=0.95) (e.g., **Table 3** above) and it was concluded that it was slightly better to use double biomarkers instead of quadruple and pentadruple biomarkers. The results are not shown in a form of ROC curve.

**Table 5: Performance of the tested quadruple biomarkers.**

| **Biomarker** | **AUC** |
|---|---|
| **PSA** | 0.42 |
| **MAA+AAL+Con A+SNA** | 0.90 |
| **MAA+AAL+Con A+WFA** | 0.89 |
| **MAA+ Con A+SNA+WFA** | 0.91 |
| **MAA+AAL+Con A+SNA+WFA** | 0.92 |

### Conclusion 1

**Single biomarkers:** Three out of four lectins, i.e., AAL, Con A and SNA-I are negative predictor of PCa, while MAA-II is a positive predictor of the disease. The best biomarker is MAA-II, when AUC (0.87) values and sensitivity and specificity of PCa diagnosis is taking into account and the performance of other lectins for diagnosis is quite similar in terms of AUC values and sensitivity and specificity of PCa diagnosis.

**Double biomarkers:** Combination of MAA+Con A (AUC=0.95) and MAA+SNA (AUC=0.95) showed the best performance and combination of MAA+AAL (AUC=0.91) showed slightly better performance compared to single MAA (AUC=0.87).

**Triple, quadruple and pentadruple biomarkers:** Combination of three, four and five lectins did not outperform performance of double biomarkers MAA+Con A and MAA+SNA, so these two double biomarkers are the best biomarkers to distinguish BPH and PCa patients.

### Example 2:

In this example, PCa samples with metastasis (PCa+) and PCa samples without metastasis (PCa-) were analysed by the method of the present invention (e.g., **Fig. 1E**).

### Results

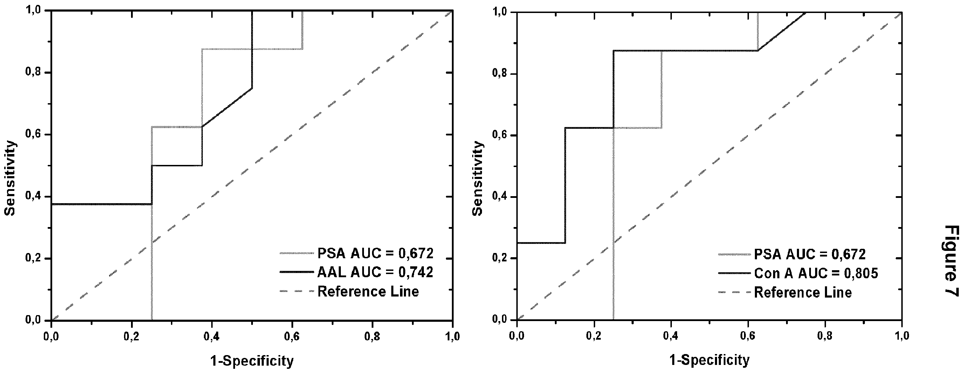

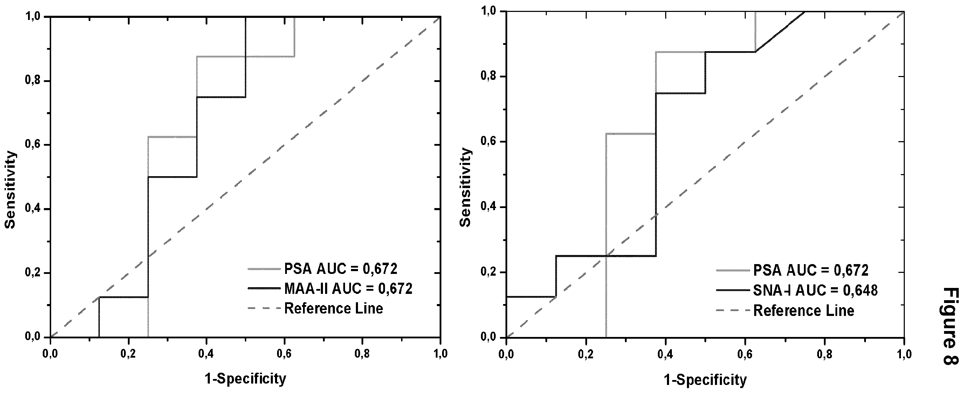

Here, the possibility to use glycan biomarkers to distinguish PCa+ patients from PCa-patients using lectins was investigated and results showed that lectin AAL (AUC=0.742), Con A (AUC=0.805) (e.g., **Fig. 7** **and** **8**) had a better predictive value than PSA (AUC=0.672). Lectin MAA, which was able to discriminate the best between BPH and PCa patients had the same predictive value PCa+ vs. PCa+ (AUC=0.672) as PSA (AUC=0.672) (e.g., **Fig. 7** **and** **8**). Lectin SNA (AUC=0.648) had slightly lower predictive value compared to PSA (AUC=0.672) (e.g., **Fig. 7** **and** **8**). This means that lectins can be effectively applied for diagnosis of different PCa stages.

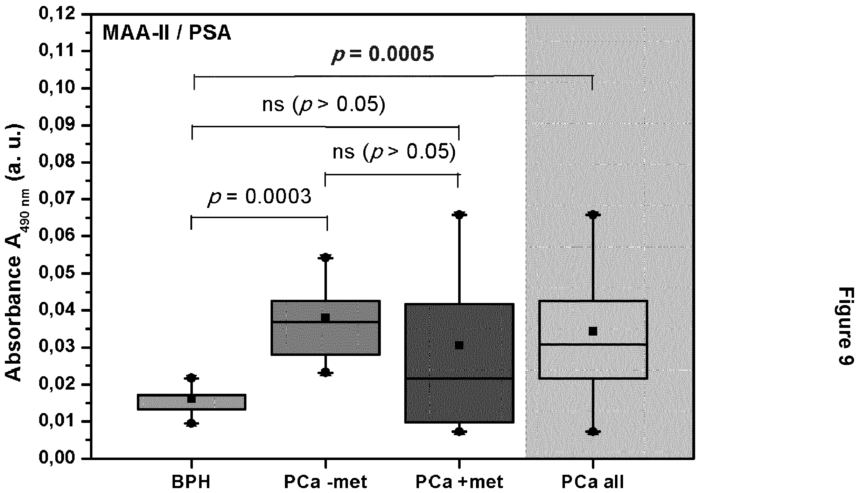

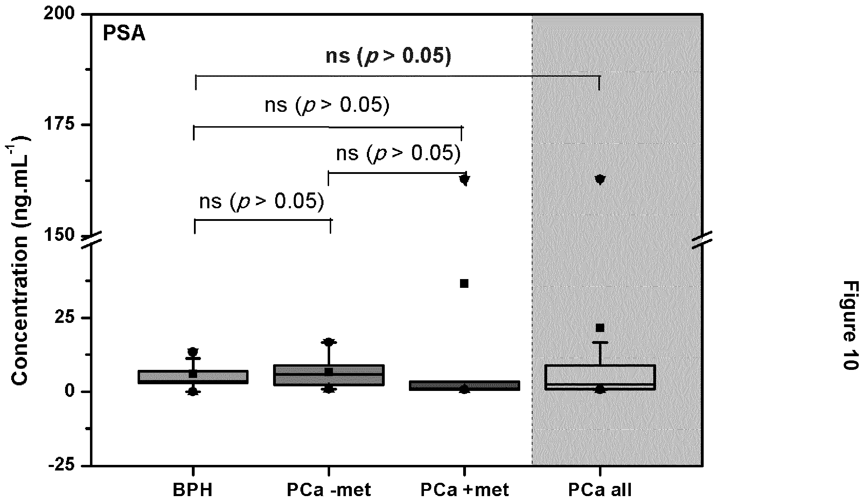

Performance of the best single biomarker MAA II was compared to performance of PSA in a form of a box plot as shown in **Figs. 9** and **10**. MAA lectin can very well distinguish between BPH and PCa- patients (p=0.0003), while ability to distinguish between PCa- and PCa+ patients was slightly worse (p>0.05), what can be caused by the treatment of PCa+ patients. MAA II also had ability to distinguish very well between BPH and PCa patients (combined PCa- and PCa+ patients) with p=0.0005. As already discussed using ROC curves MAA II does not discriminate very well between PCa- and PCa+ patients with p>0.05 (**Figs. 9** and **10**). When considering PSA, these biomarkers performed slightly worse, when compared to MAA II biomarker (**Figs. 9** and **10**).

### Conclusion 2

It was concluded that two lectins have ability to discriminate between PCa- and PCa+ samples (i.e., AAL and Con A better than PSA and that there is a potential to apply lectins not only for distinguishing BPH *vs.* PCa patients, but also to distinguish various stages of PCa.

### Example 3:

In this example glycoprofiling of the whole sera was carried out without using an antibody on BPH and PCa samples.

Firstly, magnetic particles (MPs) were incubated with human serum diluted to have the same PSA level (as in the previous experiments 0.72 ng/ml) using only 20 µL of a sample as usual, followed by the analysis of the same number of human serum samples as usual.

### Results

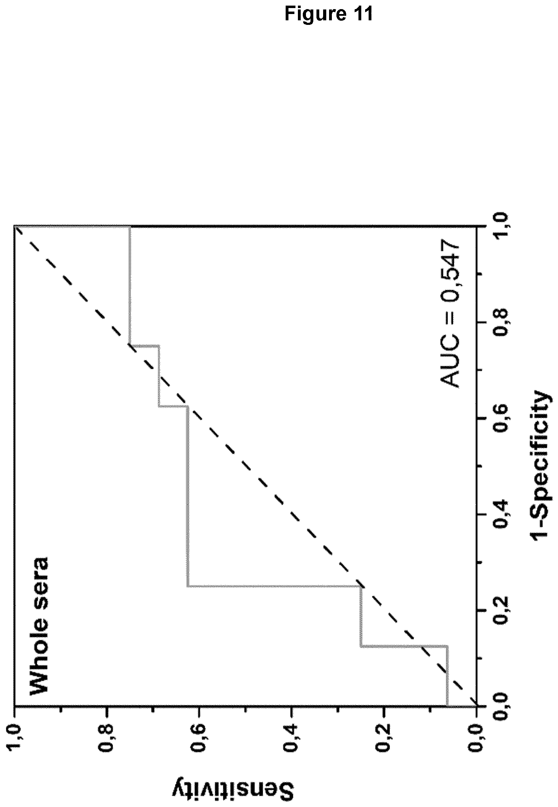

The results showed AUC value of only 0.547 (e.g., **Fig. 11**), what is very low value, indicating almost no discrimination power between BPH and PCa patients.

### Example 4:

In this example glycoprofiling of PSA after its release from magnetic particles was carried out using the approach from the prior art, i.e., using Ag/Ab gentle elution buffer, pH 6.6

Incubation of diluted human serum was carried out as described above for Experiment 3 with MPs with immobilised anti-PSA antibody (Abcam, UK). Release of PSA from magnetic particles was repeated 3 times using an elution buffer (Ag/Ab gentle elution buffer, pH 6.6). Desalting of released PSA was carried out using Zeba spin desalting columns, MWCO 10 kDa (Thermo). Incubation of released and desalted PSA with ELISA plate having immobilised anti-PSA antibody, Incubation of the ELISA plates was carried out with a HRP-MAA-II conjugate (EYLabs, USA).

### Results

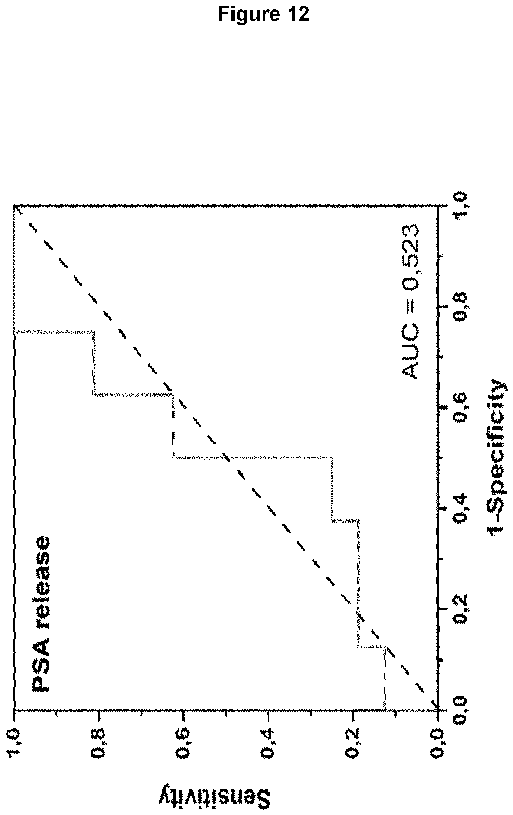

The obtained results showed AUC value of only 0.523 (e.g., **Fig. 12**), which is again a very low value, indicating almost no discrimination power between BPH and PCa patients.

### Example 5:

In this example, glycoprofiling of PSA was carried out as described above with an antibody immobilized on a solid surface, here an ELISA plate. MAA-II was used as lectin.

### Results

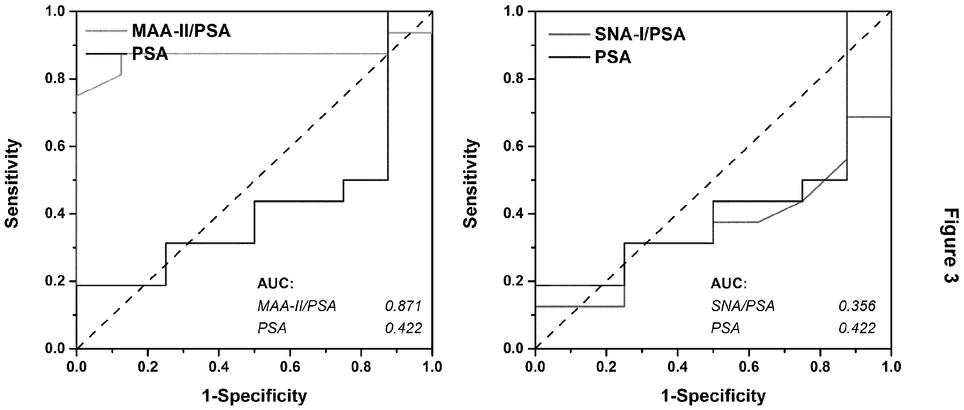

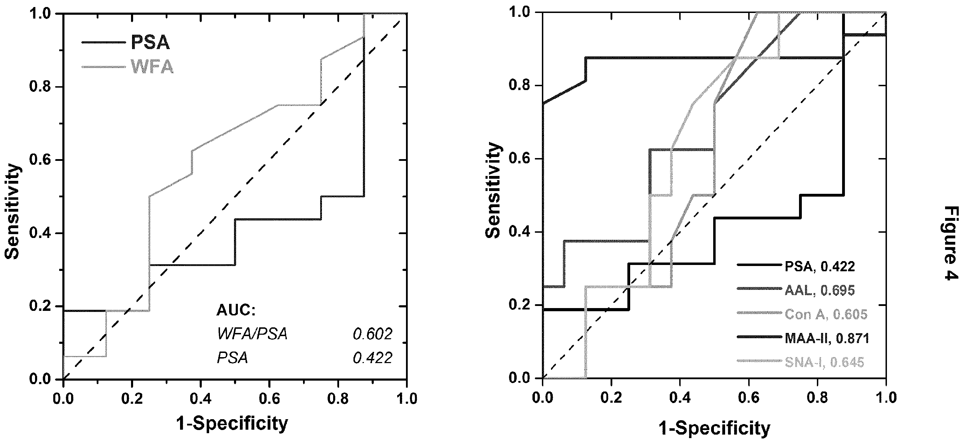

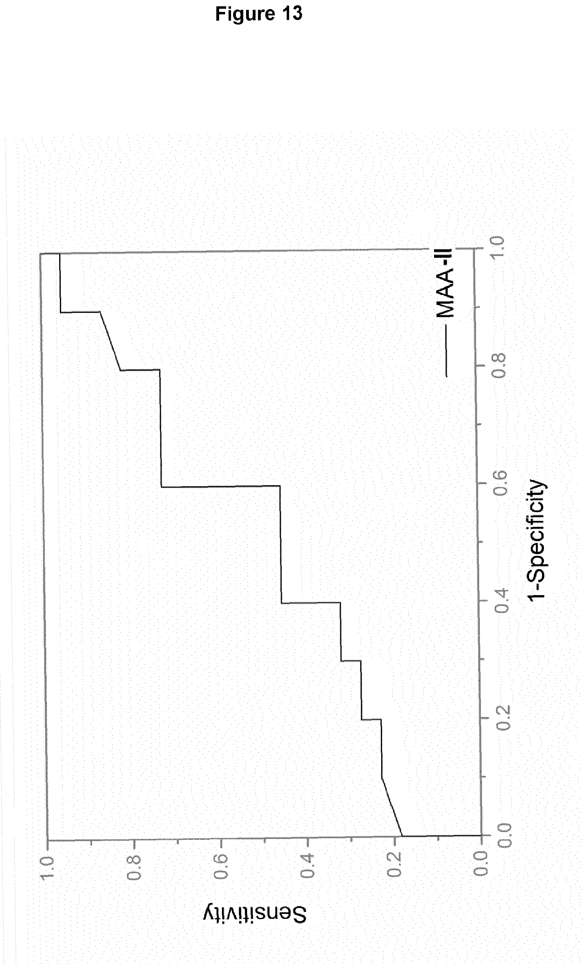

AUC value for MAA lectin was 0.518 (see Fig. 13). This AUC value is lower than those AUC values observed when using an antibody which is not immobilized on a solid surface, but bound to a bead, e.g. a magnetic bead (see, e.g. Fig. 3 or 4). The low AUC value indicates that almost no discrimination power between BPH and PCa patients is possible.

## Claims

1. A method of determining the glycoprofile of a protein, comprising
(a) contacting a sample comprising said protein with an antibody directed against said protein to form an antibody-protein complex;
(b) isolating the antibody-protein complex obtained in step (a); and
(c) contacting the antibody-protein complex with one or more lectins to determine the glycoprofile of said protein,
wherein said antibody of step (a) is not immobilized on a solid surface, and wherein said protein is not released from said antibody while performing the method.

2. The method of any one of the preceding claims, further comprising step (d) comparing the glycoprofile of said protein with a control glycoprofile of said protein to determine whether the glycoprofile of said protein may deviate from the glycoprofile of said control glycoprofile.

3. The method of any one of the preceding claims, wherein said protein is a cancer biomarker protein, an autoimmune disease biomarker protein or an inflammatory disease biomarker protein.

4. The method of claim 3, wherein said cancer biomarker protein is an ovarian cancer biomarker protein, breast cancer biomarker protein, colorectal cancer biomarker protein, pancreatic cancer biomarker protein, prostate cancer biomarker protein, thyroid cancer biomarker protein, liver cancer biomarker protein, lung cancer biomarker protein, stomach cancer biomarker protein, testicular cancer biomarker protein or bladder cancer biomarker protein.

5. The method of claim 4, wherein prostate cancer biomarker protein is β-haptoglobin, TIMP-1, PSA, fPSA ortPSA.

6. The method of any one of the preceding claims, wherein said antibody comprises a bead, which allows the isolation of said antibody.

7. The method of any one of the preceding claims, wherein said one or more lectins are specific for core fucose, antennary fucose, Fucα1-6GlcNAc-*N*-Asn containing N-linked oligosaccharides, Fucα1-6/3GlcNAc, α-L-Fuc, Fucα1-2Galβ1-4(Fucα1-3)GlcNAc, Fucα1-2Gal, Fucα1-6GlcNAc, Manβ1-4GlcNAcβ1-4GIcNAc, branched *N*-linked hexa-saccharide, Manα1-3Man, α-D-Man, (GlcNAcβ1-4)₂₋₄, Galβ1-4GlcNAc, GlcNAcα1-4Galβ1-4GlcNAc, (GlcNAcβ1-4)₂₋₅, Neu5Ac(sialic acid), Galβ1-3GalNAc-serine/threonine, Galα1-3GalNAc, Galβ1-6Gal, Galβ1-4GlcNAc, Galβ1-3GalNAc, GalNAcα,1-3GalNAc, GalNAcα1-3Gal, GalNAcα/β1-3/4Gal, α-GalNAc, GalNAcβ1-4Gal, GalNAcα1-3(Fucα1-2)Gal, GalNAcα1-2Gal, GalNAcα1-3GalNAc, GalNAcβ1-3/4Gal, GalNAc-Ser/Thr (Tn antigen), Galβ1-3GalNAc-Ser/Thr (T antigen), GalNAcβ1-4GlcNAc (LacdiNAc), α-2,3Neu5Ac (α2-3 linked sialic acid), α-2,6Neu5Ac (α2-6 linked sialic acid), α-2,8Neu5Ac (α2-8 linked sialic acid), sialic acid (α-2,3Neu5Ac, α-2,6Neu5Ac or α-2,8Neu5Ac), Neu5Acα4/9-O-Ac-Neu5Ac, Neu5Acα2-3Galβ1-4Glc/GlcNAc, Neu5Acα2-6Gal/GalNAc, *N*-linked bi-antennary, *N*-linked tri/tetra-antennary, branched β1-6GlcNAc, Galα1-3(Fucα1-2)Galβ1-3/4GlcNAc, Galβ1-3(Fucα1-4)GIcNAc, NeuAcα2-3Galβ1-3(Fucα1-4)GlcNAc, Fucα1-2Galβ1-3(Fucα1-4)GIcNAc, Galβ1-4(Fucα1-3)GlcNAc, NeuAcα,2-3Galβ1-4(Fuca1-3)GlcNAc, Fucα1-2Galβ1-4(Fuca1-3)GlcNAc, high mannose, sialyl Lewis^{a} (sialyl Le^{a}) antigen, sialyl Lewis^{x} (sialyl Le^{x}) antigen, Lewis^{x} (Le^{x}) antigen, sialyl Tn antigen, sialyl T antigen, Lewis^{y} (Le^{y}) antigen, sulfated core1 glycan, Tn antigen, T antigen, core 2 glycan, Lewis^{a} (Le^{a}) antigen, (GlcNAcβ1-4)ₙ, β-D-GlcNAc, GalNAc, Gal-GlcNAc, GlcNAc, Galα1-3Gal, Galβ1-3GalNAc, α-Gal, α-GalNAc, (GlcNAc)ₙ, branched (LacNAc)ₙ.

8. A method for diagnosing whether a subject may be at a risk or may suffer from cancer, comprising
(a) contacting a sample obtained from said subject, said sample comprising a cancer biomarker protein, with an antibody directed against said cancer biomarker protein to form an antibody-cancer biomarker protein complex; and
(b) isolating the antibody-protein complex obtained in step (a); and
(c) contacting the antibody-cancer biomarker protein complex with one or more lectins to determine the glycoprofile of said cancer biomarker protein,
wherein said antibody of step (a) is not immobilized on a solid surface, and wherein said protein is not released from said antibody while performing the method,
wherein a deviation of said glycoprofile from the healthy glycoprofile of said cancer biomarker protein is indicative that said subject may be at a risk or may suffer from cancer.

9. A method for diagnosing whether a subject may be at a risk or may suffer from an autoimmune disease, comprising
(a) contacting a sample obtained from said subject, said sample comprising an autoimmune disease biomarker protein, with an antibody directed against said autoimmune disease biomarker protein to form an antibody-autoimmune disease biomarker protein complex; and
(b) isolating the antibody-protein complex obtained in step (a); and
(c) contacting the antibody-autoimmune disease biomarker protein complex with one or more lectins to determine the glycoprofile of said autoimmune disease biomarker protein,
wherein said antibody of step (a) is not immobilized on a solid surface, and wherein said protein is not released from said antibody while performing the method,
wherein a deviation of said glycoprofile from the healthy glycoprofile of said autoimmune disease biomarker protein is indicative that said subject may be at a risk or may suffer from an autoimmune disease.

10. A method for diagnosing whether a subject may be at a risk or may suffer from an inflammatory disease, comprising
(a) contacting a sample obtained from said subject, said sample comprising an inflammatory disease biomarker protein, with an antibody directed against said inflammatory disease biomarker protein to form an antibody-inflammatory biomarker protein complex; and
(b) isolating the antibody-protein complex obtained in step (a); and
(c) contacting the antibody-inflammatory biomarker protein complex with one or more lectins to determine the glycoprofile of said inflammatory disease biomarker protein,
wherein said antibody of step (a) is not immobilized on a solid surface, and wherein said protein is not released from said antibody while performing the method,
wherein a deviation of said glycoprofile from the healthy glycoprofile of said inflammatory disease biomarker protein is indicative that said subject may be at a risk or may suffer from an inflammatory disease.

11. A kit for performing the method of claim 8, comprising an antibody specific for a cancer biomarker protein as defined in claim 4 and one or more lectins as defined in claim 7.

12. A kit for performing the method of claim 9, comprising an antibody specific for an autoimmune disease biomarker protein, which is IgG and one or more lectins as defined in claim 7.

13. A kit for performing the method of claim 10, comprising an antibody specific for an inflammatory biomarker protein, which is IgG, IgA or CRP and one or more lectins as defined in claim 7.

14. The method of any one of claims 1-10, wherein said one or more lectins are immobilized.

15. The method of any one of claims 1-10, wherein said antibody is bound to a magnetic carrier.
